# EUROPEAN PATENT SPECIFICATION

(11) **EP 1 105 422 B1**
(45) Date of publication and mention of the grant of the patent: **15.02.2006**
(21) Application number: 99938085.0
(22) Date of filing: 09.08.1999
(51) Int. Cl.: C07K 14/81, C07K 5/10, C07K 7/06, A61K 38/55, A61P 31/14

(54) **HEPATITIS C INHIBITOR PEPTIDES**
HEPATITIS C INHIBITOR PEPTIDE
PEPTIDES INHIBITEURS DE L'HEPATITE C

(30) Priority: 10.08.1998 US 95945 P
(43) Date of publication of application: 13.06.2001
(73) Proprietor: BOEHRINGER INGELHEIM (CANADA) LTD., Laval, Quebec, H7S 2G5 (CA)
(72) Inventor: LLINAS-BRUNET, Montse, Dollard-Des-Ormeaux, Québec H9B 3K6 (CA); BAILEY, Murray, D., Pierrefonds, Québec H8Y 1B3 (CA); CAMERON, Dale, Rosemère, Québec J7A 4M4 (CA); GHIRO, Elise, Laval, Québec H7X 3L8 (CA); GOUDREAU, Nathalie, Mont-Royal, Québec H3P 2C9 (CA); POUPART, Marc-André, Vimont, Québec H7M 1B3 (CA); RANCOURT, Jean, Laval, Québec H7L 4W2 (CA); TSANTRIZOS, Youla, S., Saint-Laurent, Québec H4L 4P7 (CA)
(74) Representative: Kläs, Heinz-Gerd
(86) International application number: PCT/CA1999/000737
(87) International publication number: WO 2000/009558

(56) References cited:
- WO-A-98/17679
- WO-A-99/07733
- INGALLINELLA P ET AL: "Potent Peptide Inhibitors of Human Hepatitis C Virus NS3 Protease Are Obtained by Optimizing the Clevage Products" BIOCHEMISTRY, vol. 37, 1998, pages 8906-8914, XP002900817
- MONTSE LLINÁS-BRUNET ET AL: "Peptide-based inhibitors of the hepatitis C virus serine protease" BIOOGANIC & MEDICINAL CHEMISTRY LETTERS, vol. 8, 1998, pages 1713-1718, XP002900818
- MORI A ET AL: "The N-Terminal Region of NS3 Serine Proteinase of Hepatitis C Virus Is Important to Maintain Its Enzymatic Integrity" BIOCHEMICAL AND BIOPHYSICAL RESEARCH COMMUNICATIONS, vol. 231, 1997, pages 738-742, XP002900819

## Description

### FIELD OF THE INVENTION

The present invention relates to compounds, compositions and methods for the treatment of hepatitis C virus (HCV) infection. In particular, the present invention provides novel peptides, analogs and intermediates thereof, pharmaceutical compositions containing such peptides and methods for using these peptides in the treatment of HCV infection.

### BACKGROUND OF THE INVENTION

Hepatitis C virus (HCV) is the major etiological agent of post-transfusion and community-acquired non-A non-B hepatitis worldwide. It is estimated that over 150 million people worldwide are infected by the virus. A high percentage of carriers become chronically infected and many progress to chronic liver disease, so called chronic hepatitis C. This group is in turn at high risk for serious liver disease such as liver cirrhosis, hepatocellular carcinoma and terminal liver disease leading to death.

The mechanism by which HCV establishes viral persistence and causes a high rate of chronic liver disease has not been thoroughly elucidated. It is not known how HCV interacts with and evades the host immune system. In addition, the roles of cellular and humoral immune responses in protection against HCV infection and disease have yet to be established. Immunoglobulins have been reported for prophylaxis of transfusion-associated viral hepatitis. However, the Center for Disease Control does not presently recommend immunoglobulins for this purpose.

The lack of an effective protective immune response is hampering the development of a vaccine or adequate post-exposure prophylaxis measures, so in the near-term, hopes are firmly pinned on antiviral interventions.

Various clinical studies have been conducted with the goal of identifying pharmaceutical agents capable of effectively treating HCV infection in patients afflicted with chronic hepatitis C. These studies have involved the use of interferon-alpha, alone and in combination with other antiviral agents. Such studies have shown that a substantial number of the participants do not respond to these therapies, and of those that do respond favorably, a large proportion were found to relapse after termination of treatment.

Until recently, interferon (IFN) was the only available therapy of proven benefit approved in the clinic for patients with chronic hepatitis C. However the sustained response rate is low, and interferon treatment also induces severe side-effects (i.e. retinopathy, thyroiditis, acute pancreatitis, depression) that diminish the quality of life of treated patients. Recently, interferon in combination with ribavirin has been approved for patients non-responsive to IFN alone. However, the side effects caused by IFN are not alleviated with this combination therapy.

Therefore, a need exists for the development of effective antiviral agents for treatment of HCV infection that overcomes the limitations of existing pharmaceutical therapies.

HCV is an enveloped positive strand RNA virus in the Flaviviridae family. The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature nonstructural proteins (NS2, NS3, NS4A, NS4B, NS5A, and NS5B) is effected by two viral proteases. The first one, as yet poorly characterized, cleaves at the NS2-NS3 junction; the second one is a serine protease contained within the N-terminal region of NS3 (henceforth referred to as NS3 protease) and mediates all the subsequent cleavages downstream of NS3, both in *cis,* at the NS3-NS4A cleavage site, and in *trans,* for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B is a RNA-dependent RNA polymerase that is involved in the replication of HCV.

A general strategy for the development of antiviral agents is to inactivate virally encoded enzymes that are essential for the replication of the virus. In this vein, patent application WO 97/06804 describes the (-) enantiomer of the nucleoside analogue cytosine-1,3-oxathiolane (also known as 3TC) as active against HCV. This compound, although reported as safe in previous clinical trials against HIV and HBV, has yet to be clinically proven active against HCV and its mechanism of action against the virus has yet to be reported.

Intense efforts to discover compounds which inhibit the NS3 protease or RNA helicase of HCV have led to the following disclosures:

WO 99/07733 describes peptides and analogues thereof that are inhibitory to the NS3 protease of the hepatitis C virus.

US patent 5,633,388 describes heterocyclic-substituted carboxamides and analogues as being active against HCV. These compounds are directed against the helicase activity of the NS3 protein of the virus but clinical tests have not yet been reported.

A phenanthrenequinone has been reported by Chu *et al* (Tet. Lett., (1996), 7229-7232) to have activity against the HCV NS3 protease *in vitro.* No further development on this compound has been reported.

A paper presented at the Ninth International Conference on Antiviral Research, Urabandai, Fukyshima, Japan (1996) (Antiviral Research, 30, 1, 1996; A23 (abstract 19)) reports thiazolidine derivatives to be inhibitory to the HCV protease.

Several studies have reported compounds inhibitory to other serine proteases, such as human leukocyte elastase. One family of these compounds is reported in WO 95/33764 (Hoechst Marion Roussel, 1995). The peptides disclosed in that application are morpholinylcarbonyl-benzoyl-peptide analogues that are structurally different from the peptides of the present invention.

WO 98/17679 from Vertex Pharmaceuticals Inc. discloses inhibitors of serine protease, particularly, Hepatitis C virus NS3 protease. These inhibitors are peptide analogues based on the NS5A/5B natural substrate. All of these peptides contain C-terminal activated carbonyl function as an essential feature. These peptides were also reported to be active against other serine protease and are therefore not specific for HCV NS3 protease.

Hoffman LaRoche has also reported hexapeptides that are proteinase inhibitors useful as antiviral agents for the treatment of HCV infection. These peptides contain an aldehyde or a boronic acid at the C-terminus.

Steinkühler *et al.* and Ingallinella *et al.* have published on N terminal cleavage product inhibition (Biochemistry (1998), 37, 8899-8905 and 8906-8914). However, the peptides and peptide analogues presented do not include nor do they lead to the design of the peptides of the present invention.

WO 98/46597 from Emory University discloses serine protease inhibitors, particularly Hepatitis C virus protease. All of the compounds disclosed are structurally different from the peptides of the present invention.

WO 98/46630 from Peptide Therapeutics Ltd. discloses hepatitis C NS3 protease inhibitors. However, none of the peptides disclosed are related to the peptides of the invention.

JP10298151 from Japan Energy Corp. discloses N-(2,3-dihydroxybenzoyl)-substituted serine derivatives as serine protease inhibitors, specifically as hepatitis C viral protease inhibitors. These compounds do not contain any structural similarity to the peptide analogs of the present invention.

One advantage of the present invention is that it provides peptides that are inhibitory to the NS3 protease of the hepatitis C virus.

A further advantage of one aspect of the present invention resides in the fact that these peptides specifically inhibit the NS3 protease and do not show significant inhibitory activity at concentrations up to 300 µM against other serine proteases such as human leukocyte elastase (HLE), porcine pancreatic elastase (PPE), or bovine pancreatic chymotrypsin, or cysteine proteases such as human liver cathepsin B (Cat B).

### SUMMARY OF THE INVENTION

Included in the scope of the invention are racemates, diastereoisomers and optical isomers of a compound of formula (I): 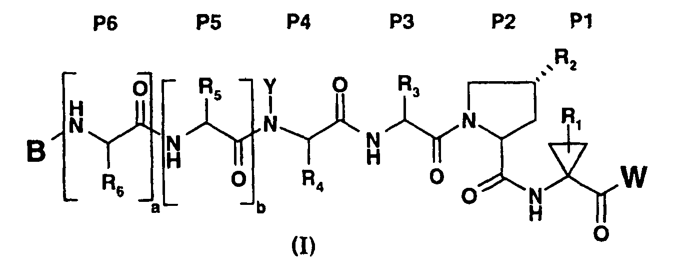
wherein
- **a**: is 0 or 1**; b** is 0 or 1; **Y** is H or C₁₋₆ alkyl;
- **B**: is H, an acyl derivative of formula **R**₇-C(O)- or a sulfonyl of formula **R**₇-SO₂ wherein **R**_{**7**} is (i) C₁₋₁₀ alkyl optionally substituted with carboxyl, C₁₋₆ alkanoyloxy or C₁₋₆ alkoxy;
(ii) C₃₋₇ cycloalkyl optionally substituted with carboxyl, (C₁₋₆ alkoxy)carbonyl or phenylmethoxycarbonyl;
(iii) C₆ or C₁₀ aryl or C₇₋₁₆ aralkyl optionally substituted with C₁₋₆ alkyl, hydroxy, or amino optionally substituted with C₁₋₆alkyl; or
(iv) Het optionally substituted with C₁₋₆ alkyl, hydroxy, amino optionally substituted with C₁₋₆ alkyl, or amido optionally substituted with C₁₋₆ alkyl;
- **R**_{**6**}: is C₁₋₆ alkyl substituted with carboxyl;
- **R**_{**5**}: is C₁₋₆ alkyl optionally substituted with carboxyl;
- **R**_{**4**}: is C₁₋₁₀ alkyl, C₃₋₇ cycloalkyl or C₄₋₁₀ (alkylcycloalkyl);
- **R**_{**3**}: is C₁₋₁₀ alkyl, C₃₋₇ cycloalkyl or C₄₋₁₀ (alkylcycloalkyl);
- **R**_{**2**}: is CH₂-R₂₀, NH-**R**_{**20**}, O-R₂₀ or S-**R**_{**20**}, wherein **R**_{**20**} is a saturated or unsaturated C₃₋₇ cycloalkyl or C₄₋₁₀(alkyl cycloalkyl) being optionally mono-, di- or tri-substituted with **R**_{**21**},
- or **R**_{**20**}: is a C₆ or C₁₀ aryl or C₇₋₁₆ aralkyl optionally mono-, di- or tri-substituted with **R**_{**21**}**,**
- or **R**_{**20**}: is Het or (lower alkyl)-Het optionally mono-, di- or tri-substituted with R₂₁,
wherein each **R**_{**21**} is independently C₁₋₆alkyl; C₁₋₆alkoxy; amino optionally mono- or di-substituted with C₁₋₆ alkyl; sulfonyl; NO₂; OH; SH; halo; haloalkyl; amido optionally mono-substituted with C₁₋₆ alkyl, C₆ or C₁₀ aryl, C₇₋₁₆ aralkyl, Het or (lower alkyl)-Het; carboxyl; carboxy(lower alkyl); C₆ or C₁₀ aryl, C₇₋₁₆ aralkyl or Het, said aryl, aralkyl or Het being optionally substituted with **R**_{**22**}**;**
wherein **R**_{**22**} is C₁₋₆ alkyl; C₁₋₆ alkoxy; amino optionally mono- or di-substituted with C₁₋₆ alkyl; sulfonyl; NO₂; OH; SH; halo; haloalkyl; carboxyl; amide; or (lower alkyl)amide;
- **R**_{**1**}: is C₁₋₆alkyl or C₂₋₆alkenyl optionally substituted with halogen; and
- **W**: is hydroxy or a N-substituted amino;

or a pharmaceutically acceptable salt or ester thereof.

Included within the scope of this invention is a pharmaceutical composition comprising an anti-hepatitis **C** virally effective amount of a compound of formula I, or a therapeutically acceptable salt or ester thereof, in admixture with a pharmaceutically acceptable carrier medium or auxiliary agent.

An important aspect of the invention involves the use of a compound of formula I or a therapeutically acceptable salt or ester thereof for the preparation of a medicament for treating a hepatitis C viral infection.

Another important aspect involves a in vitro method of inhibiting the replication of hepatitis C virus by exposing the virus to a hepatitis C viral NS3 protease inhibiting amount of the compound of formula I, or a therapeutically acceptable salt or ester thereof or a composition as described above.

Still another aspect involves the use of a combination of the compound of formula I or a therapeutically acceptable salt or ester thereof and an interferon for the preparation of a medicament for treating a hepatitis C viral infection.

### DETAILED DESCRIPTION OF THE INVENTION

### Definitions

As used herein, the following definitions apply unless otherwise noted:

With reference to the instances where (*R*) or (*S*) is used to designate the configuration of a radical, e.g. R₄ of the compound of formula I, the designation is done in the context of the compound and not in the context of the radical alone.

The natural amino acids, with exception of glycine, contain a chiral carbon atom. Unless otherwise specifically indicated, the compounds containing natural amino acids with the L-configuration are preferred. However, applicants contemplate that when specified, some amino acids of the formula I can be of either D- or L-configuration or can be mixtures of D- and L-isomers, including racemic mixtures.

The designation "P1, P2, P3 etc." as used herein refer to the position of the amino acid residues starting from the C-terminus end of the peptide analogues and extending towards the N-terminus (i.e. P1 refers to position 1 from the C-terminus, P2: second position from the C-terminus, etc.) (see Berger A. & Schechfer I., Transactions of the Royal Society London series B257, 249-264 (1970)).

The abbreviations for the α-amino acids are set forth in Table A.

**TABLE A**

| **AMINO ACID** | **SYMBOL** |
|---|---|
| Alanine | Ala |
| Aspartic acid | Asp |
| Cysteine | Cys |
| Cyclohexylglycine (also named: 2-amino-2-cyclohexylacetic acid) | Chg |
| Glutamic acid | Glu |
| Isoleucine | Ile |
| Leucine | Leu |
| Phenylalanine | Phe |
| Proline | Pro |
| Valine | Val |
| *tert*-Butylglycine | Tbg |

As used herein the term "1-aminocyclopropyl-carboxylic acid" (Acca) refers to a compound of formula:

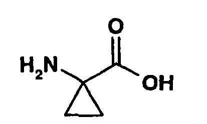

As used herein the term "*tert*-butylglycine" (Tbg) refers to a compound of formula:

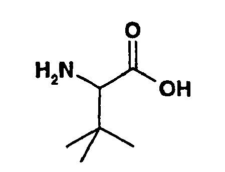

The term "residue" with reference to an amino acid or amino acid derivative means a radical derived from the corresponding α-amino acid by eliminating the hydroxyl of the carboxy group and one hydrogen of the α-amino group. For instance, the terms Gln, Ala, Gly, Ile, Arg, Asp, Phe, Ser, Leu, Cys, Asn, Sar and Tyr represent the "residues" of L-glutamine, L-alanine, glycine, L-isoleucine, L-arginine, L-aspartic acid, L-phenylalanine, L-serine, L-leucine, L-cysteine, L-asparagine, sarcosine and L-tyrosine, respectively.

The term "side chain" with reference to an amino acid or amino acid residue means a group attached to the α-carbon atom of the α-amino acid. For example, the R-group side chain for glycine is hydrogen, for alanine it is methyl, for valine it is isopropyl. For the specific R-groups or side chains of the α-amino acids reference is made to A.L. Lehninger's text on Biochemistry (see chapter 4 of LEHNINGER A.L.: Biochemistry 2nd edition, Worth Publishers, Inc., N.Y. 1975).

The term "halo" as used herein means a halogen radical selected from bromo, chloro, fluoro or iodo.

The term "C₁₋₆ alkyl" or "(lower)alkyl" as used herein, either alone or in combination with another radical, means straight chain or branched alkyl radicals containing up to six carbon atoms and includes, for example, methyl, ethyl, propyl, butyl, hexyl, 1-methylethyl, 1-methylpropyl, 2-methylpropyl, 1,1-dimethylethyl (e.g. *tert*-butyl).

The term "C₃₋₇ cycloalkyl" as used herein, either alone or in combination with another radical, means a cycloalkyl radical containing from three to seven carbon atoms and includes cyclopropyl, cyclobutyl, cyclopentyl, cyclohexyl and cycloheptyl.

The term "unsaturated cycloalkyl" includes, for example, the cyclohexenyl:

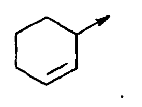

The term "C₄₋₁₀ (alkylcycloalkyl) as used herein means a cycloalkyl radical containing from three to seven carbon atoms linked to an alkyl radical, the linked radicals containing up to ten carbon atoms; for example, cyclopropylmethyl, cyclopentylethyl, cyclohexylmethyl, cyclohexylethyl or cycloheptylethyl.

The term "C₂₋₁₀ alkenyl" as used herein, either alone or in combination with another radical, means an alkyl radical as defined above containing from 2 to 10 carbon atoms, and further containing at least one double bond. For example alkenyl includes allyl and vinyl.

The term "C₁₋₆ alkanoyl" as used herein, either alone or in combination with another radical, means straight or branched 1-oxoalkyl radicals containing one to six carbon atoms and includes formyl, acetyl, 1-oxopropyl (propionyl), 2-methyl-l-oxopropyl, 1-oxohexyl and the like.

The term "C₁₋₆ alkoxy" as used herein, either alone or in combination with another radical, means the radical -O(C₁₋₆alkyl) wherein alkyl is as defined above containing up to six carbon atoms. Alkoxy includes methoxy, ethoxy, propoxy, 1-methylethoxy, butoxy and 1,1-dimethylethoxy. The latter radical is known commonly as *tert*-butoxy.

The term "C₃₋₇ cycloalkoxy" as used herein, either alone or in combination with another radical, means a C₃₋₇ cycloalkyl group linked to an oxygen atom, such as, for example:

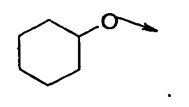

The term "C₆ or C₁₀ aryl" as used herein, either alone or in combination with another radical, means either an aromatic monocyclic group containing 6 carbon atoms or an aromatic bicyclic group containing 10 carbon atoms. For example, aryl includes phenyl, 1-naphthyl or 2-naphthyl.

The term "C₇₋₁₆ aralkyl" as used herein, either alone or in combination with another radical, means a C₆ or C₁₀ aryl as defined above linked to an alkyl group, wherein alkyl is as defined above containing from 1 to 6 carbon atoms. C₇₋₁₆ Aralkyl includes for example benzyl, butylphenyl, and 1-naphthylmethyl.

The term "amino aralkyl" as used herein, either alone or in combination with another radical, means an amino group substituted with a C₇₋₁₆ aralkyl group, such as, for example, the amino aralkyl:

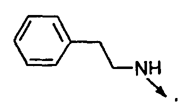

The term "carboxy(lower)alkyl" as used herein, either alone or in combination with another radical, means a carboxyl group (COOH) linked through a (lower)alkyl group as defined above and includes for example butyric acid.

The term "heterocycle" or **"Het"** as used herein, either alone or in combination with another radical, means a monovalent radical derived by removal of a hydrogen from a five-, six-, or seven-membered saturated or unsaturated (including aromatic) heterocycle containing from one to four heteroatoms selected from nitrogen, oxygen and sulfur. Furthermore, "Het" as used herein, means a heterocycle as defined above fused to one or more other cycle be it a heterocycle or any other cycle. Examples of suitable heterocycles include: pyrrolidine, tetrahydrofuran, thiazolidine, pyrrole, thiophene, diazepine, 1H-imidazole, isoxazole, thiazole, tetrazole, piperidine, 1,4-dioxane, 4-morpholine, pyridine, pyrimidine, thiazolo[4,5-b]-pyridine, quinoline, or indole, or the following heterocycles:

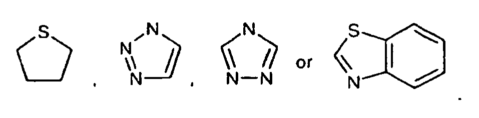

The term "(lower alkyl)-Het" as used herein, means a heterocyclic radical as defined above linked through a chain or branched alkyl group, wherein alkyl is as defined above containing from 1 to 6 carbon atoms. Examples of (lower alkyl)-Het include:

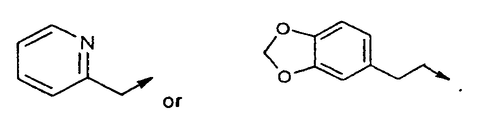

The term "pharmaceutically acceptable ester" as used herein, either alone or in combination with another radical, means esters of the compound of formula I in which any of the carboxyl functions of the molecule, but preferably the carboxy terminus, is replaced by an alkoxycarbonyl function: 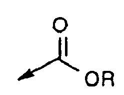
in which the R moiety of the ester is selected from alkyl (e.g. methyl, ethyl, *n*-propyl, t-butyl, *n*-butyl); alkoxyalkyl (e.g. methoxymethyl); alkoxyacyl (e.g. acetoxymethyl); aralkyl (e.g. benzyl); aryloxyalkyl (e.g. phenoxymethyl); aryl (e.g. phenyl), optionally substituted with halogen, C₁₋₄ alkyl or C₁₋₄ alkoxy. Other suitable prodrug esters can be found in Design of prodrugs, Bundgaard, H. Ed. Elsevier (1985). Such pharmaceutically acceptable esters are usually hydrolyzed *in vivo* when injected in a mammal and transformed into the acid form of the compound of formula I.

With regard to the esters described above, unless otherwise specified, any alkyl moiety present advantageously contains 1 to 16 carbon atoms, particularly 1 to 6 carbon atoms. Any aryl moiety present in such esters advantageously comprises a phenyl group.

In particular the esters may be a C₁₋₁₆ alkyl ester, an unsubstituted benzyl ester or a benzyl ester substituted with at least one halogen, C₁₋₆ alkyl, C₁₋₆ alkoxy, nitro or trifluoromethyl.

The term "pharmaceutically acceptable salt" as used herein includes those derived from pharmaceutically acceptable bases. Examples of suitable bases include choline, ethartolamine and ethylenediamine. Na⁺, K⁺, and Ca⁺⁺ salts are also contemplated to be within the scope of the invention (also see Pharmaceutical salts, Birge, S.M. et al., J. Pharm. Sci. (1977), 66, 1-19).

### Preferred embodiments

Included within the scope of this invention are compounds of formula I wherein
**B** is preferably **R**_{**7**}-SO₂ wherein **R**_{**7**} is preferably C₆ or C,o aryl, a C₇₋₁₆ aralkyl or Het all optionally substituted with C₁₋₆ alkyl.

Alternatively, **B** is preferably H or an acyl derivative of formula **R**_{**7**}C(O)- wherein **R**_{**7**} is preferably C₁₋₆ alkyl; C₁₋₆ alkoxy; C₃₋₇ cycloalkyl optionally substituted with hydroxy; amido optionally substituted with C₁₋₆ alkyl or Het; C₆ or C₁₀ aryl, C₇₋₁₆ aralkyl or Het all optionally substituted with C₁₋₆ alkyl or hydroxy. More preferably, **B** is H or **R**_{**7**}C(O)- wherein **R**_{**7**} is more preferably C₁₋₆ alkyl or Heterocycles such as: 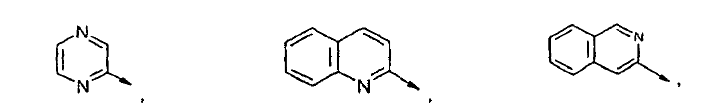 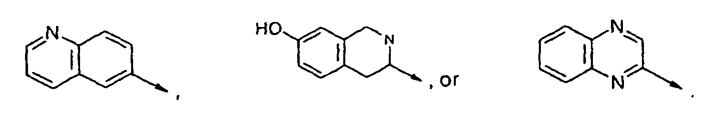 Most preferably, **B** is H; acetyl;

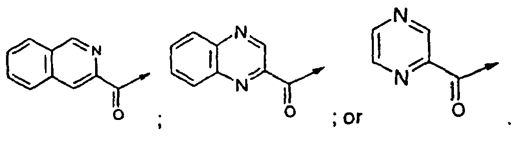

Even most preferably, **B** is acetyl.

Included within the scope of the invention are compounds of formula I wherein **R**_{**6**} is preferably the side chain of Asp or Glu. Most preferably, **R**_{**6**}, is the side chain of Asp. Alternatively, preferably, a is 0 and then **R**_{**6**} is absent.

Included within the scope of the invention are compounds of formula I wherein, preferably, **R**_{**5**}**,** is the side chain of an amino acid selected from the group consisting of: D-Asp, L-Asp, D-Glu, L-Glu, D-Val, L-Val, D-tert-butylglycine (Tbg), and L-Tbg. More preferably, **R**_{**5**}, is the side chain of D-Asp, D-Val, or D-Glu. Most preferably, **R**_{**5**}, is the side chain of D-Glu.

Included within the scope of the invention are compounds of formula I wherein, preferably, **R**_{**4**} is the side chain of an amino acid selected from the group consisting of: Val, cyclohexylglycine (Chg), Tbg, Ile or Leu. More preferably, **R**_{**4**} is the side chain of Chg or Ile. Most preferably, **R**_{**4**} is the side chain of Chg.

Included within the scope of the invention are compounds of formula I wherein, preferably, **Y** is H, or Me. Most preferably, **Y** is H.

Included within the scope of the invention are compounds of formula I wherein, preferably, **R**_{**3**} is the side chain of an amino acid selected from the group consisting of: IIe, Chg, Val or Tbg. More preferably, **R**_{**3**} is the side chain of Val, Chg or Tbg. Most preferably, **R**_{**3**} is the side chain of Val or Tbg.

Included within the scope of the invention are compounds of formula I wherein, preferably, **R**_{**2**} is S-**R**_{**20**} or O-**R**_{**20**} wherein **R**_{**20**} is preferably a C₆ or C₁₀ aryl, C₇₋₁₆ aralkyl, Het or -CH₂-Het, all optionally mono-, di- or tri-substituted with **R**_{**21**}.
Preferably, **R**_{**21**} is C₁₋₆ alkyl; C₁₋₆ alkoxy; amino; mono- or di-(lower alkyl)amino; amido optionally mono-substituted with C₁₋₆ alkyl, C₆ or C₁₀ aryl, C₇₋₁₆ aralkyl, Het or (lower alkyl)-Het; NO₂; OH; halo; trifluoromethyl; carboxyl; C₆ or C₁₀ aryl, C₇₋₁₆ aralkyl, or Het, said aryl, aralkyl or Het being optionally substituted with **R**_{**22**}**.** More preferably, **R**_{**21**} is C₁₋₆ alkyl; C₁₋₆ alkoxy; amino; di(lower alkyl)amino; (lower alkyl)amide; C₆ or C₁₀ aryl, or Het, said aryl or Het being optionally substituted with **R**_{**22**}**.**
Preferably, **R**_{**22**} is C₁₋₆ alkyl; C₁₋₆ alkoxy; amino; mono- or di-(lower alkyl)amino; (lower alkyl)amide; NO₂; OH; halo; trifluoromethyl; or carboxyl. More preferably, **R**_{**22**} is C₁₋₆ alkoxy; amino; di(lower alkyl)amino; (lower alkyl)amide; halo; or trifluoromethyl.

More preferably, **R**_{**2**} is 1-naphthylmethoxy; 2-naphthylmethoxy; benzyloxy, 1-naphthyloxy; 2-naphthyloxy; or quinolinoxy unsubstituted , mono- or di-substituted with **R**_{**21**} as defined above. Most preferably, **R**_{**2**} is 1-naphtylmethoxy; or quinolinoxy unsubstituted, mono- or di-substituted with **R**_{**21**} as defined above.

Still, most preferably, **R**_{**2**} is :

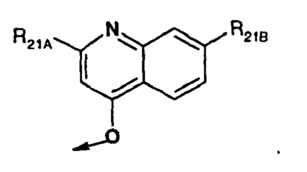

More preferably, **R**_{**21A**} is amido optionally mono-substituted with C₁₋₆ alkyl, C₆ or C₁₀ aryl, C₇₋₁₆ aralkyl or Het; or C₆ or C₁₀ aryl or Het optionally substituted with **R**_{**22**}**.** Most preferably, **R**_{**21A**} is C₆ or C₁₀ aryl or Het, all optionally substituted with **R**_{**22**}**.** Most preferably, **R**_{**22**} is amino; di(lower alkyl)amino; or (lower alkyl)amide. Even most preferably, **R**_{**22**} is amino; dimethylamino; or acetamido.

Even most preferably, **R**_{**21A**} is C₆ or C₁₀ aryl or Het, all unsubstituted.

Preferably, **R**_{**21B**} is C₁₋₆ alkyl; C₁₋₆ alkoxy; amino; di(lower alkyl)amino; (lower alkyl)amide; NO₂; OH; halo; trifluoromethyl; or carboxyl. More preferably, **R**_{**21B**} is C₁₋₆ alkoxy; or di(lower alkyl)amino. Most preferably, **R**_{**21B**} is methoxy.

Included within the scope of the invention are compounds of formula I wherein, preferably, **R**_{**1**} is methyl, ethyl, propyl, vinyl all of which optionally substituted with halo. More preferably, **R**_{**1**} is ethyl, vinyl or bromovinyl. Most preferably, **R**_{**1**} is vinyl.

Included within the scope of the invention are compounds of formula I wherein, preferably, **W** is hydroxy or a pharmaceutically acceptable salt or ester thereof; or (lower alkyl)amino, di(lower alkyl)amino or amino aralkyl. More preferably, **W** is hydroxy, or **N(R**_{**13a**}**)R**_{**13b**} wherein **R**_{**13a**} and **R**_{**13b**} are independently H, aryl or C₁₋₆ alkyl optionally substituted with hydroxy or phenyl; or a pharmaceutically acceptable salt thereof. Most preferably, **W** is -OH, -NH-benzyl or -NH-CH(Me)Ph. Still most preferably, **W** is -OH or -NH-(S)CH(Me)-phenyl.

When **W** is an ester, such ester is preferably selected from C₁₋₆ alkoxy, phenoxy, or aryl(C₁₋₆ alkoxy). More preferably such ester is methoxy, ethoxy, phenoxy, benzyloxy, or PhCH(Me)-O-.

As described hereinabove the **P1** segment of the compounds of formula I is a cyclopropyl ring system of formula: 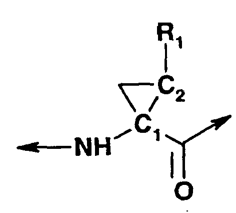
wherein **C**_{**1**} and **C**_{**2**} each represent an asymmetric carbon atom at positions 1 and 2 of the cyclopropyl ring. Notwithstanding other possible asymmetric centers at other segments of the compounds of formula I, the presence of these two asymmetric centers means that the compound of formula I can exist as racemic mixtures of diastereoisomers. As illustrated in the examples hereinafter, the racemic mixtures can be prepared and thereafter separated into individual optical isomers, or these optical isomers can be prepared by chiral synthesis.

Hence, the compound of formula I can exist as a racemic mixture of diastereoisomers wherein **R**_{**1**} at position 2 is orientated *syn* to the carbonyl at position **1**, represented by the radical: 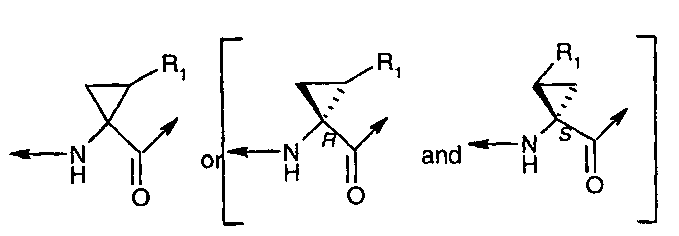
or the compound of formula I can exist as a racemic mixture of diastereoisomers wherein **R**_{**1**} at position 2 is orientated *anti* to the carbonyl at position **1**, represented by the radical:

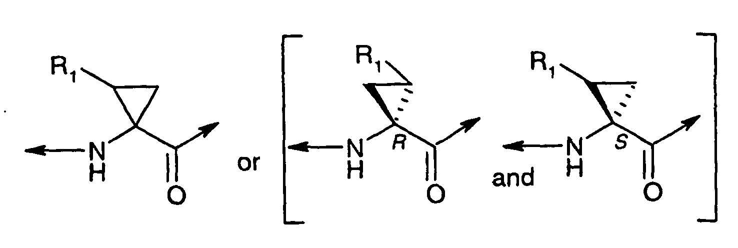

In turn, the racemic mixtures can be separated into individual optical isomers.

A most interesting finding of this invention pertains to the spatial orientation of the P1 segment. The finding concerns the configuration of the asymmetric carbon at position 1. A preferred embodiment is one wherein asymmetric carbon at position 1 has the *R* configuration.

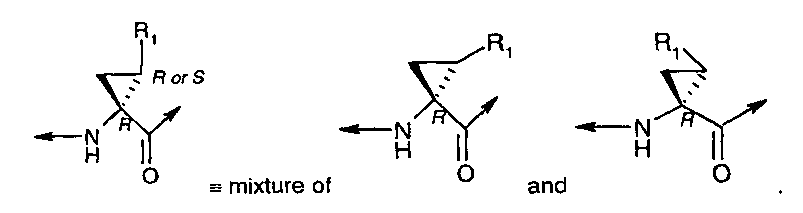

More explicitly, when carbon 1 has the R configuration, HCV NS3 protease inhibition is further enhanced by the position of the substituent **R**_{**1**} (e.g. alkyl or alkylene) at carbon 2 of the cyclopropyl ring. A most preferred compound is an optical isomer having the **R**_{**1**} substituent and the carbonyl in a *syn* orientation in the following absolute configuration:

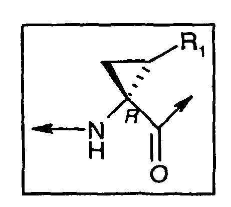

In the case where **R**_{**1**} is ethyl, for example, the asymmetric carbon atoms at positions 1 and 2 have the *R,R* configuration.

By way of illustrating the role of the absolute configuration of the substituent on the level of potency of the compound, compound **112** (Table 1) having the absolute configuration as *1R,2R*, has an IC₅₀ of 1.6 µM whereas the corresponding *1S,2S* isomer (compound **113**) has an IC₅₀ of 27.5 µM. Therefore, the *1R,2R* isomer is 25 fold more potent than the corresponding *1S,2S* isomer.

Further included in the scope of the invention are compounds of formula I, wherein **B** is H, lower alkyl-C(O)- or Het-C(O)-;
**R**_{**6**}**,** when present, is the side chain of Asp or Glu;
**R**_{**5**}**,** when present, is the side chain of D- or L-: Asp, Glu, Val, or Tbg;
**Y** is H or methyl;
**R**_{**4**} is the side chain of Val, Chg, Tbg, Ile or Leu;
**R**_{**3**} is the side chain of Ile, Chg, Val or Tbg;
**R**_{**2**} is 1-naphthylmethoxy, 2-naphthylmethoxy, O-Bn, 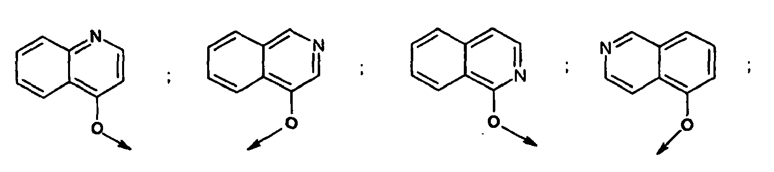 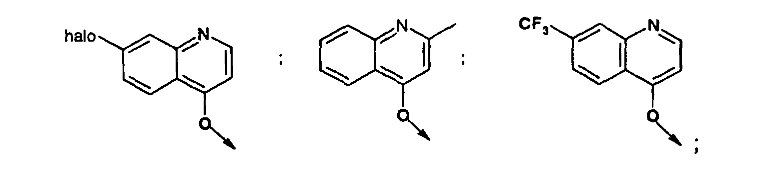 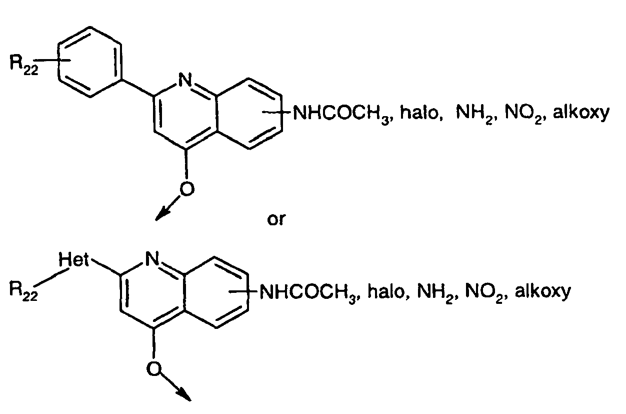
and **R**_{**22**} is amino; di(lower alkyl)amino; (lower alkyl)amide; NO₂; OH; halo; CF₃; or carboxy;
P1 is a cyclopropyl ring system of formula 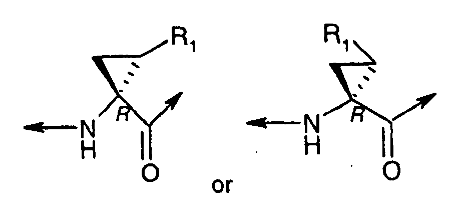
wherein **R**_{**1**} is ethyl, vinyl or bromovinyl; and
**W** is hydroxy or **N(R**_{**13a**}**)R**_{**13b**} wherein **R**_{**13a**} and **R**_{**13b**} are independently H, aryl or C₁₋₆ alkyl optionally substituted with hydroxy or phenyl; or a pharmaceutically acceptable salt or ester thereof.
A further preferred group of compounds is represented by formula I wherein **B** is H, acetyl or Het-C(O)-; **R**_{**6**}**,** when present, is the side chain of Asp; **R**_{**5**}, when present, is the side chain of D-Asp, D-Glu or D-Val; **Y** is H; **R**_{**4**} is the side chain of Chg or Ile; **R**_{**3**} is the side chain of Val, Chg or Tbg; **R**_{**2**} is 1-naphthylmethoxy, benzyloxy, 4-quinolinoxy, or 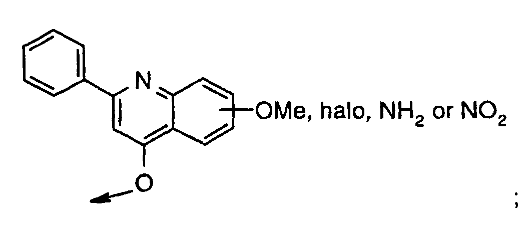
**P1** is a cyclopropyl ring system of formula 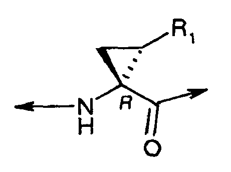
wherein **R**_{**1**} is Et or -CH=CH₂ or -CH=CHBr; and
**W** is hydroxy or -NH-(S)CH(Me)Ph,
or a pharmaceutically acceptable salt or ester thereof.

An even further preferred group of compounds is represented by formula I wherein **B** is acetyl; **R**_{**6**}**,** when present, is the side chain of Asp; **R**_{**5**}**,** when present, is the side chain of D-Glu; **Y** is H; **R**_{**4**} is the side chain of Chg; **R**_{**3**} is the side chain of Val or Tbg; **R**_{**2**} is :

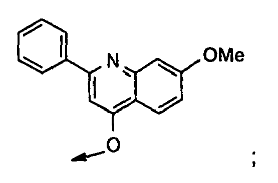

**P1** is: 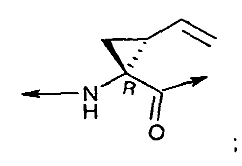 and
**W** is hydroxy, or a pharmaceutically acceptable salt or ester thereof.

Finally, included in the scope of the invention is each compound of formula I presented in Tables 1 to 5.

According to an alternate embodiment, the pharmaceutical compositions of this invention may additionally comprise another anti-HCV agent. Examples of anti-HCV agents include α- or β-interferon, ribavirin and amantadine.

According to another alternate embodiment, the pharmaceutical compositions of this invention may additionally comprise other inhibitors of HCV protease.

According to yet another alternate embodiment, the pharmaceutical compositions of this invention may additionally comprise an inhibitor of other targets in the HCV life cycle, including but not limited to, such as helicase, polymerase, metalloprotease or internal ribosome entry site (IRES).

The pharmaceutical compositions of this invention may be administered orally, parenterally or via an implanted reservoir. Oral administration or administration by injection is preferred. The pharmaceutical compositions of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intra-articular, intrasynovial, intrasternal, intrathecal, and intralesional injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example Tween® 80) and suspending agents.

The pharmaceutical compositions of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added.

Other suitable vehicles or carriers for the above noted formulations and compositions can be found in standard pharmaceutical texts, e.g. in "Remington's Pharmaceutical Sciences", The Science and Practice of Pharmacy, 19^{th} Ed. Mack Publishing Company, Easton, Penn., (1995).

Dosage levels of between about 0.01 and about 100 mg/kg body weight per day, preferably between about 0.5 and about 75 mg/kg body weight per day of the protease inhibitor compounds described herein are useful in a monotherapy for the prevention and treatment of HCV mediated disease. Typically, the pharmaceutical compositions of this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (*w*/*w*). Preferably, such preparations contain from about 20% to about 80% active compound.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician. Generally, treatment is initiated with small dosages substantially less than the optimum dose of the peptide. Thereafter, the dosage is increased by small increments until the optimum effect under the circumstances is reached. In general, the compound is most desirably administered at a concentration level that will generally afford antivirally effective results without causing any harmful or deleterious side effects.

When the compositions of this invention comprise a combination of a compound of formula I and one or more additional therapeutic or prophylactic agent, both the compound and the additional agent should be present at dosage levels of between about 10 to 100%, and more preferably between about 10 and 80% of the dosage normally administered in a monotherapy regimen.

When these compounds or their pharmaceutically acceptable salts are formulated together with a pharmaceutically acceptable carrier, the resulting composition may be administered *in vivo* to mammals, such as man, to inhibit HCV NS3 protease or to treat or prevent HCV virus infection. Such treatment may also be achieved using the compounds of this invention in combination with agents which include, but are not limited to: immunomodulatory agents, such as α-, β-, or γ-interferons; other antiviral agents such as ribavirin, amantadine; other inhibitors of HCV NS3 protease; inhibitors of other targets in the HCV life cycle, which include but not limited to, helicase, polymerase, metalloprotease, or internal ribosome entry site (IRES); or combinations thereof. The additional agents may be combined with the compounds of this invention to create a single dosage form. Alternatively these additional agents may be separately administered to a mammal as part of a multiple dosage form.

Accordingly, this invention provides methods of inhibiting HVC NS3 protease activity in mammals by administering a compound of the formula I, wherein the substituents are as defined above.

Preferred methods are useful in decreasing HCV NS3 protease activity in a mammal. If the pharmaceutical composition comprises only a compound of this invention as the active component, such methods may additionally comprise the step of administering to said mammal an agent selected from an immunomodulatory agent, an antiviral agent, a HCV protease inhibitor, or an inhibitor of other targets in the HCV life cycle such as helicase, polymerase, metalloprotease or IRES. Such additional agent may be administered to the mammal prior to, concurrently with, or following the administration of the compositions of this invention.

Alternate methods are useful for inhibiting viral replication in a mammal. Such methods are useful in treating or preventing HCV disease. If the pharmaceutical composition comprises only a compound of this invention as the active component, such methods may additionally comprise the step of administering to said mammal an agent selected from an immunomodulatory agent, an antiviral agent, a HCV protease inhibitor, or an inhibitor of other targets in the HCV life cycle. Such additional agent may be administered to the mammal prior to, concurrently with, or following the administration of the composition according to this invention.

The compounds set forth herein may also be used as laboratory reagents. The compounds of this invention may also be used to treat or prevent viral contamination of materials and therefore reduce the risk of viral infection of laboratory or medical personnel or patients who come in contact with such materials (e.g. blood, tissue, surgical instruments and garments, laboratory instruments and garments, and blood collection apparatuses and materials).

The compounds set forth herein may also be used as research reagents. The compounds of this invention may also be used as positive control to validate surrogate cell-based assays or *in vitro* or *in vivo* viral replication assays.

### PROCESS

The compounds of the present invention were synthesized according to the process as illustrated in scheme I (wherein CPG is a carboxyl protecting group and APG is an amino protecting group):

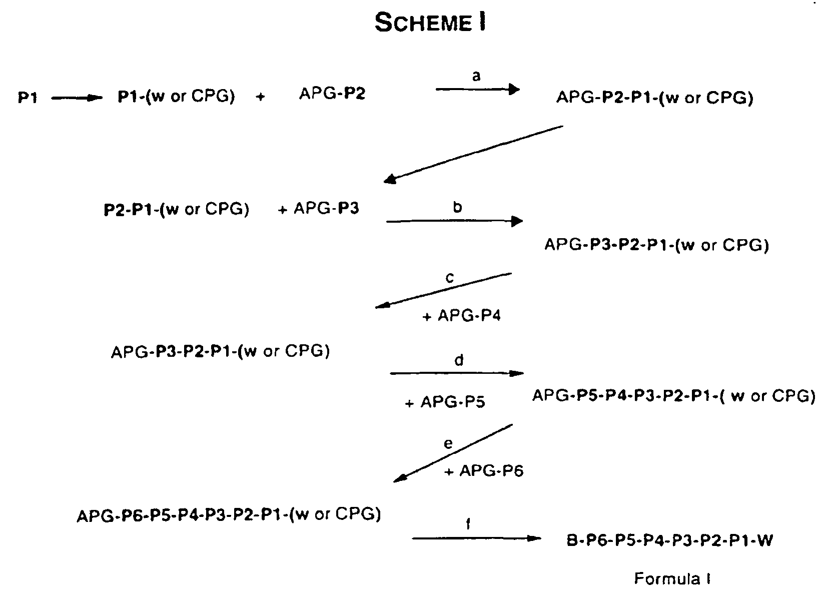

Briefly, the P1, P2, P3, P4, and optionally P5 and P6 can be linked by well known peptide coupling techniques. The P1, P2, P3, P4, and P5 and P6 groups may be linked together in any order as long as the final compound corresponds to peptides of formula I. For example, P6 can be linked to P5 to give P5-P6 that is linked to P4-P3-P2-P1 ; or P6 linked to P5-P4-P3-P2 then linked to an appropriately C-terminal protected P1.

Generally, peptides are elongated by deprotecting the α-amino group of the N-terminal residue and coupling the unprotected carboxyl group of the next suitably N-protected amino acid through a peptide linkage using the methods described. This deprotection and coupling procedure is repeated until the desired sequence is obtained. This coupling can be performed with the constituent amino acids in stepwise fashion, as depicted in Scheme I, or by condensation of fragments (two or several amino acids), or combination of both processes, or by solid phase peptide synthesis according to the method originally described in Merrifield, J. Am. Chem. Soc. (1963), 85, 2149-2154.

Coupling between two amino acids, an amino acid and a peptide, or two peptide fragments can be carried out using standard coupling procedures such as the azide method, mixed carbonic-carboxylic acid anhydride (isobutyl chloroformate) method, carbodiimide (dicyclohexylcarbodiimide, diisopropylcarbodiimide, or water-soluble carbodiimide) method, active ester (p-nitrophenyl ester, N-hydroxysuccinic imido ester) method, Woodward reagent K-method, carbonyldiimidazole method, phosphorus reagents or oxidation-reduction methods. Some of these methods (especially the carbodiimide method) can be enhanced by adding 1-hydroxybenzotriazole. These coupling reactions can be performed in either solution (liquid phase) or solid phase.

More explicitly, the coupling step involves the dehydrative coupling of a free carboxyl of one reactant with the free amino group of the other reactant in the presence of a coupling agent to form a linking amide bond. Descriptions of such coupling agents are found in general textbooks on peptide chemistry, for example, M. Bodanszky, "Peptide Chemistry", 2nd rev ed., Springer-Verlag, Berlin, Germany, (1993). Examples of suitable coupling agents are *N*,*N*'-dicyclohexylcarbodiimide, 1-hydroxybenzotriazole in the presence of *N,N'*-dicyclohexylcarbodiimide or *N*-ethyl-*N'-*[(3-dimethylamino)propyl]carbodiimide. A very practical and useful coupling agent is the commercially available (benzotriazol-1-yloxy)tris-(dimethylamino)phosphonium hexafluorophosphate, either by itself or in the presence of 1-hydroxybenzotriazole. Another very practical and useful coupling agent is commercially available 2-(1H-benzotriazol-1-yl)-*N,N,N'*,*N'*-tetramethyluronium tetrafluoroborate. Still another very practical and useful coupling agent is commercially available O-(7-azabenzotriazol-1-yl)-*N,N,N',N'*-tetramethyluronium hexafluorophosphate.

The coupling reaction is conducted in an inert solvent, e.g. dichloromethane, acetonitrile or dimethylformamide. An excess of a tertiary amine, e.g. diisopropylethylamine, *N*-methylmorpholine or N-methylpyrrolidine, is added to maintain the reaction mixture at a pH of about 8. The reaction temperature usually ranges between 0°C and 50°C and the reaction time usually ranges between 15 min and 24 h.

When a solid phase synthetic approach is employed, the C-terminal carboxylic acid is attached to an insoluble carrier (usually polystyrene). These insoluble carriers contain a group that will react with the carboxylic group to form a bond that is stable to the elongation conditions but readily cleaved later. Examples of which are: chloro- or bromomethyl resin, hydroxymethyl resin, and aminomethyl resin. Many of these resins are commercially available with the desired C-terminal amino acid already incorporated. Alternatively, the amino acid can be incorporated on the solid support by known methods Wang, S.-S., J. Am. Chem. Soc., (1973), 95, 1328; Atherton, E.; Shepard, R.C. "Solid-phase peptide synthesis; a practical approach" IRL Press: Oxford, (1989); 131-148. In addition to the foregoing, other methods of peptide synthesis are described in Stewart and Young, "Solid Phase Peptide Synthesis", 2^{nd} ed., Pierce Chemical Co., Rockford, IL (1984); Gross, Meienhofer, Udenfriend, Eds., "The Peptides: Analysis, Synthesis, Biology". Vol. 1, 2, 3, 5, and 9, Academic Press, New-York, (1980-1987); Bodansky et al., "The Practice of Peptide Synthesis" Springer-Veriag, New-York (1984).

The functional groups of the constituent amino acids generally must be protected during the coupling reactions to avoid formation of undesired bonds. The protecting groups that can be used are listed in Greene, "Protective Groups in Organic Chemistry", John Wiley & Sons, New York (1981) and "The Peptides: Analysis, Synthesis, Biology", Vol. 3, Academic Press, New York (1981).

The α-carboxyl group of the C-terminal residue is usually protected as an ester (CPG) that can be cleaved to give the carboxylic acid. Protecting groups that can be used include: 1) alkyl esters such as methyl, trimethylsilylethyl and *t*-butyl, 2) aralkyl esters such as benzyl and substituted benzyl, or 3) esters that can be cleaved by mild base treatment or mild reductive means such as trichloroethyl and phenacyl esters.

The α-amino group of each amino acid to be coupled to the growing peptide chain must be protected (APG). Any protecting group known in the art can be used. Examples of such groups include: 1) acyl groups such as formyl, trifluoroacetyl, phthalyl, and *ρ*-toluenesulfonyl; 2) aromatic carbamate groups such as benzyloxycarbonyl (Cbz or Z) and substituted benzyloxycarbonyls, and 9-fluorenylmethyloxycarbonyl (Fmoc); 3) aliphatic carbamate groups such as *tert-*butyloxycarbonyl (Boc), ethoxycarbonyl, diisopropylmethoxycarbonyl, and allyloxycarbonyl; 4) cyclic alkyl carbamate groups such as cyclopentyloxycarbonyl and adamantyloxycarbonyl; 5) alkyl groups such as triphenylmethyl and benzyl; 6) trialkylsilyl such as trimethylsilyl; and 7) thiol containing groups such as phenylthiocarbonyl and dithiasuccinoyl. The preferred α-amino protecting group is either Boc or Fmoc. Many amino acid derivatives suitably protected for peptide synthesis are commercially available.

The α-amino protecting group of the newly added amino acid residue is cleaved prior to the coupling of the next amino acid. When the Boc group is used, the methods of choice are trifluoroacetic acid, neat or in dichloromethane, or HCl in dioxane or in ethyl acetate. The resulting ammonium salt is then neutralized either prior to the coupling or *in situ* with basic solutions such as aqueous buffers, or tertiary amines in dichloromethane or acetonitrile or dimethylformamide. When the Fmoc group is used, the reagents of choice are piperidine or substituted piperidine in dimethylformamide, but any secondary amine can be used. The deprotection is carried out at a temperature between 0°C and room temperature (RT), usually 20-22°C.

Any of the amino acids having side chain functionalities must be protected during the preparation of the peptide using any of the above-described groups. Those skilled in the art will appreciate that the selection and use of appropriate protecting groups for these side chain functionalities depend upon the amino acid and presence of other protecting groups in the peptide. The selection of such protecting groups is important in that the group must not be removed during the deprotection and coupling of the α-amino group.

For example, when Boc is used as the α-amino protecting group, the following side chain protecting group are suitable: p-toluenesulfonyl (tosyl) moieties can be used to protect the amino side chain of amino acids such as Lys and Arg; acetamidomethyl, benzyl (Bn), or *t*-butylsulfonyl moieties can be used to protect the sulfide containing side chain of cysteine; benzyl (Bn) ethers can be used to protect the hydroxy containing side chains of serine, threonine or hydroxyproline; and benzyl esters can be used to protect the carboxy containing side chains of aspartic acid and glutamic acid.

When Fmoc is chosen for the α-amine protection, usually *tert*-butyl based protecting groups are acceptable. For instance, Boc can be used for lysine and arginine, *tert-*butyl ether for serine, threonine and hydroxyproline, and *tert*-butyl ester for aspartic acid and glutamic acid. Triphenylmethyl (Trityl) moiety can be used to protect the sulfide containing side chain of cysteine.

When **W** is an amide (**w**), P1 is coupled to an appropriate amine prior to the coupling to P2. Such amination will be readily recognized by persons skilled in the art.

Once the elongation of the peptide is completed all of the protecting groups are removed. When a liquid phase synthesis is used, the protecting groups are removed in whatever manner is dictated by the choice of protecting groups. These procedures are well known to those skilled in the art.

When a solid phase synthesis is used, the peptide is cleaved from the resin simultaneously with the removal of the protecting groups. When the Boc protection method is used in the synthesis, treatment with anhydrous HF containing additives such as dimethyl sulfide, anisole, thioanisole, or *p*-cresol at 0°C is the preferred method for cleaving the peptide from the resin. The cleavage of the peptide can also be accomplished by other acid reagents such as trifluoromethanesulfonic acid/trifluoroacetic acid mixtures. If the Fmoc protection method is used, the N-terminal Fmoc group is cleaved with reagents described earlier. The other protecting groups and the peptide are cleaved from the resin using solution of trifluoroacetic acid and various additives such as anisole, etc.

### Synthesis of capping group B and P6, P5, P4, and P3 moieties

Different capping groups **B** are introduced to protected P4, P5 or P6 or to any peptide segment with an appropriate acyl chloride or sulfonyl chloride that is either commercially available or for which the synthesis is well known in the art.

Different **P6** to **P3** moieties are available commercially or the synthesis is well known in the art.

### 1.Synthesis of P2 moieties.

### 1.1 Synthesis of precursors:

### A) Synthesis of haloarylmethane derivatives.

The preparation of halomethyl-8-quinoline **IId** was done according to the procedure of K.N. Campbell et al., J. Amer. Chem. Soc., (1946), 68, 1844.

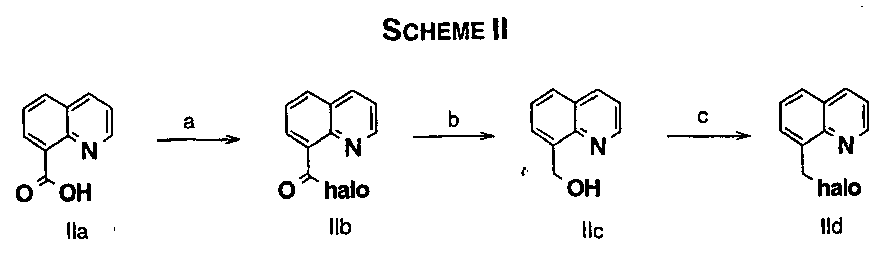

Briefly, 8-quinoline carboxylic acid **IIa** was converted to the corresponding alcohol **IIc** by reduction of the corresponding acyl halide **IIb** with a reducing agent such as lithium aluminium hydride. Treatment of alcohol **IIb** with the appropriate hydrohaloacid gives the desired halo derivative **IId**. A specific embodiment of this process is presented in Example 1A.

### B) Synthesis of aryl alcohols derivatives:

2-phenyl-4-hydroxyquinoline derivatives **IIIc** were prepared according to Giardina et al. (J. Med. Chem., (1997), 40, 1794-1807). 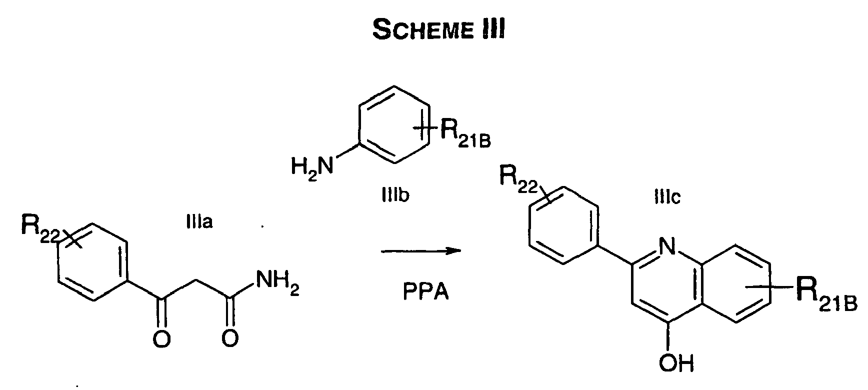
**R**_{**22**} **& R**_{**21B**} = alkyl, OH, SH, halo, NH₂, NO₂.

Benzoylacetamide (**IIIa**) was condensed with the appropriate aniline (**IIIb**) and the imine obtained was cyclized with polyphosphoric acid to give the corresponding 2-phenyl-4-hydroxyquinoline (**IIIc**)**.** A specific embodiment of this process is presented in Example 1B and 1C.

### 1.2. Synthesis of P2:

A) The synthesis of 4-substituted proline (wherein **R**^{**2**} is attached to the ring via a carbon atom) (with the stereochemistry as shown): 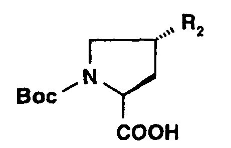
   is done as shown in Scheme IV according to the procedures described by J. Ezquerra et al. (Tetrahedron, (1993), 38, 8665-8678) and C. Pedregal et al. (Tetrahedron Lett., (1994), 35, 2053-2056). 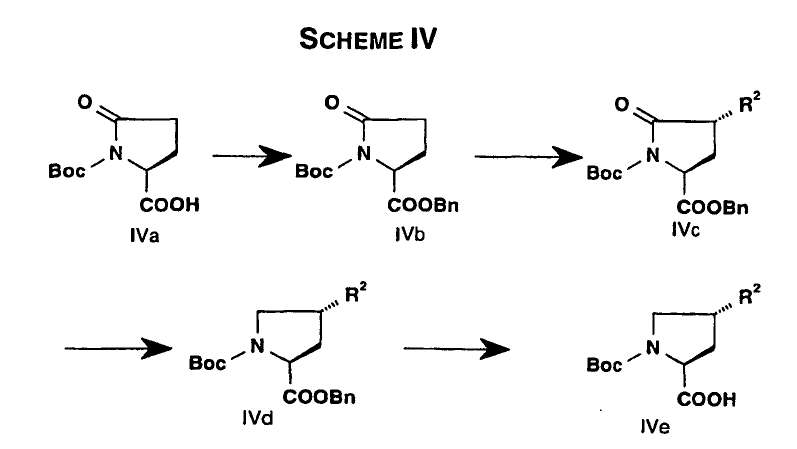
   Briefly, Boc-pyroglutamic acid is protected as a benzyl ester. Treatment with a strong base such as lithium diisopropylamide followed by addition of an alkylating agent (Br-**R**^{**20**} or **I-R**^{**20**}) gives the desired compounds **IVe** after reduction of the amide and deprotection of the ester.
B) The synthesis of O-aralkylated 4-(*R*)-hydroxyproline: 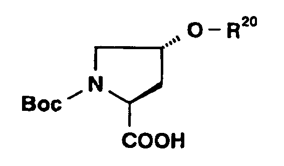
   When **R**^{**20**} is aryl, Het, aralkyl, or (lower alkyl)-Het, the process can be carried out according to the procedure described by E.M. Smith et al. (J. Med. Chem. (1988), 31, 875-885). Briefly, commercially available Boc-4(*R*)-hydroxyproline is treated with a base such as sodium hydride or K-*t*BuO and the resulting alkoxide reacted with an halo-**R**²⁰ (Br-**R**²⁰, I-**R**²⁰, etc..) to give the desired compounds. Specific embodiments of this process are presented in Examples 2, 3 and 4B.
C) Alternatively, when **R**^{**20**} is aryl or Het, the compounds can also be prepared via a Mitsunobu reaction (Mitsunobu (1981), Synthesis, January, 1-28; Rano *et al.,* (1995), Tet. Lett. 36(22), 3779-3792; Krchnak *et al.,* (1995), Tet. Lett. 36(5), 62193-6196; Richter *et al.,* (1994), Tet. Lett. 35(27), 4705-4706). Briefly, commercially available Boc-4(S)-hydroxyproline methyl ester is treated with the appropriate aryl alcohol or thiol in the presence of triphenylphosphine and diethylazodicarboxylate (DEAD) and the resulting ester is hydrolyzed to the acid. Specific embodiment of this process is presented in Example 4A.

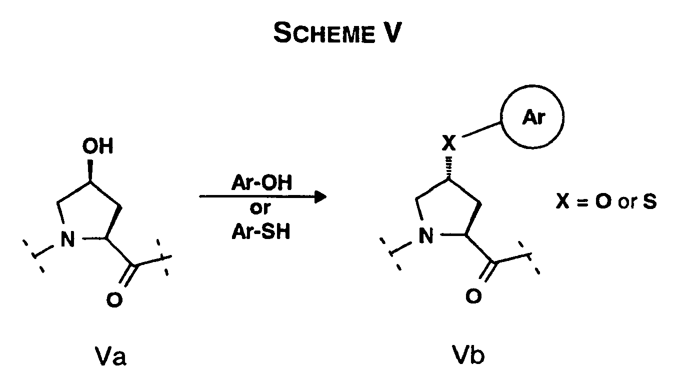

Alternatively, the Mitsunobu reaction can be produced in solid phase (Scheme V). The 96-well block of the Model 396 synthesizer (advanced ChemTech) is provided with aliquots of resin-bound compound (**Va**) and a variety of aryl alcohols or thiols and appropriate reagents are added. After incubation, each resin-bound product (**Vb**) is washed, dried, and cleaved from the resin.
A Suzuki reaction (Miyaura *et al*., (1981), Synth. Comm. 11, 513; Sato *et al*., (1989), Chem. Lett., 1405; Watanabe *et al*., (1992), Synlett., 207; Takayuki *et al*., (1993), J. Org. Chem. 58, 2201; Frenette *et al*., (1994), Tet. Lett. 35(49), 9177-9180; Guiles *et al*., (1996), J. Org. Chem. 61, 5169-5171) can also be used to further functionalize the aryl substituent.

### 2. Synthesis of P1 moieties (2-substituted 1-aminocyclopropyl carboxylic acid)

The synthesis was done according to scheme VI. 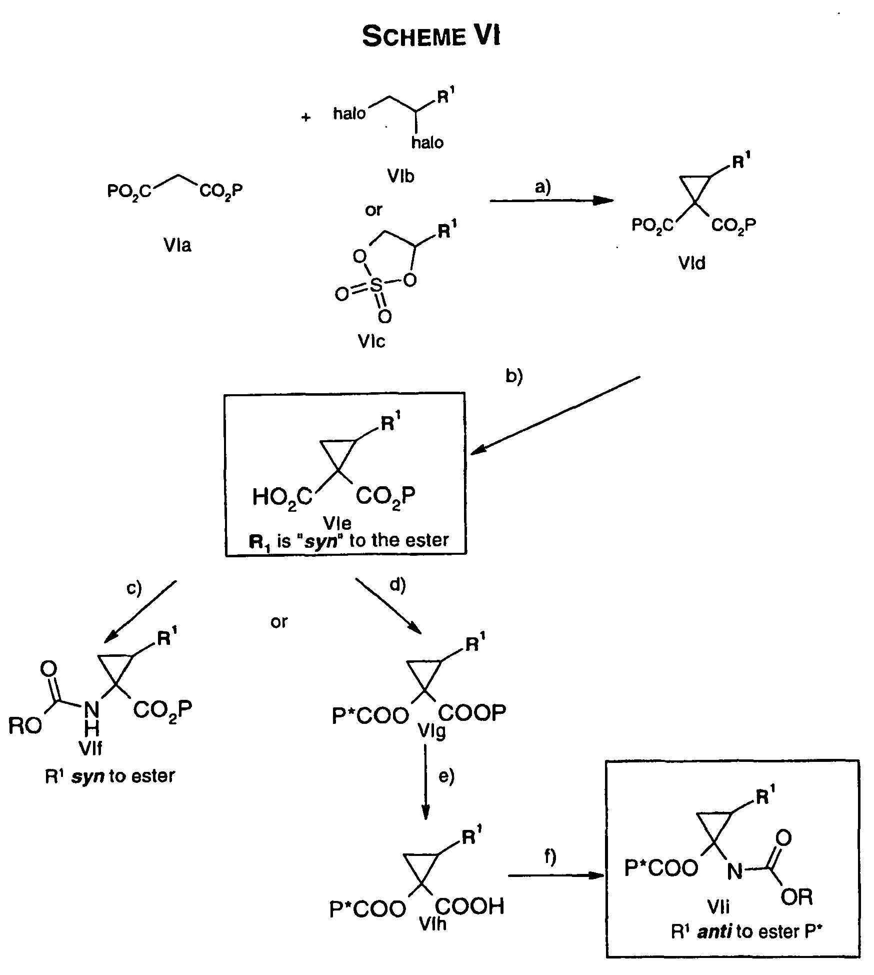
a) Briefly, di-protected malonate **VIa** and 1,2-dihaloalkane **VIb** or cyclic sulfate **VIc** (synthesized according to K. Burgess and Chun-Yen KE (Synthesis, (1996), 1463-1467) are reacted under basic conditions to give the diester **VId.**
b) A regioselective hydrolysis of the less hindered ester is performed to give the acid **VIe**.
c) This acid **VIe** is subjected to a Curtius rearrangement to give a racemic mixture of 1-aminocyclopropylcarboxylic acid derivatives **VIf** with **R**^{**1**} being *syn* to the carboxyl group. A specific embodiment for this synthesis is presented in Example 5.
d, e) Alternatively, selective ester formation from the acid **VIe** with an appropriate halide (P*CI) or alcohol (P*OH) forms diester **Vlg** in which the P* ester is compatible with the selective hydrolysis of the P ester. Hydrolysis of P ester provides acid **Vlh**.
f) A Curtius rearrangement on **Vlh** gives a racemic mixture of 1-aminocyclopropylcarboxylic acid derivatives **VIi** with R¹ group being *anti* to the carboxyl group. A specific embodiment for this synthesis is presented in Example 10.

An alternative synthesis for the preparation of derivatives **Vlf** (when R¹ is vinyl, *syn* to the carboxyl group) is described below.

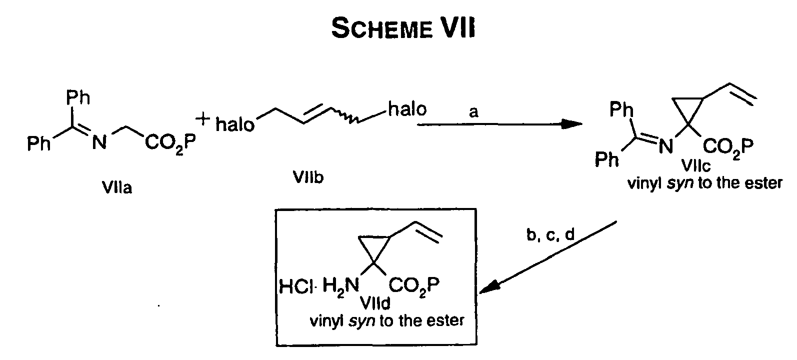

Treatment of commercially available imine **VIIa** with 1,4-dihalobutene **Vllb** in presence of a base produces, after hydrolysis of the resulting imine **VIIc, VIId** having the allyl substituent *syn* to the carboxyl group This process is presented in Example 11.

Resolution of all of the above enantiomeric mixtures at carbon 1 (VIe and VIId) can be carried out via:
1) enzymatic separation (Examples 9 and 13);
2) crystallization with a chiral acid (Example 14); or
3) chemical derivatization (Example 6).

Following resolution, determination of the absolute stereochemistry can be carried out as presented in Example 7.

Resolution and stereochemistry determination can be carried out in the same manner for the enantiomeric mixtures at carbon 1 wherein the substituent at C2 is *anti* to the carboxyl group (Vii).

Accordingly, the invention further comprises a process for the preparation of a peptide analog of formula (I) wherein P1 is a substituted aminocyclopropyl carboxylic acid residue, comprising the step of:
coupling a peptide selected from the group consisting of: APG-P6-P5-P4-P3-P2; APG-P5-P4-P3-P2; APG-P4-P3-P2; APG-P3-P2; and APG-P2;
with a P1 intermediate of formula: 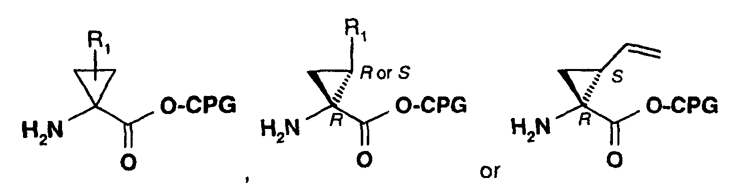
   wherein **R**_{**1**} is C₁₋₆ alkyl or C₂₋₆ alkenyl optionally substituted with halogen, CPG is a carboxyl protecting group and APG is an amino protecting group, and P6 to P2 are as defined above.

Finally, the invention also comprises the use of an intermediate of formula: 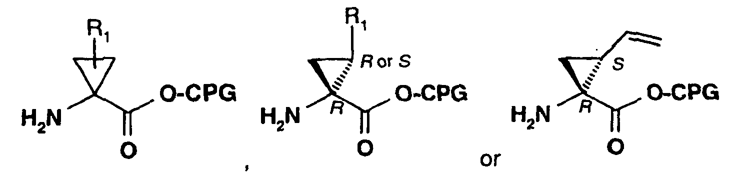
wherein **R**_{**1**} is C₁₋₆ alkyl or C₂₋₆ alkenyl optionally substituted with halogen, for the preparation of a compound of formula I as defined above.

### EXAMPLES

The present invention is illustrated in further detail by the following non-limiting examples.

Temperatures are given in degrees Celsius. Solution percentages express a weight to volume relationship, and solution ratios express a volume to volume relationship, unless stated otherwise. Nuclear magnetic resonance (NMR) spectra were recorded on a Bruker 400 MHz spectrometer; the chemical shifts (δ) are reported in parts per million. Flash chromatography was carried out on silica gel (SiO2) according to Still's flash chromatography technique (W.C. Still et al., J. Org. Chem. (1978), 43, 2923).

Abbreviations used in the examples include Bn: benzyl; Boc: tert-butyloxycarbonyl {Me₃COC(O)}; BSA: bovine serum albumin; CHAPS: 3-[(3-cholamidopropyl)-dimethylammonio]-1-propanesulfonate; DBU: 1,8-diazabicyclo[5.4.0]undec-7-ene; CH₂Cl₂= DCM: methylene chloride; DEAD: diethylazodicarboxylate; DIAD: diisopropylazodicarboxylate; DIPEA: diisopropylethylamine; DMAP: dimethylaminopyridine; DCC: 1,3-dicyclohexylcarbodiimide; DME: 1,2-dimethyoxyethane; DMF: dimethylformamide; DMSO: dimethylsulfoxide; DTT: dithiothreitol or threo-1,4-dimercapto-2,3-butanediol; DPPA: diphenylphosphoryl azide; EDTA: ethylenediaminetetraacetic acid; Et: ethyl; EtOH: ethanol; EtOAc: ethyl acetate; Et₂O: diethyl ether; HATU: [O-7-azabenzotriazol-1-yl)-1,1,3,3-tetramethyluronium hexafluorophosphate]; HPLC: high performance liquid chromatography; MS: mass spectrometry (MALDI-TOF: Matrix Assisted Laser Disorption Ionization-Time of Flight, FAB: Fast Atom Bombardment); LAH: lithium aluminum hydride; Me: methyl; MeOH: methanol; MES: (2-{N-morpholino}ethanesulfonic acid); NaHMDS: sodium bis(trimethylsilyl)amide; NMM: N-methylmorpholine; NMP: N-methylpyrrolidine; Pr: propyl; Succ: 3-carboxypropanoyl; PNA: 4-nitrophenylamino or p-nitroaniline; TBAF: tetra-n-butylammonium fluoride; TBTU: 2-(1H-benzotriazole-1-yl)-1,1,3,3-tetramethyluronium tetrafluoroborate; TCEP: tris(2-carboxyethyl) phosphine hydrochloride; TFA: trifluoroacetic acid; THF: tetrahydrofuran; TIS: triisopropylsilane; TLC: thin layer chromatography; TMSE: trimethylsilylethyl; Tris/HCl: tris(hydroxymethyl)aminomethane hydrochloride.

### P2 BUILDING BLOCKS

### EXAMPLE 1A

### Synthesis of bromomethyl-8-quinoline (1 A):

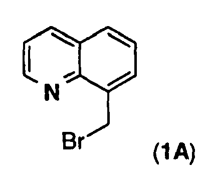

To commercially available 8-quinoline carboxylic acid (2.5 g, 14.4 mmol) was added neat thionyl chloride (10 ml, 144 mmol). This mixture was heated at 80°C for 1 h before the excess thionyl chloride was distilled off under reduced pressure. To the resulting brownish solid was added absolute EtOH (15 mL) which was heated at 80°C for 1 h before being concentrated *in vacuo*. The residue was partitioned between EtOAc and saturated aqueous NaHCO₃, and the organic phase dried (MgSO₄), filtered and concentrated to give a brownish oil (2.8 g). This material (ca. 14.4 mmol) was added dropwise over 35 min to a LAH (0.76 g, 20.2 mmol)/Et₂O suspension which was cooled to -60°C. The reaction mixture was slowly warmed to -35°C over 1.5 h before the reaction was complete. The reaction was quenched with MgSO₄.10H₂O slowly over 30 min and then wet THF. The mixture was partitioned between Et₂O and 10% aqueous NaHCO₃.The organic phase was dried (MgSO₄), filtered and concentrated to give a yellowish solid (2.31 g, 80% over 2 steps) corresponding to the alcohol. The alcohol (2.3 g, 11.44 mmol) was dissolved in AcOH/HBr (20 mL, 30% solution from Aldrich) and heated at 70°C for 2.5 h. The mixture was concentrated *in vacuo* to dryness, partitioned between EtOAc (100 mL) and saturated aqueous NaHCO₃ before being dried (MgSO₄), filtered and concentrated to give the desired compound (**1A**) as a brownish solid (2.54 g, 100%).

### EXAMPLE 1B

### Synthesis of 2-phenyl-4-hydroxyquinoline (1B):

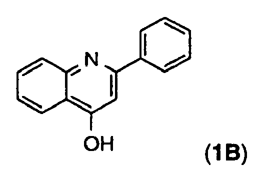

Commercially available ethyl benzoylacetate (6.00 g, 31.2 mmol) was heated at 85°C (sealed tube) in 75 mL of 30% NH₄OH for 2 hours. The solid formed upon cooling was filtered and refluxed in water for 2 hours. The solution was extracted three times with CH₂Cl₂. The organic layers were combined, dried over MgSO₄, filtered and concentrated. The yellow residue was flash chromatographed on silica gel, eluting with EtOAc:hexane (3:7), to give the corresponding amide as a white solid, 1.60 g, 31 % yield.

This amide (250 mg, 1.53 mmol) was refluxed using a Dean-Stark apparatus with aniline (143 mg, 1.53 mmol) and aniline•HCl (10 mg, 0.08 mmol) in toluene (10 mL) for 16 h. The solution was concentrated to afford a brown oil that was mixed with polyphosphoric acid (2 g) and heated at 135°C for 20 min. The reaction mixture was poured into water and adjusted to pH 8 with 5 M NaOH. The aqueous suspension was extracted twice with ethyl acetate. The organic layers were combined, washed with brine, dried over MgSO₄, filtered and concentrated. The residue was flash chromatographed on silica gel, eluting with 3% MeOH in ethyl acetate, to give 2-phenyl-4-hydroxyquinoline (**1B**), 67 mg, 20% yield.

¹H NMR (DMSO-d₆) δ 8.11 (d, J = 7 Hz, 1 H), 7.86-7.83 (m, 2 H), 7.77 (d, J = 8 Hz, 1 H), 7.68 (dd, J = 8, 7 Hz, 1 H), 7.61-7.58 (m, 3 H), 7.35 (dd, J = 8, 7 Hz, 1 H), 6.34 (s, 1 H).

### EXAMPLE 1C

### Synthesis of 4-hydroxy-2-phenyl -7-methoxyquinoline (1C)

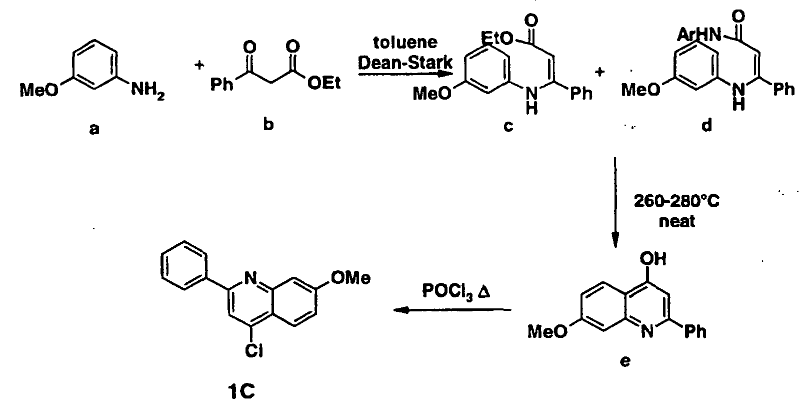

### 4-hydroxy-2-phenyl -7-methoxyquinoline (e):

A solution of ethyl benzoylacetate (**b**) (100.0 g, 0.52 mol), m-anisidine (**a**) (128.1 g, 1.04 mol) and 4N HCl / dioxane (5.2 mL) in toluene (1.0 L) was refluxed for 6.25 h in a Dean-Stark apparatus. The cooled toluene solution was successively washed with aqueous 10% HCl (2 × 300 mL), 1N NaOH (2 × 300 mL), H₂O (300 mL) and brine (150 mL). The toluene phase was dried (MgSO₄), filtered and concentrated under reduced pressure to give a 1.2:1.0 mixture of ester c and amide d (144.6 g, 45% / 38% crude yield) as a dark brown oil. The crude oil was heated to 280 °C for 80 min while distilling generated EtOH. The cooled dark solid obtained was triturated with CH₂Cl₂ (200 mL). The suspension was filtered and the resulting solid washed with CH₂Cl₂ to give **e** (22.6 g, 17% from a) as a beige solid: ¹H NMR (DMSO-d₆) δ 8.00 (d, J = 9.0 Hz, 1H), 7.81-7.82 (m, 2H), 7.57-7.59 (m, 3H), 7.20 (d, J = 2.2 Hz, 1H), 6.94 (dd, J = 9.0, 2.2 Hz, 1H), 6.26 (s, 1H), 3.87 (s, 3H).

### 4-Chloro-2-phenyl-7-methoxyquinoline (1C):

A suspension of e (8.31 g, 33.1 mmol) in POCl₃ (90 mL) was heated to reflux for 2 h (clear solution obtained upon heating). The reaction mixture was concentrated under reduced pressure. The residue was partitioned between 1N NaOH (exothermic, 10N NaOH added to maintain high pH) and EtOAc (500 mL). The organic layer was washed with H₂O (100 mL) and brine (100 mL) then was dried (MgSO₄), filtered and concentrated under reduced pressure to give 1C (8.60 g, 96%) as a pale yellow solid: ¹H NMR (DMSO-d₆) δ 8.28-8.30 (m, 2H), 8.20 (s, 1H), 8.10 (d, J = 9.1 Hz, 1H), 7.54-7.58 (m, 3H), 7.52 (d, J = 2.5 Hz, 1H), 7.38 (dd, J = 9.1, 2.5 Hz, 1H), 3.98 (s, 3H). This reaction was repeated three times and always gave 96-98% yield which is significantly higher that the 68% yield reported in J. Med. Chem. 1997, 40, 1794.

### EXAMPLE 2

### Synthesis of Boc-4(R)-(naphthalen-1-ylmethoxy) proline (2):

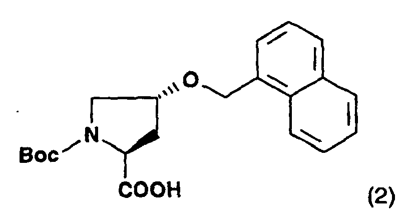

Commercially available Boc-4*(R)*-hydroxyproline (5.00 g, 21.6 mmol) was dissolved in THF (100 mL) and cooled to 0°C. Sodium hydride (60% dispersion in oil, 1.85 g, 45.4 mmol) was added portionwise over 10 minutes and the suspension was stirred at RT for 1 h. Then, 1-(bromamethyl)naphthalene (8.00 g, 36.2 mmol) (prepared as described in E.A. Dixon et al. Can. J. Chem., (1981), 59, 2629-2641) was added and the mixture was heated at reflux for 18 h. The mixture was poured into water (300 mL) and washed with hexane. The aqueous layer was acidified with 10% aqueous HCl and extracted twice with ethyl acetate. The organic layers were combined and washed with brine, dried (MgSO₄), filtered and concentrated. The residue was purified by flash chromatography (49:49:2 hexane: ethyl acetate: acetic acid) to give the title compound as a colorless oil (4.51 g, 56% yield). ¹H NMR (DMSO-d₆) indicated the presence of two rotamers: δ 8.05 (m, 1H), 7.94 (m, 1H), 7.29 (d, J=14 Hz, 1H), 7.55-7.45 (m, 4H), 4.96 (m, 2H), 4.26 (br. s, 1H), 4.12 (dd, J=J=8 Hz, 1H), 3.54-3.42 (m, 2H), 2.45-2.34 (m, 1H), 2.07-1.98 (m, 1H) 1.36 (s, (3/9) 9H), 1.34 (s, (6/9) 9H).

### EXAMPLE 3

### Synthesis of Boc-4(R)-(8-quinaline-methoxy) proline (3):

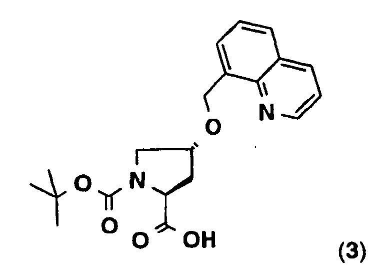

Boc-4(*R*)-hydroxyproline (1.96 g, 8.5 mmol) in anhydrous THF (20 mL) was added to a suspension of NaH (1.4 g, 60% in oil, 34 mmol) in THF (100 mL). This mixture was stirred 30 min before bromomethyl-8-quinoline from Example 1 A (2.54 g, 11.44 mmol) was added in THF (30 mL). The reaction mixture was heated at 70°C (5 h) before the excess NaH was destroyed carefully with wet THF. The reaction was concentrated *in vacuo* and the resulting material was dissolved in EtOAc and H₂O. The basic aqueous phase was separated and acidified with 10% aqueous HCl to pH ∼5 before being extracted with EtOAc (150 mL). The organic phase was dried (MgSO₄), filtered and concentrated to give a brown oil. Purification by flash chromatography (eluent: 10% MeOH/CHCl₃) gave the desired compound as a pale yellow solid (2.73 g, 86%). HPLC (97.5%); ¹H-NMR (DMSO-d₆) shows rotamer populations in a 6:4 ratio, δ 12-11.4 (bs, 1H). 8.92 (2 x d, J = 4.14 and 4.14 Hz, 1H), 8.38 (2 x d, J = 8.27 and 8.27 Hz, 1H), 7.91 (d, J = 7.94 Hz, 1H), 7.77 (d, J = 7.0 Hz, 1H), 7.63-7.54 (m, 2H), 5.14 (2 x s, 2H), 4.32-4.29 (m, 1H), 4.14-4.07 (m, 1H), 3.52-3.44 (m, 2H), 2.43-2.27 (m, 1H), 2.13-2.04 (m, 1H), 1.36 and 1.34 (2 x s, 9H).

### EXAMPLE 4A

### Preparation of Boc-4(R)-(7-chloroquinoline-4-oxo)proline (4A):

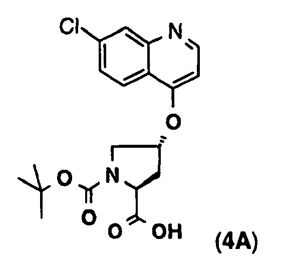

Commercially available Boc-4(*S*)-hydroxyproline methyl ester (500 mg, 2.04 mmol) and 7-chloro-4-hydroxyquinoline (440 mg, 2.45 mmol) were placed in dry THF (10 mL) at 0°C. Triphenylphosphine (641 mg, 2.95 mmol) was added, followed by slow addition of DIAD (426 mg, 2.45 mmol). The mixture was stirred at RT for 20 h. The reaction mixture was then concentrated, taken up in ethyl acetate and extracted three times with HCl 1N. The aqueous phase was basified with Na₂CO₃ and extracted twice with ethyl acetate. The organic layers were combined, dried over MgSO₄, filtered and concentrated to give a yellow oil. The oil was purified by flash chromatography to give compound 4A methyl ester as a white solid, 498 mg, 58% yield.

This methyl ester (400 mg, 0.986 mmol) was hydrolyzed with 1 M aqueous sodium hydroxide (1.7 mL, 1.7 mmol) in methanol (4 mL), at 0°C, for 3 h. The solution was concentrated to remove the methanol and neutralized with 1 M aqueous HCl. The suspension was concentrated to dryness and taken up in methanol (20 mL), the salts were filtered off and the filtrate concentrated to give the desired compound **4A** as a white solid, 387 mg, quant. yield.

¹H NMR (DMSO-d₆) (ca. 1:1 mixture of rotamers) δ 8.74 (d, J = 5 Hz, 1 H), 8.13-8.09 (m, 1 H), 7.99 and 7.98 (s, 1 H), 7.58 (d, J = 9 Hz, 1 H), 7.02 (d, J = 5 Hz, 1 H), 5.26-5.20 (m, 1 H), 4.10- 4.01 (m, 1 H), 3.81-3.72 (m, 1 H), 3.59 (dd, J = 12, 10 Hz, 1 H), 2.41-2.31 (m, 2 H), 1.34 and 1.31 (s, 9H).

### EXAMPLE 4B

### Synthesis of Boc-4(R)-(2-phenyl-7-methoxyquinoline-4-oxo) proline (4B):

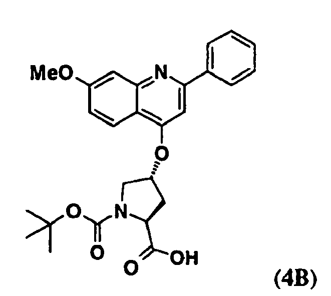

### 1-[(1,1-Dimethylethoxy)carbonyl]-4(R)-[(7-methoxy-2-phenyl-4-quinolinyl)oxy]-L-proline (4B):

Potassium tert-butoxide (8.16 g, 72.7 mmol) was added in small portions, over 15 min, to a solution of commercially available 4-(*S*)-hydroxyproline (6.73 g, 29.1 mmol) in DMSO (83 mL) maintained at 25°C. The mixture was stirred at 25°C for 1.5 h. Chloro-2-phenyl-7-methoxyquinoline **1C** (8.61 g, 32.0 mmol) was added in 4 portions over 15 min to the reaction mixture. The reaction mixture was stirred at 25°C for 19 h. The resulting suspension was poured in H₂O (650 mL) and the mixture was washed with Et₂O (3 x 150 mL) to remove excess chloroquinoline (EtOAc was later found to be more efficient). The aqueous layer was acidified with aqueous 1 N HCl (38 mL of calculated 1.5 equiv. required, 43.6 mL) to pH 4 - 5. The white solid that precipitated was recovered by filtration. The moist solid was dried under reduced pressure over P₂O₅ to give the proline derivative **4B** (12.6 g, 91%, contains 2.3% w/w of DMSO) as a beige solid:

¹H NMR (DMSO-d₆) δ (2:1 mixture of rotamers) 8.27 (d, J = 7.0 Hz, 2H), 8.00, 7.98 (2d, J = 9.2, -9.2 Hz, 1H), 7.48-7.56 (m, 3H), 7.45, 7.43 (2s, 1H), 7.39 (d, J = 2.5 Hz, 1H), 7.17 (dd, J = 9.2, 2.5 Hz, 1H), 5.53-5.59 (m, 1H), 4.34-4.41 (m, 1H), 3.93 (s, 3H), 3.76 (broad s, 2H), 2.63-2.73 (m, 1H), 2.32-2.43 (m, 1H), 1.36, 1.33 (2s, 9H).

### P1 BUILDING BLOCKS

### EXAMPLE 5

### Synthesis of mixture of (1R, 2R)/(1S, 2R) 1-amino-2-ethylcyclopropyl carboxylic acid

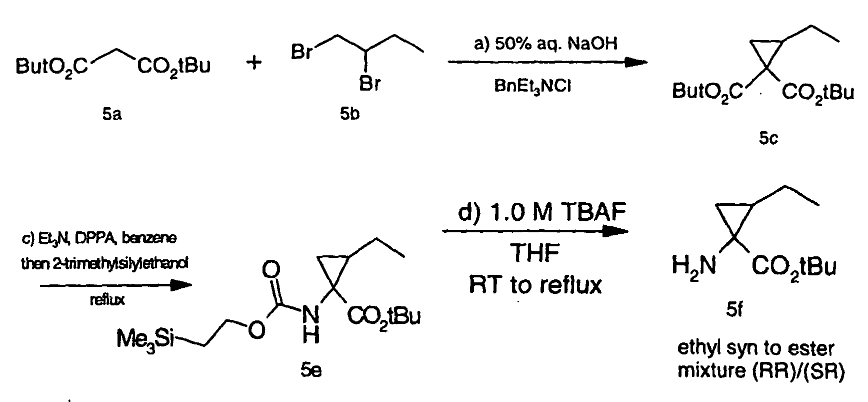

a) To a suspension of benzyltriethylammonium chloride (21.0 g, 92.19 mmol) in a 50% aqueous NaOH solution (92.4 g in 185 mL H₂O) were successively added di-*tert*-butylmalonate (20.0 g, 92.47 mmol) and 1,2-dibromobutane (30.0 g, 138.93 mmol). The reaction mixture was vigorously stirred overnight at RT, a mixture of ice and water was then added. The crude product was extracted with CH₂Cl₂ (3x) and sequentially washed with water (3x) and brine. The organic layer was dried (MgSO₄), filtered and concentrated. The residue was flash chromatographed (7 cm, 2 to 4 % Et₂O in hexane) to afford the desired cyclopropane derivative 5c (19.1 g, 70.7 mmol, 76% yield). ¹H NMR (CDCl₃) δ 1.78-1.70 (m, 1H), 1.47 (s, 9H), 1.46 (s, 9H), 1.44-1.39 (m, 1H), 1.26-1.64 (m, 3H), 1.02 (t, 3H, J= 7.6 Hz).
b) To a suspension of potassium *tert*-butoxide (6.71 g, 59.79 mmol, 4.4 eq.) in dry ether (100 mL) at 0°C was added H₂O (270 µL, 15.00 mmol, 1.1 eq.). After 5 min diester 5c (3.675 g, 13.59 mmol) in ether (10 mL) was added to the suspension. The reaction mixture was stirred overnight at RT, then poured in a mixture of ice and water and washed with ether (3x). The aqueous layer was acidified with a 10% aq. citric acid solution at 0°C and extracted with AcOEt (3x). The combined organic layer was successively washed with water (2x) and brine. After the usual treatment (Na₂SO₄, filtration, concentration), the desired acid **5d** was isolated as a pale yellow oil (1.86g, 8.68 mmol, 64% yield). ¹H NMR (CDCl₃) δ 2.09-2.01 (m, 1H), 1.98 (dd, J= 3.8, 9.2 Hz, 1H), 1.81- 1.70 (m, 1H), 1.66 (dd, J= 3.0, J= 8.2 Hz, 1H), 1.63-1.56 (m, 1H), 1.51 (s, 9H), 1.0 (t, J= 7.3 Hz, 3H).
c) To the acid 5d (2.017 g, 9.414 mmol) in dry benzene (32 mL) were successively added Et₃N (1.50 mL, 10.76 mmol, 1.14 eq.) and DPPA (2.20 mL, 10.21 mmol, 1.08 eq.). The reaction mixture was refluxed for 3.5 h then 2-trimethylsilylethanol (2.70 mL, 18.84 mmol, 2.0 eq.) was added. The reflux was maintained overnight then the reaction mixture was diluted with Et₂O and successively washed with a 10 % aqueous citric acid solution, water, saturated aqueous NaHCO₃, water (2x) and brine. After the usual treatment (MgSO₄, filtration, concentration) the residue was purified by flash chromatography (5 cm, 10% AcOEt- hexane) to afford the desired carbamate 5e (2.60 g, 7.88 mmol, 84% yield) as a pale yellow oil. MS (FAB) 330 (MH⁺); ¹H NMR (CDCl₃) δ 5.1 (bs, 1H), 4.18-4.13 (m, 2H), 1.68-1.38 (m, 4H), 1.45 (s, 9H), 1.24-1.18 (m, 1H), 1.00-0.96 (m, 5H), 0.03 (s, 9H).
d) To carbamate 5e (258 mg, 0.783 mmol) was added a 1.0 M TBAF solution in THF (940 µL, 0.94 mmol, 1.2 eq.). After 4.5 h an additional amount of 1.0 M TBAF was added (626 µL, 0.63 mmol, 0.8 eq.). The reaction mixture was stirred overnight at RT, refluxed for 30 min and then diluted with AcOEt. The solution was successively washed with water (2x) and brine. After the usual treatment (MgSO₄, filtration and concentration) the desired amine **5f** was isolated (84 mg, 0.453 mmol, 58 % yield) as a pale yellow liquid. ¹H NMR (CDCl₃) δ 1.96 (bs, 2H), 1.60-1.40 (m, 2H), 1.47 (s, 9H), 1.31-1.20 (m, 1H), 1.14 (dd, J= 4.1, 7.3 Hz, 1H), 1.02 (dd, J= 4.1, 9.2 Hz, 1H), 0.94 (t, J= 7.3 Hz, 3H).

### EXAMPLE 6

### Chemical resolution of t-butyl-(1R,2R)/(1S,2R) 1-amino-2-ethylcyclopropyl carboxylate (from Example 5):

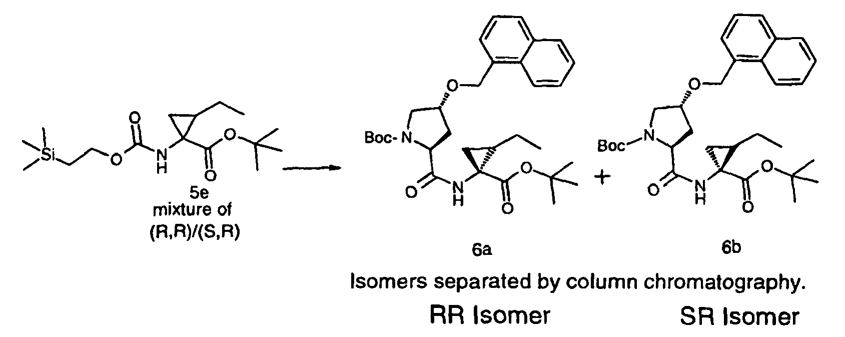

Compound **5e** from Example 5 (8.50 g , 25.86 mmol) was treated with 1M TBAF/THF (26 mL) at reflux for 45 min. The cooled reaction mixture was diluted with EtOAc, washed with water (3x) and brine (1 x), then, dried (MgSO₄), filtered and evaporated to provide the free amine as a light yellow oil. The free amine was dissolved in anhydrous CH₂Cl₂ (120 mL), NMM (8.5 mL, 77.57 mmol), compound **2** (Example 2) (10.08 g, 27.15 mmol) and HATU (11.79 g , 31.03 mmol) were added successively. The reaction mixture was stirred at RT overnight, then worked up as described previously. The crude diastereomeric mixture was separated by flash chromatography (eluent - hexane : Et₂O ; 25 : 75) to provide the dipeptide 6a (the less polar eluting spot) as a white foam (4.42 g ; 64% of the theoretical yield) and 6b (the more polar eluting spot) as an ivory foam (4 g., 57% of theoretical yield). At this time both isomers were separated but the absolute stereochemistry was still not known.

### EXAMPLE 7

### Determination of the absolute stereochemistry of compounds 6a and 6b by correlation with known t-butyl (1R-amino-2R-ethylcyclopropyl carboxylate

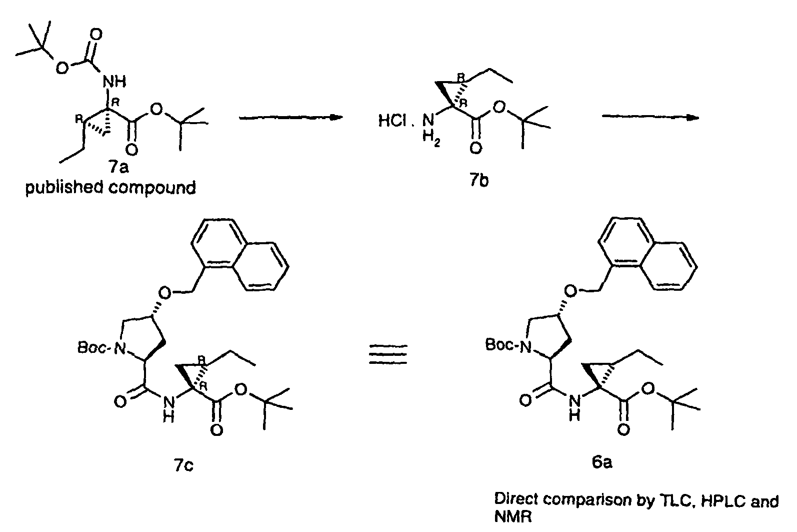

Prof . A. Charette , from the University of Montreal , provided compound **7a** having the absolute stereochemistry as shown, which was determined by X-ray crystallography (J. Am. Chem. Soc., 1995, 117, 12721) . Compound **7a** (13.2 mg , 0.046 mmol) was dissolved in 1 M HCl/EtOAc (240 µL) and stirred approximately 48 hours. The mixture was evaporated to dryness to provide compound 7b as a light yellow paste and was coupled to compound 2 (18 mg , 0.049 mmol) as described in Example 6, using NMM (20.3 µL, 0.185 mmol) and HATU (21.1 mg, 0.056 mmol) in CH₂Cl₂. The crude material was purified by flash chromatography (eluent - hexane : Et₂O ; 50:50) to provide the dipeptide 7c as an oil (7.7 mg ; 31%). By TLC, HPLC and NMR comparison , dipeptide 7c, was found to be identical to the less polar compound **6a** obtained in Example 6, thus identifying the absolute stereochemistry of **6a** as (*1R,2R*).

### EXAMPLE 8

### Preparation of (1R, 2R)/(1S, 2R) 1-Boc-amino-2-ethylcyclopropylcarboxylic acid (8a):

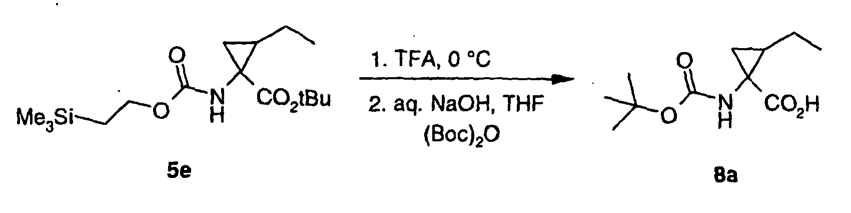

The carbamate **5e** from example 5 (2.6 g, 7.88 mmol) was stirred for 40 min in TFA at 0°C. The mixture was then concentrated and diluted with THF (10 mL). An aqueous NaOH solution (700 mg, 17.5 mmol in 8.8 mL of H₂O) was added followed by a THF (13 mL) solution of (Boc)₂O (2.06 g, 9.44 mmol, 1.2 eq.). The reaction mixture was stirred overnight at RT (the pH was maintained at 8 by adding a 10 % aqueous NaOH solution when needed), then diluted with H₂O, washed with Et₂O (3X) and acidified at 0°C with a 10 % aq. citric acid solution. The aqueous layer was extracted with EtOAc (3X) and successively washed with H₂O (2X) and brine. After the usual treatment (MgSO₄, filtration and concentration) the desired Boc-protected amino acid (**8a**) (788 mg, 3.44 mmol, 44 % yield) was isolated. ¹H NMR (CDCl₃) δ 5.18 (bs, 1H), 1.64-1.58 (m, 2H), 1.55-1.42 (m, 2H), 1.45 (s, 9H), 1.32-1.25 (m, 1H), 0.99 (t, 3H, J= 7.3 Hz).

### Preparation of (1R, 2R)/(1S, 2R)-1-Boc-amino-2-ethylcyclopropylcarboxylic acid methyl ester (8b):

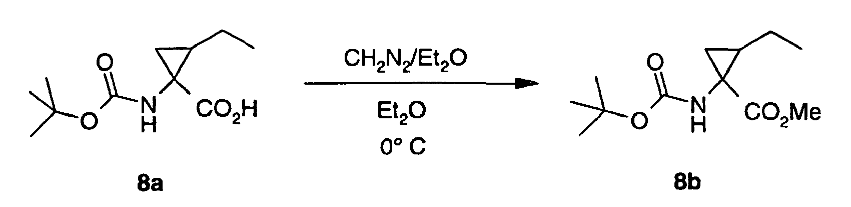

The Boc derivative **8a** (0.30 g, 1.31 mmol) was dissolved in Et₂O (10 mL) and treated with freshly prepared diazomethane in Et₂O at 0°C until the yellow color of a slight excess of diazomethane remained. After stirring for 20 min at RT the reaction mixture was concentrated to dryness to give **8b** as a clear colorless oil (0.32 g, 100%). ¹H NMR (CDCl₃) δ 5.1 (bs, 1H), 3.71 (s, 3H), 1.62-1.57 (m, 2H), 1.55 (s, 9H), 1.53-1.43 (m, 1H), 1.28-1.21 (m, 2H), 0.95 (t, J = 7.3 Hz, 3H).

### EXAMPLE 9

### Enzymatic resolution of methyl (1R, 2R)/(1S, 2R) Boc-1-amino-2-ethylcyclopropyl carboxylate:

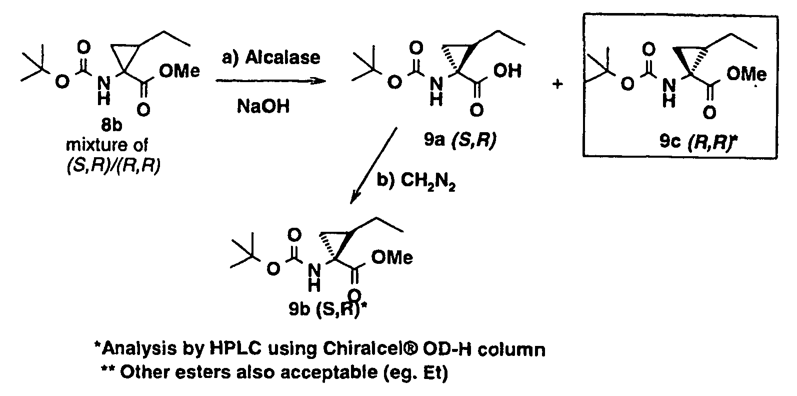

a) The enantiomeric mixture of *(1S, 2R)*/*(1R, 2R)* 1-Boc-amino-2-ethylcarboxylic acid methyl ester of Example 8 (0.31 g, 1.27 mmol) was dissolved in acetone (3 mL) and then diluted with water (7 mL) while being rapidly stirred. The pH of the solution was adjusted to 7.5 with 0.05M aqueous NaOH before Alcalase® [2.4L extract from Novo Nordisk Industrials] (300 mg) was added. During incubation pH was stabilized with NaOH and a pH stat was set up to monitor the addition of the NaOH solution. After 40 h the mixture was diluted with EtOAc and H₂O (with 5 mL sat. NaHCO₃) and the phases separated. The aqueous phase was acidified with 10% aqueous HCl and extracted with EtOAc, dried (MgSO₄), filtered and concentrated to give acid 9a (48.5 mg). The absolute stereochemistry was determined using the correlation described in Examples 6 and 7.
b) Treatment of an aliquot of acid **9a** with diazomethane in Et₂O to give the methyl ester followed by analysis by HPLC using a chiral column [Chiralcel® OD-H, 2.5% Isopropanol/hexane, isocratic] showed a 51:1 ratio of the (*1S,2R*) isomer.
a')The organic phase was dried (MgSO₄), filtered and concentrated to give the unhydrolyzed esters (0.248 g). This material was re-subjected to the above enzyme protocol until the pH remained stable (98 h). After extraction as before, 0.146 mg (100%) of unhydrolyzed ester was recovered. Analysis by HPLC using a chiral column showed a ratio of >50:1 in favor of the *(1R,2R)* isomer.
b') The aqueous phase was acidified with 10% aqueous HCl and extracted with EtOAc, dried (MgSO₄), filtered and concentrated to give the acid analog (82 mg). A portion of this material was treated with diazomethane and then analyzed by HPLC using a chiral column as before which showed a ratio of 65:1 of the *(1S,2R)* derivative.

### EXAMPLE 10

### Synthesis of (1R, 2S)/(1S, 2S) 1-amino-2-ethylcyclopropyl carboxylic acid:

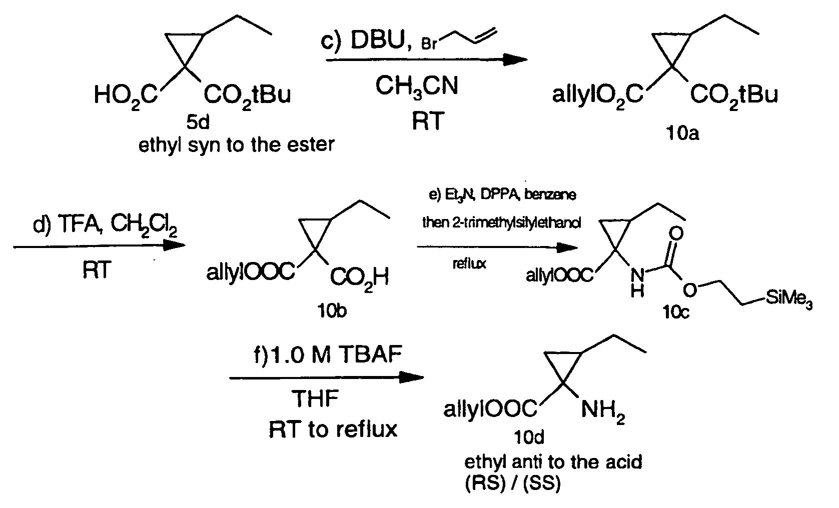

Starting from acid **5d** described in Example 5:
c) To **5d** (1.023 g, 4.77 mmol) in CH₃CN (25 mL) were successively added DBU (860 µL, 5.75 mmol, 1.2 eq.) and allyl bromide (620 µL, 7.16 mmol, 1.5 eq.). The reaction mixture was stirred for 4 h at RT and then concentrated. The residue was diluted with Et₂O and successively washed with a 10 % aq. citric acid solution (2x), H₂O, saturated aqueous NaHCO₃, H₂O (2x) and brine. After the usual treatment (MgSO₄, filtration and concentration) the desired ester **10a** was isolated (1.106 g, 3.35 mmol, 91 % yield) as a colorless oil. MS (FAB) 255 (MH⁺); ¹H NMR (CDCl₃) δ 5.96-5.86 (m, 1H), 5.37-5.22 (m, 2H), 4.70-4.65 (m, 1H), 4.57-4.52 (m, 1H), 1.87-1.79 (m, 1H), 1.47 (s, 9H), 1.45-1.40 (m, 1H), 1.33-1.24 (m, 3H), 1.03 (t, J=7.3 Hz, 3H).
d) To ester **10a** (1.106 g, 4.349 mmol) in dry CH₂Cl₂ (5 mL) at RT was added TFA (5 mL). The reaction mixture was stirred for 1.5 h and then concentrated to afford **10b** (854 mg, 4.308 mmol, 99 % yield). MS (FAB) 199 (MH⁺); ¹H NMR (GDCl₃) δ 5.99-5.79 (m, 1H), 5.40-5.30 (m, 2H), 4.71-4.62 (m, 2H), 2.22-2.00 (m, 2H), 1.95-1.88 (m, 1H), 1.84-1.57 (m, 2H), 0.98 (t, J= 7.3 Hz, 3H).
e) To acid **10b** (853 mg, 4.30 mmol) in dry benzene (14.8 mL) were successively added Et₃N (684 µL, 4.91 mmol, 1.14 eq.) and DPPA (992 µL, 4.60 mmol, 1.07 eq.). The reaction mixture was refluxed for 4.5 h then 2-trimethylsilylethanol (1.23 mL, 8.58 mmol, 2.0 eq.) was added. The reflux was maintained overnight then the reaction mixture was diluted with Et₂O and successively washed with a 10 % aqueous citric acid solution, water, saturated aq. NaHCO₃, water (2x) and brine. After the usual treatment (MgSO₄, filtration, concentration) the residue was flash chromatographed (5 cm, 10 to 15 % AcOEt- hexane) to afford carbamate **10c** (1.212g, 3.866 mmol, 90 % yield) as a pale yellow oil. MS (FAB) 314 (MH⁺); ¹H NMR (CDCl₃) δ 5.93-5.84 (m, 1H), 5.32-5.20 (m, 2H), 5.05 (bs, 1H), 4.60-4.56 (m, 2H), 4.20-4.11 (m, 2H), 1.71-1.60 (m, 3H), 1.39-1.22 (m, 1H), 1.03 (t, J= 7.6 Hz, 3H), 0.96-0.86 (m, 1H), 0.04 (s, 9H).
f) To carbamate **10c** (267 mg, 0.810 mmol) was added a 1.0 M TBAF solution in THF (1.62 mL, 1.62 mmol, 2.0 eq.). The reaction mixture was stirred overnight at RT, refluxed for 30 min and then diluted with AcOEt. The solution was successively washed with water (2x) and brine. After the usual treatment (MgSO₄, filtration and concentration) the desired amine **10d** was isolated (122 mg, 0.721 mmol, 89 % yield) as a pale yellow liquid. ¹H NMR (CDCl₃) δ 5.94-5.86 (m,1H), 5.31-5.22 (m, 2H), 4.58 (d, J= 5.7 Hz, 2H), 1.75 (bs, 2H), 1.61-1.53 (m, 2H), 1.51-1.42 (m, 2H), 1.00 (t, J= 7.3 Hz, 3H), 0.70-0.62 (m, 1H).

### EXAMPLE 11

### Synthesis of ethyl-(1R,2S)/(1S,2S)-1-amino-2-vinylcyclopropyl carboxylate:

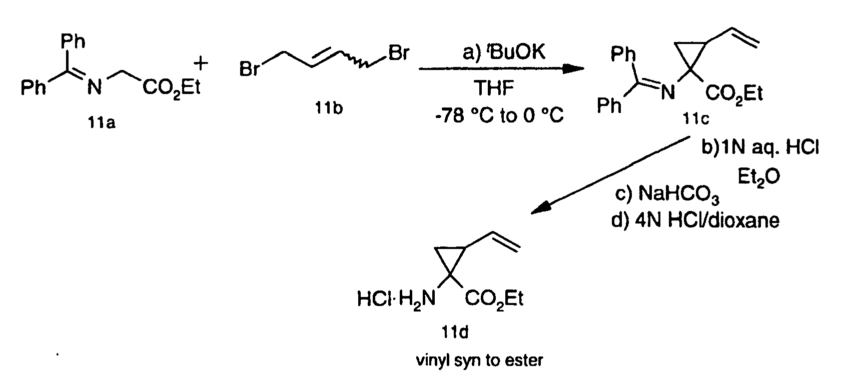

a) To a THF solution (180 mL) of potassium *tert*-butoxide (4.62 g, 41.17 mmol, 1.1 eq.) at -78°C was added commercially available imine 11 a (10.0 g, 37.41 mmol) in THF (45 mL). The reaction mixture was warmed to 0°C and stirred at this temperature for 40 min. The mixture was then cooled back to -78°C for the addition of 1,4-dibromobutene **11b** (8.0 g, 37.40 mmol) and then stirred at 0°C for 1 h and cooled back to -78 °C for the addition of potassium *tert*-butoxide (4.62 g, 41.17 mmol, 1.1 eq.). The reaction mixture was finally stirred one more hour at 0°C and concentrated to yield compound **11c**.
b, c, d) **11c** was taken up in Et₂O (265 mL) and treated with a 1N aq. HCl solution (106 mL). After 3.5 h at RT, the layers were separated and the aqueous layer was washed with Et₂O (2x) and basified with a saturated aq. NaHCO₃ solution. The desired amine was extracted with Et₂O (3x) and the combined organic extract was washed with brine. After the usual treatment (MgSO₄, filtration and concentration) the residue was treated with a 4N HCl solution in dioxane (187 mL, 748 mmol). After concentration, hydrochloride salt **11d** was isolated as a brown solid (2.467 g, 12.87 mmol, 34 % yield). ¹H NMR (CDCl₃) δ 9.17 (bs, 3H), 5.75-5.66 (m, 1H), 5.39 (d, J= 17.2 Hz, 1H), 5.21 (d, J= 10.2 Hz, 1H), 4.35-4.21 (m, 2H), 2.77-2.70 (m, 1H), 2.05 (dd, J= 6.4, 10.2 Hz, 1H), 1.75 (dd, J= 6.4, 8.3 Hz, 1H), 1.33 (t, J= 7.0 Hz, 3H).

### EXAMPLE 12

### Preparation of (1R,2S/1S,2S)-1-Boc-amino-2-vinylcyclopropyl carboxylic acid ethyl ester:

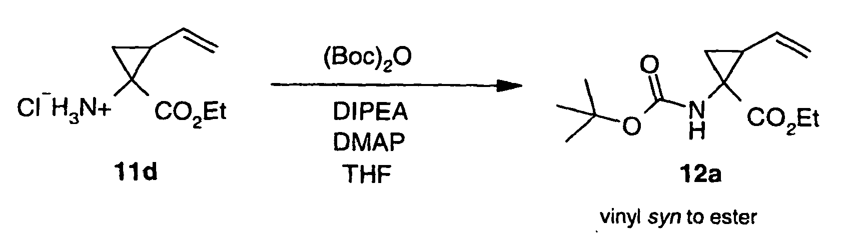

The hydrochloride salt **11d** (1.0 g, 5.2 mmol) and (Boc)₂O (1.2 g, 5.7 mmol) were dissolved in THF (30 mL) and treated with DMAP (0.13 g, 1.04 mmol, 0.2 equiv.) and diisopropylethylamine (2.8 mL, 15.6 mmol). The reaction mixture was stirred 24 h before being diluted with EtOAc (40 mL) and washed successively with sat. NaHCO₃ (aq), 5% aqueous HCl, and sat. brine. The organic phase was dried (MgSO₄), filtered and concentrated to give after purification by flash chromatography (15% EtOAc/hexane), **12a** (0.29 g, 23%). ¹H NMR (CDCl₃) δ 5.80-5.72 (m, 1H), 5.29-5.25 (dd, J =17.2, 17.2 Hz, 1H), 5.24-5.1 (bs, 1H), 5.10 (dd, J = 9.2, 9.2 Hz, 1H), 4.22-4.13 (m, 2H), 2.15-2.04 (m, 1H), 1.85-1.73 (bs, 1H), 1.55-1.5 (m, 1H), 1.49 (s, 9H), 1.26 (t, J = 7.3 Hz, 3H).

### EXAMPLE 13

### Enzymatic resolution of ethyl (1R,2S)/(1S,2S) 1-amino-2-vinylcyclopropyl carboxylate:

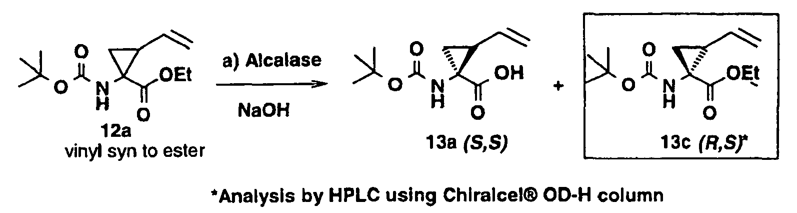

a) Racemic derivative **12a** (0.29 g, 1.14 mmol) was dissolved in acetone (5 mL) and diluted with H₂O (10 mL). The pH was adjusted with 0.2N aqueous NaOH to 7.2 before Alcalase® was added (300 mg). To keep the pH constant during incubation, a NaOH solution was added by a pH stat titrator over 9 days until the theoretical amount of base had been added. Following acid/base extraction as described in Example 9, the unhydrolyzed ester (0.15 g, 100%) and the hydrolyzed material (0.139 g, 95%) were isolated. Analysis of the unhydrolyzed ester by HPLC using a chiral column showed a ratio of 43:1 of the desired compound **13c**. Compound **206** (wherein **R**_{**1**} is vinyl, Table 2) was hydrogenated (10.8 mg, 0.015 mmol in 1 mL of EtOH with about 1mL of 20% Pd(OH)₂ under 1 atm of H₂ for 45 min) to yield compound **214** (wherein **R**_{**1**} is ethyl, Table 2). Compound **214** had been assigned the *(1R,2R)* stereochemistry based on chemical correlation as described in Examples 6 and 7 indicating that compound **206** (**R**₁ = vinyl) has the same absolute configuration as represented by **13c** (albeit 1*R,2S* because **R**_{**1**}=vinyl).

Conditions for HPLC analysis: Chiralcel® OD-H (4.6 mm x 25 cm), isocratic conditions using a mobile phase of 2.5% isopropanol/hexane.

### EXAMPLE 14

### Resolution of (1R,2S)/(1S,2S) 1-amino-2-vinylcyclopropyl carboxylate by crystallization with dibenzoyl-D-tartaric acid

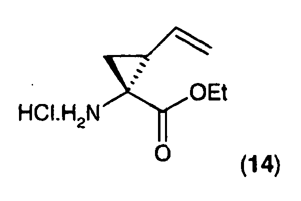

To a solution of crude racemic (1*S*,2*S* and 1*R*, 2*S*) ethyl 1-amino-2-vinylcyclopropyl carboxylate [obtained from N-(diphenylmethylene)glycine ethyl ester (25.0 g, 93.5 mol) as described in Example 13] in EtOAc (800 mL) was added dibenzoyl-D-tartaric acid (33.5 g, 93.5 mol). The mixture was heated to reflux, left at RT for 15 min then cooled to 0°C. A white solid was obtained after 30 min. The solid was filtered, washed with EtOAc (100 mL) and air-dried. The solid was suspended in acetone (70 mL), sonicated and filtered (3x). The solid was next recrystallized twice in hot acetone (crop A). The mother liquors were concentrated and the residue was recrystallized three times in hot acetone (crop B). The two crops of the amorphous white solids of dibenzoyl-D-tartaric acid salt were combined (5.53 g) and suspended in a mixture of Et₂O (250 mL) and saturated NaHCO₃ solution (150 mL). The organic layer was washed with brine, dried (MgSO₄) and filtered. The filtrate was diluted with 1 N HCl/Et₂O (100 mL) and concentrated under reduced pressure. The oily residue was evaporated with CCl₄ to afford ethyl 1*(R)*-amino-2*(S)*-vinyl cyclopropanecarboxylate hydrochloride (940 mg, 11 % yield) as a white hygroscopic solid for which absolute stereochemistry was assigned by correlation with compound **13c** of Example 13.

${[ α ]}_{D}^{25}$ +39.5°C (c 1.14 MeOH); ${[ α ]}_{365}^{25}$ +88.5°C (c 1.14 MeOH); ¹H NMR (DMSO-d₆) δ 9.07 (broad s, 2H), 5.64 (ddd, J=17.2, 10.4, 8.7 Hz, 1H), 5.36 (dd, J=17.2, 1.6 Hz, 1H), 5.19 (dd, J=10.4, 1.6 Hz, 1H), 4.24-4.16 (m, 2H), 2.51-2.45 (m, peaks hindered by DMSO, 1H), 1.84 (dd, J=10.0, 6.0 Hz, 1H), 1.64 (dd, J=8.3, 6.0 Hz, 1H), 1.23 (t, J=7.1 Hz, 3H); MS (ESI) m/z 156 (MH)⁺; the enantiomeric purity was determined to be 91% ee by HPLC analysis (CHIRALPAK AS® column, Hex:*i*-PrOH) of the Boc derivative. (Example 13)

### P4-P2 BUILDING BLOCKS

### Example 15

### Synthesis of segment: Ac-Chg-Chg-Pro (4(R)-naphthalen-1-ylmethoxy)-OH (15g)

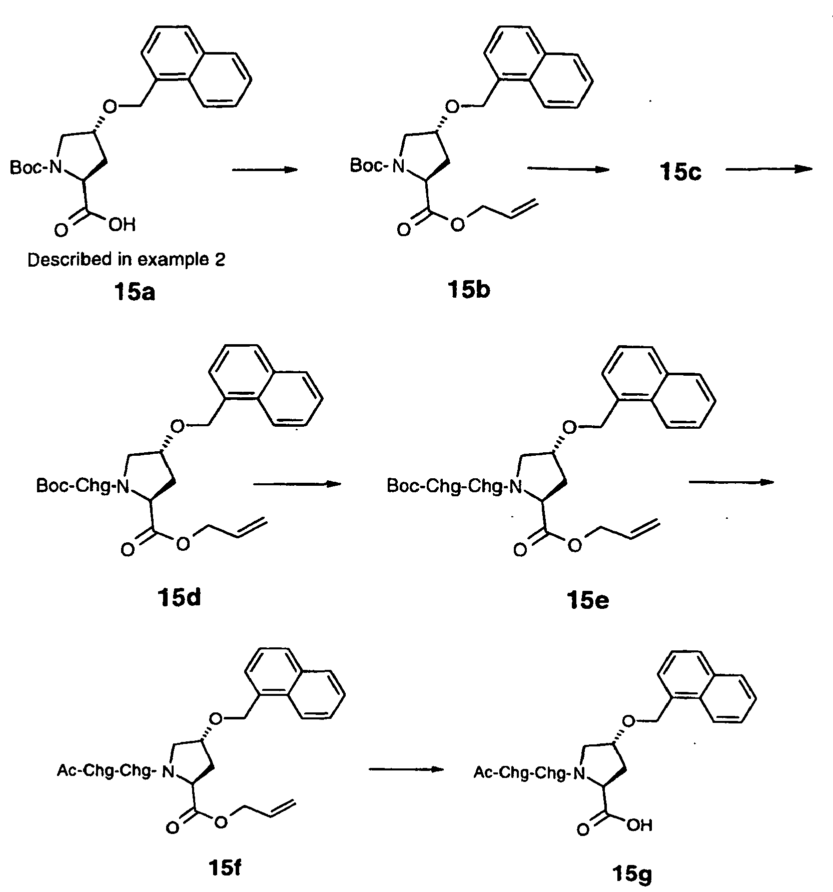

Compound **15a** (same as compound 2 from Example 2)(4.45 g, 11.98 mmol) was dissolved in anhydrous CH₃CN (60 mL), DBU (2.2 mL, 14.38 mmol) and allyl bromide (1.1 mL, 13.18 mmol) were added successively and the reaction mixture was stirred 24 h at RT. The mixture was concentrated, the resulting oil was diluted with EtOAc and water and successively washed with water (2x) and brine (1x). The EtOAc layer was dried (MgSO₄), filtered and evaporated to dryness. The yellow oil was purified by flash chromatography (eluent:hexane:EtOAc;90:10 to 85:15) to provide the product **15b** as a yellow oil (2, 4.17 g; 85% yield). MS (FAB) 412 MH⁺

¹H NMR (CDCl₃), mixture of rotamers ca.1:2 , δ (d, J= 8Hz, 1H), 7.87 (d, J= 8Hz, 1H), 7.82 (d, J= 8Hz, 1H), 7.55-7.41 (m, 4H), 5.95-5.85 (m, 1H), 5.34-5.21 (m, 2H), 5.03-4.88 (m, 2H), 4.70-4.56 (m, 2H), 4.48 & 4.39 (t, J= 8, 15Hz, 1H), 4.28-4.23 (m, 1H), 3.81-3.55 (m, 2H), 2.46-2.36 (m, 1H), 2.13-2.05 (m, 1H), 1.44 &1.41 (s, 9H).

Compound **15b** (2.08 g , 5.05 mmol) was treated for 30 min at RT with 4N HCl / dioxane. Evaporation to dryness provided the corresponding amine-HCl as an oil. The amine-HCl **15c** was dissolved in anhydrous DCM (25 mL) and NMM (2.2 mL, 20.22 mmol), Boc-Chg-OH • H₂O (1.53 g, 5.56 mmol) and TBTU (1.95 g, 6.07 mmol) were added successively. The reaction mixture was stirred at RT overnight, then, diluted with EtOAc and successively washed with 10% aqueous citric acid (2x), saturated aqueous NaHCO₃ (2x), water (2x), and brine (1x). The EtOAc layer was dried (MgSO₄), filtered and evaporated to dryness to provide the crude product **15d** as a yellowish-white foam (ca 2.78 g, 100% yield). MS (FAB) 551.4 MH⁺. ¹H NMR (CDCl₃) δ 8.03(d, J= 8Hz, 1H), 7.86 (b d, J= 8.5Hz, 1H), 7.84 (d, J= 8Hz, 1H), 7.56-7.40 (m, 4H), 5.92-5.85 (m, 1H), 5.31 (dd, J= 1, 17Hz, 1H), 5.22 (dd, J= 1, 10Hz, 1H), 5.17 (d, J= 9Hz, 1H), 5.05 (d, J= 12Hz, 1H), 4.91 (d, J= 12Hz, 1H), 4.67-4.60 (m, 3H), 4.31-4.27 (m, 2H), 4.16 (b d, J= 11Hz, 1H), 3.71 (dd, J= 4, 11Hz, 1H), 2.47-2.41 (m, 1H), 2.08-1.99 (m, 1H), 1.85-1.63 (m, 5H), 1.44-1.40 (m, 1H), 1.36 (s, 9H), 1.28-1.00 (m, 5H).

The crude dipeptide **15d** (ca.5.05 mmol) was treated with 4N HCl/dioxane (25 mL) as described for the synthesis of compound 15c. The crude hydrochloride salt was coupled to Boc-Chg-OH • H₂O (1.53 g, 5.55 mmol) with NMM (2.22 mL, 20.22 mmol) and TBTU (1.95 g, 6.07 mmol) in DCM (25 mL) as described for the synthesis of compound 15d to yield crude tripeptide **15e** as a yellow-oil foam. The crude material was purified by flash chromatography (eluent:hexane:EtOAc;80:20 to 75:25) to provide the tripeptide 15e as a white foam (2.75 g; 79% yield over 2 steps). MS (FAB) 690.5 MH⁺. ¹H NMR (CDCl₃), mainly one rotamer, δ 8.06 (d, J= 8Hz, 1H), 7.87 (b d, J= 8.5Hz, 1H), 7.82 (d, J= 8Hz, 1H), 7.57-7.40 (m, 4H), 6.41 (d, J= 8.5Hz, 1H), 5.92-5.84 (m, 1H), 5.31 (dd, J= 1, 17Hz, 1H), 5.23 (dd, J= 1, 10.5Hz, 1H), 5.04 (d, J= 12Hz, 1H), 4.98 (b d; J= 7Hz, 1H), 4.93 (d, J=12Hz, 1H), 4.63-4.58 (m, 4H), 4.29-4.25 (m, 1H), 4.10-4.07 (m, 1H), 3.90-3.84 (m, 1H), 3.72 (dd, J= 4, 11Hz, 1H), 2.48-2.40 (m, 1H), 2.07-1.99 (m, 1H), 1.83-1.55 (m, 12H), 1.43 (s, 9H), 1.23-0.89 (m, 10H).

The tripeptide **15e** (2.75 g , 3.99 mmol) was treated with 4N HCl/dioxane (20 mL) as described for the synthesis of compound **15c**. The crude hydrochloride salt was dissolved in anhydrous DCM (20 mL). NMM (1.75 mL, 15.94 mmol) and acetic anhydride (752 µl, 7.97mmol) were added successively. The reaction mixture was stirred overnight at RT, then diluted with EtOAc. The organic layer was washed successively with 10% aqueous citric acid (2x), saturated aqueous NaHCO₃ (2x), water (2x) and brine (1x), dried (MgSO₄), filtered, and evaporated to dryness to provide the crude tripeptide 15f as a white foam (2.48g, 98% yield).

MS (FAB) 632.4 MH+I. ¹H NMR (CDCl₃), mainly one rotamer, δ 8.06(b d, J= 8Hz, 1H), 7.87 (b d, J= 8Hz, 1H), 7.83 (d, J= 8Hz, 1H), 7.58-7.40 (m, 4H), 6.36 (d, J= 9Hz, 1H), 6.01 (d, J= 9Hz, 1H), 5.94-5.83 (m, 1H), 5.34-5.28 (m, 1H), 5.25-5.21 (m, 1H), 5.05 (d, J= 12Hz, 1H), 4.94 (d, J= 12Hz, 1H), 4.64-4.57 (m, 4H), 4.30-4.23 (m, 2H), 4.12-4.08 (m, 1H), 3.73 (dd, J= 4, 11Hz, 1H), 2.49-2.42 (m, 1H), 2.08-2.01 (m, 1H), 1.99 (s, 3H), 1.85-1.53 (m, 11H), 1.25-0.88 (m, 11H).

The crude tripeptide **15f** (2.48 g, 3.93 mmol) was dissolved in an anhydrous mixture of CH₃CN : DCM (20 mL). Triphenylphosphine (53.5 mg, 0.200 mmol) and tetrakis(triphenylphosphine)-palladium (0) catalyst (117.9 mg, 0.102 mmol) were added successively, followed by pyrrolidine (353.9 µL, 4.24 mmol). The reaction mixture was stirred at RT for 18 h. Thereafter, the solvent was evaporated. The residue was dissolved in EtOAc and 10% aqueous citric acid, and further washed twice more with 10% aqueous citric acid, water (2x), and brine (1x). The organic layer was dried (MgSO₄), filtered and evaporated. The crude product was triturated in Et₂O: DCM (85:15) to provide after filtration the tripeptide **15g** as a white solid (2.09 g, 90% yield). MS (FAB) 592.4 MH⁺ 614.3 (M+Na)⁺. ¹H NMR (CDCl₃), mainly one rotamer, δ 8.08 (d, J= 8Hz, 1H), 7.93 (b d, J= 9Hz, 1H), 7.88 (b d, J= 8Hz, 1H), 7.82 (d, J= 8Hz, 1H), 7.57-7.41 (m, 4H), 6.47 (d, J= 8.5Hz, 1H), 5.05 (d, J= 12.5Hz, 1H), 4.94 (d, J= 12.5Hz, 1H), 4.73 (t, J= 9.5, 19Hz, 1H), 4.44-4.35 (m, 2H), 4.26 (b s, 1H), 4.19 (d, J= 11.5Hz, 1H), 3.75 (dd, J= 4, 11Hz, 1H), 2.47 (b dd, J= 7.5, 13.5Hz, 1H), 2.20-2.11 (m, 1H), 2.04 (s, 3H), 1.88-1.41 (m, 11H), 1.30-0.80 (11H).

### EXAMPLE16

### Synthesis of segment Ac-Chg-Val-Pro(4(R)-naphthalen-1-ylmethoxy)-OH (16e)

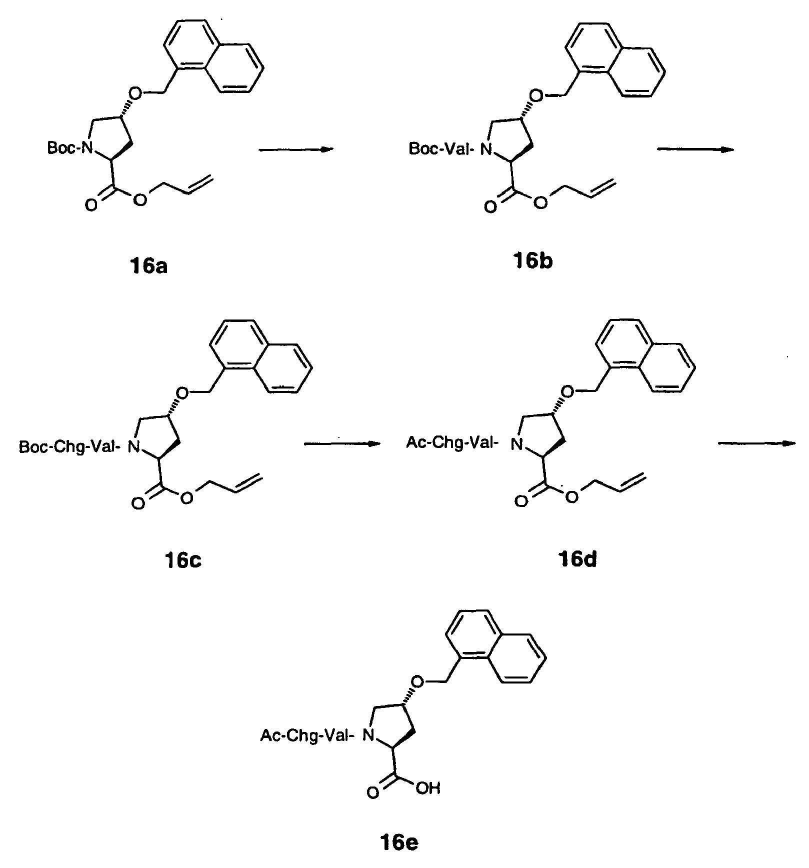

Compound **16a** (2.89 g, 7.02 mmol) was treated with 4N HCl/dioxane (30 mL) as described for the synthesis of compound **15c**. The crude hydrochloride salt was coupled to Boc-Val-OH (1.53 g, 7.73 mmol) with NMM (3.1 mL, 28.09 mmol) and TBTU (2.71 g, 8.43 mmol) in DCM (35 mL) for 3 1/2 h as described for the synthesis of compound **15d** to provide the crude dipeptide **16b** as an ivory oil-foam (ca.3.60 g, 100% yield). MS (FAB) 509.3 MH⁻ 511.3 MH⁺ 533.2 (M+Na)⁺. ¹H NMR (CDCl₃) δ 8.04 (b d, J= 8Hz, 1H), 7.87 (b d, J= 7Hz, 1H), 7.82 (d, J= 8Hz, 1H), 7.56-7.40 (m, 4H), 5.93-5.85 (m, 1H), 5.34-5.28 (m, 1H), 5.24-5.19 (m, 2H), 5.04 (d, J= 12Hz, 1H), 4.92 (d, J= 12Hz, 1H), 4.67-4.60 (m, 3H), 4.31-4.26 (m, 2H), 4.11-4.09 (m, 1H), 3.72 (dd, J= 4, 11Hz, 1H), 2.48-2.41 (m, 1H), 2.07-1.99 (m, 1H), 1.44-1.36 (m, 1H), 1.37 (s, 9H), 1.01 (d, J= 7Hz, 3H), 0.93 (d, J= 7Hz, 3H).

The crude dipeptide **16b** (ca.7.02 mmol) was treated with 4N HCl/dioxane (30mL) as described for the synthesis of compound **15c.** The crude hydrochloride salt was coupled to Boc-Chg-OH H₂O (2.13 g, 7.73 mmol) with NMM (3.1 mL, 28.09 mmol) and TBTU (2.71 g, 8.43 mmol) in CH₂Cl₂ (35 mL) as described for the synthesis of compound **15d** to provide the crude tripeptide **16c** as an ivory foam (ca.4.6 g, 100% yield). MS (FAB) 648.5 MH⁻ 672.4 (M+Na)⁺. ¹H NMR (CDCl₃) δ 8.06 (b d, J=8Hz, 1H), 7.87 (b d, J= 7.5 Hz, 1H), 7.82 (b d , J= 8Hz, 1H), 7.57-7.40 (m, 4H), 6.46 (b d, J= 8.5Hz, 1H), 5.94-5.84 (m, 1H), 5.31 (dd, J= 1, 17Hz, 1H), 5.23 (dd, J= 1, 10.5Hz, 1H), 5.03 (d, J= 12Hz, 1H), 5.00-4.97 (m, 1H), 4.93 (d, J=, 12Hz, 1H), 4.63-4.59 (m, 4H), 4.29-4.27 (m, 1H), 4.10-4.07 (m, 1H), 3.92-3.86 (m, 1H), 3.72 (dd, J= 5, 11Hz, 1H), 2.48-2.41 (m, 1H), 2.10-1.99 (m, 1H), 1.76-1.57 (m, 6H), 1.43 (s, 9H), 1.20-0.92 (m, 6H), 1.00 (d, J= 7Hz, 3H), 0.93 (d, J= 7Hz, 3H).

The crude tripeptide **16c** (ca.7.02 mmol) was treated with 4N HCl/dioxane (30 mL) as described for the synthesis of compound **15c**. The crude hydrochloride salt was further treated with acetic anhydride (1.33 mL, 14.05 mmol) and NMM (3.1 mL, 28.09 mmol) in CH₂Cl₂ (35 mL) as described for the synthesis of compound **15d.** The crude product was flash purified (eluent:hexane:EtOAc;30:70) to provide the acetylated protected tripeptide **16d** as a white foam (3.39 g, 81% yield over 3 steps). MS (FAB) 590.3 MH⁻ 592.4 MH⁺ 614.4 (M+Na)⁺

¹H NMR (CDCl₃), mainly one rotamer, δ 8.06 (d, J= 8Hz, 1H), 7.88 (b d, J= 8Hz, 1H), 7.83 (d, J= 8Hz, 1H), 7.58-7.41 (m, 4H), 6.37 (d, J= 9Hz, 1H), 5.97 (d, J= 8.5 Hz, 1H), 5.94-5.84 (m, 1H), 5.31 (dd, J= 1, 17Hz, 1H), 5.24 (dd, J= 1, 10.5 Hz, 1H), 5.05 (d, J= 12Hz, 1H), 4.94 (d, J= 12Hz, 1H), 4.66-4.57 (m, 4H), 4.31-4.22 (m, 2H), 4.11-4.05 (m, 1H), 3.73 (dd, J= 4.5, 11Hz, 1H), 2.50-2.43 (m, 1H), 2.09-2.01 (m, 2H), 2.00 (s, 3H), 1.68-1.55 (m, 5H), 1.15-0.89 (m, 6H), 0.99 (d, J= 7Hz, 3H), 0.91 (d, J= 7Hz, 3H).

The acetylated tripeptide **16d** (3.39 g, 5.73 mmol) was deprotected by tetrakis(triphenylphosphine)- palladium (0) catalyst (172.1 mg, 0.149 mmol) with triphenylphosphine (78.1 mg, 0.298 mmol) and pyrrolidine (516 µL, 6.19 mmol) in a 1:1 mixture of anhydrous CH₃CN : DCM (30 mL) as described for the synthesis of compound **15g.** The crude light yellow foam product was triturated in Et₂O : DCM (85:15) to provide after filtration the tripeptide **16e** as an off-white solid (3.0g ; 95% yield). MS (FAB) 550.3 MH⁻

¹H NMR (CDCl₃) δ 8.08 (d, J= 8Hz, 1H), 8.04 (b d, J= 9Hz, 1H), 7.88 (b d, J= 7.5Hz, 1H), 7.82 (d, J= 8Hz, 1H), 7.58-7.37 (m, 5H), 5.05 (d, J= 12Hz, 1H), 4.94 (d, J= 12Hz, 1H), 4.61 (t, J= 9.5, 19.5Hz, 1H), 4.46-4.37 (m, 2H), 4.27 (b s, 1H), 4.17 (d, J= 11Hz, 1H), 3.74 (dd, J= 4, 11Hz, 1H), 2.49 (b dd, J= 7.5, 13Hz, 1H), 2.17-2.09 (m, 1H), 2.04 (s, 3H), 2.03-1.94 (m, 1H), 1.79 (b d, J= 12.5Hz, 1H), 1.62-1.43 (m, 5H), 1.08-0.85 (m, 5H), 1.00 (d, J= 7Hz, 3H), 0.90 (d, J= 7Hz, 3H).

### COMPOUNDS OF TABLES 1 TO 4

### EXAMPLE 17

### Synthesis of compound 104 of Table 1

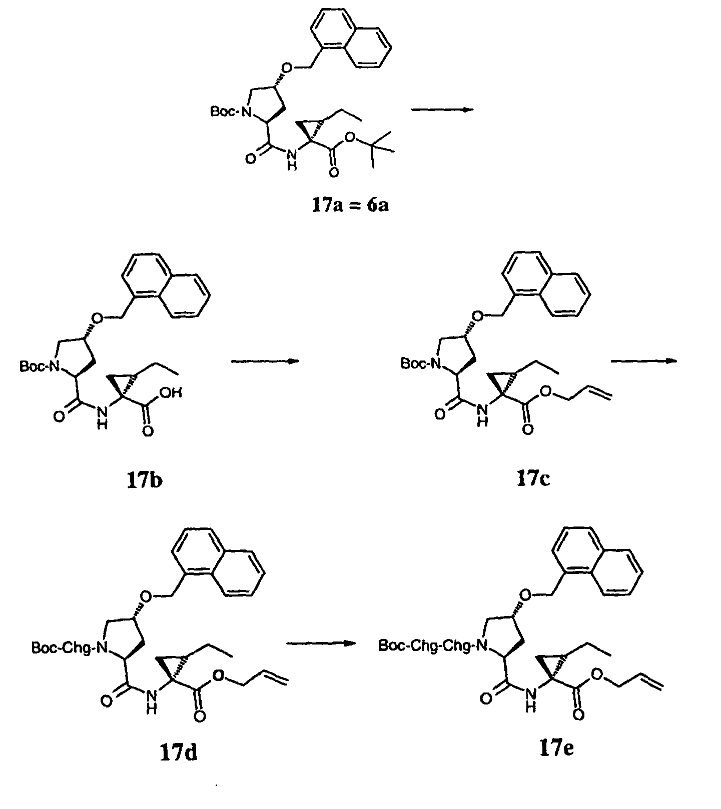

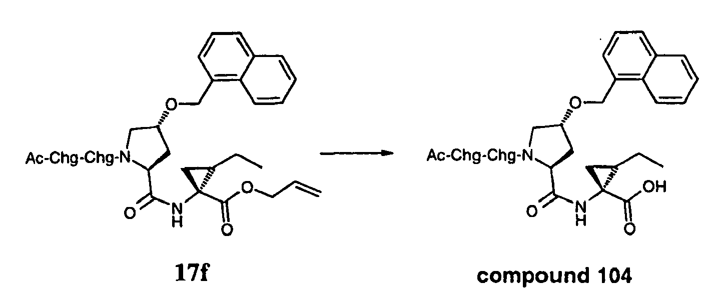

Compound **17a** (4.27 g, 7.93 mmol, described as compound **6a** in Example 6) was treated with 4N HCl/dioxane (40 mL) for 5 h as described for compound **15c**. The crude hydrochloride salt was dissolved in THF (10 mL) and a solution of NaOH (348.7 mg, 8.72 mmol) in H₂O (5 mL) was added, followed by a dropwise addition of (Boc)₂O (1.73 g, 7.93 mmol) dissolved in THF (13 mL). The pH was maintained at 8 by the addition of 10% aqueous NaOH as required. The reaction mixture was stirred vigorously, then diluted with Et₂O and H₂O and extracted one time more with Et₂O. The water layer was acidified to pH 3 with 10% aqueous citric acid. The mixture was extracted with EtOAc (3x). The combined EtOAc extracts were washed with H₂O (2x), brine(1x), dried (MgSO₄), filtered and evaporated to dryness to provide crude compound **17b** as an ivory foam (ca.7.93mmol). MS (FAB) 481.3 MH^{- 1}H NMR (CDCl₃), ca.1:1 mixture of rotamers, δ 8.04 (bd, J= 7.5Hz, 1H), 7.87 (b d, J= 7.5Hz, 1H), 7.82 (d, J= 7.5Hz, 1H), 7.56-7.40 (m, 5H), 4.96 (b s, 2H), 4.33 (t, J= 7.5, 14.5Hz, 1H), 4.21-4.09 (m, 0.5H), 3.99-3.84 (m, 0.5H), 3.78-3.75 (m, 0.5H), 3.68-3.62 (m, 0.5H), 3.61-3.42 (m, 1H), 2.55-2.41 (m, 1H), 2.22-2.1 (m, 1H), 1.61-1.52 (m, 3H), 1.43 (s, 9H), 1.40-1.31 (m, 1H), 1.25-1.19 (m, 1H), 0.99 (t, J= 7.5, 14.5Hz, 3H).

Compound **17b** (ca.7.93 mmol) was treated with DBU (1.18 mL, 93 mmol) and allylbromide (4.12 mL, 47.61 mmol) in anhydrous CH₃CN (40 mL) for 48 h as described for compound **15b** to provide the allylated dipeptide **17c** as an ivory foam (3.54 g; 86% yield over 2 steps). MS (FAB) 521.3 MH⁻ 545.2 (M+Na)⁺. ¹H NMR (CDCl₃), ca.1:1 mixture of rotamers, δ 8.05 (b d, J= 8Hz, 1H), 7.86 (b d, J= 7.5Hz, 1H), 7.82 (d, J= 8Hz, 1H), 7.55-7.40 (m, 5H), 5.88-5.79 (m, 1H), 5.27 (b d, J= 17.5Hz, 1H), 5.18 (b d, J= 10Hz, 1H), 5.03-4.89 (m, 2H), 4.63-4.50 (m, 2H), 4.44-4.19 (m, 2H), 4.00-3.40 (m, 2H), 2.70-2.02 (m, 2H), 1.66-1.35 (m, 5H), 1.44 (s, 9H), 0.95 (t, J= 7.5, 14.5Hz, 3H).

The crude dipeptide **17c** (1.18 g, 2.26 mmol) was treated with 4N HCl/dioxane (35 mL) as described for compound **15c**. The crude hydrochloride salt was coupled to Boc-Chg-OH • H₂O (684 mg, 2.48 mmol) with NMM (993 µL, 9.03 mmol) and TBTU (870 mg, 2.71 mmol) in DCM (11 mL) as described for compound 15d to provide the crude tripeptide **17d** as an ivory foam (1.41 g; 95%). MS (FAB) 660.4 MH⁻ 662.3 MH⁺. ¹H NMR (CDCl₃), mainly one rotamer, δ 8.03 (b d, J= 8Hz, 1H), 7.85 (b d, J= 8Hz, 1H), 7.81 (d, J= 8Hz, 1H), 7.56-7.39 (m, 5H), 5.88-5.77 (m, 1H), 5.26 (dd, J= 1.5, 17Hz, 1H), 5.15 (dd, J= 1.5, 10.5Hz, 1H), 5.12 (s, 1H), 5.02-4.92 (m, 2H), 4.72-4.59 (m, 1H), 4.57-4.46 (m, 1H), 4.42-4.35 (m, 1H), 4.33-4.20 (m, 1H), 4.02-3.90 (m, 1H), 3.78-3.70 (m, 1H), 3.67-3.51 (m, 1H), 2.71-2.61 (m, 1H), 2.12-2.02 (m, 1H), 1.79-1.48 (m, 10H), 1.45-1.39 (m, 1H), 1.38 (s, 9H), 1.25-1.01 (m, 5H), 0.94 (t, J=7.5, 14Hz, 3H).

The crude tripeptide **17d** (265 mg, 0.400 mmol) was treated with 4N HCl/dioxane (3 mL) as described for compound **15c**. The crude hydrochloride salt was coupled to Boc-Chg-OH • H₂O (143.3 mg, 0.521 mmol) with NMM (176 µL, 1.60 mmol) and TBTU (154.3 mg, 0.481 mmol) in DCM (3 mL) as described for compound **15d** to provide crude tetrapeptide **17e** as an ivory foam (ca.0.400 mmol ; 100%). MS (FAB) 799.5 MH⁻ 801.5 MH⁺ 823 (M+Na)⁺. ¹H NMR (CDCl₃), ca. 1 : 1 mixture of rotamers, δ 8.05 (b d, J= 8.5Hz, 1H), 7.87 (b d, J= 7.5Hz, 1H), 7.81 (d, J= 8.5Hz, 1H), 7.55-7.40 (m, 4H), 7.37 (s, 1H), 6.58-6.41 (m, 1H), 5.89-5.78 (m, 1H), 5.26 (b dd, J= 1.5, 17Hz, 1H), 5.16 (b dd, J= 1.5, 10.5Hz, 1H), 5.20-4.92 (m, 3H), 4.68-4.58 (m, 2H), 4.57-4.47 (m, 1H), 4.43-4.26 (m, 1H), 3.99-3.81 (m, 2H), 3.78-3.60 (m, 2H), 2.67-2.60 (m, 1H), 2.11-2.02 (m, 1H), 1.78-1.42 (m, 14H), 1.44 &1.43 (s, 9H), 1.25-0.91 (m, 13H), 0.95 (t, J= 7.5, 15Hz, 3H).

The crude tetrapeptide **17e** (ca.0.400 mmol) was treated with 4N HCl/dioxane (3 mL) as described for compound **15c**. The crude hydrochloride salt was further treated with acetic anhydride (83 µL, 0.884 mmol) and NMM (194 µL, 1.77 mmol) in DCM (3 mL) as described for compound **15f** to provide the crude acetylated tetrapeptide **17f** as an ivory foam (ca.0.400 mmol).

MS (FAB) 741.5 MH⁻ 743.4 MH⁺ 765.4 (M+Na)⁺.

¹H NMR (CDCl₃) δ 8.05 (b d, J= 8.5Hz, 1H), 7.87 (b d, J= 7.5Hz, 1H), 7.82 (d, J= 8.5Hz, 1H), 7.55-7.41 (m, 4H), 7.39 (s, 1H), 6.63-6.48 (m, 1H), 6.01 (d, J= 8.5Hz, 1H), 5.90-5.79 (m, 1H), 5.27 (b dd, J= 1.5, 17Hz, 1H), 5.16 (b dd, J= 1.5, 10.5Hz, 1H), 5.01 (d, J= 12Hz, 1H), 4.96 (d, J= 12Hz, 1H), 4.69-4.48 (m, 3H), 4.44-4.37 (m, 1H), 4.36-4.22 (m, 1H), 3.96 (dd, J= 4, 11Hz, 1H), 3.78-3.60 (m, 2H), 2.67-2.59 (m, 1H), 2.10-2.00 (m, 1H), 2.01 (s, 3H), 1.78-1.48 (m, 13H), 1.45-1.35 (m, 1H), 1.26-0.89 (m, 13H), 0.95 (t, J= 7.5, 15Hz, 3H).

The acetylated tetrapeptide **17f** (ca.0.400 mmol) was deprotected by tetrakis(triphenylphosphine)- palladium (0) catalyst (11.3 mg, 0.010 mmol) with triphenylphosphine (5.12 mg , 0.020 mmol) and pyrrolidine (34 µL, 0.406 mmol) in a 1:1 mixture of anhydrous CH₃CN : DCM (2 mL) as described for compound **15g.** The crude product was purified by flash chromatography (eluent - 1^{st} EtOAc, then, 2^{nd} 1.92% HOAc, 3.85% MeOH in DCM) to provide, after lyophilization, the tetrapeptide compound 104 of Table 1 as an off-white amorphous solid (193.1 mg; 73% yield over 5 steps).

MS (FAB) 701.4 MH⁻ 703.4 MH⁺ 725.4 (M+Na)⁺.

¹H NMR (DMSO), ca.1 : 5 mixture of rotamers, δ 8.57 & 8.32 (s, 1H), 8.04 (d, J= 7.5Hz, 1H), 7.94 (b d, J= 7.5Hz, 1H), 7.88 (d, J= 8Hz, 1H), 7.83-7.78 (m, 2H), 7.58-7.30 (m, 4H), 4.99 (d, J= 12Hz, 1H), 4.90 (d, J= 12Hz, 1H), 4.44-4.29 (m, 2H), 4.29-4.05 (m, 3H), 3.87-3.73 (m, 1H). 2.23-2.13 (m, 1H). 2.05-1.95 (m, 1H), 1.91 & 1.84 (s, 3H), 1.75-1.40 (m, 15H), 1.29-0.84 (m, 12H), 0.91 (t, J= 7.5, 14.5Hz, 3H).

### EXAMPLE 18

### Synthesis of compound 105 of Table 1

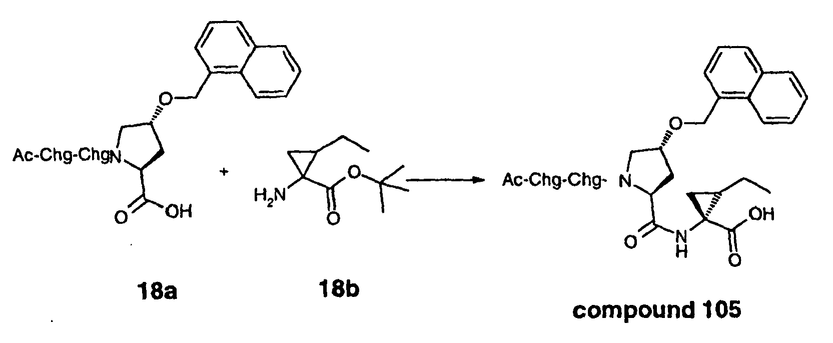

Compound **18b,** i.e. corresponding to compound **5f** of Example 5, was coupled to the preformed tripeptide **18a** described previously in Example 15. More specifically, compound **18b** (ca.0.521 mmol) was combined with compound **18a** (323.6 mg, 0.547 mmol) in DCM (3 mL) and NMM (172 µL, 1.562 mmol), followed by the addition of HATU (237.6 mg, 0.625 mmol). The reaction mixture was stirred at RT for 18 h, after which it was worked up as described for compound **15d** to give the crude tetrapeptide as a racemic mixture at P1. Both isomers were partially separated by flash chromatography (eluent- toluene : EtOAc ; 40:60). Combination of the first eluting fractions gave a 9:1 mixture in which analogous *tert*-butyl ester of **17f** was the major component (58 mg). The middle fractions contain different ratios of the corresponding *tert*-butyl esters of **17f** and compound **105** *t*-butyl ester (163 mg). The latter eluting fractions provided the corresponding *tert*-butyl ester of compound 105 as the major isomer (75.8 mg).

The latter ester (74 mg, 0.0975 mmol) was dissolved in 4N HCl/dioxane (2 mL), stirred at RT for 5.5 h then evaporated to dryness to give an oil. Purification by flash chromatography (eluent - 1^{st} EtOAc, then 2^{nd} 1.92% HOAc, 3.85% MeOH, in DCM) yielded, after lyophilization, compound 105 as a white-amorphous solid (38.7 mg, 56% yield). HPLC analysis indicated a 3 : 1 ratio of compound 105 and compound **104.** MS and NMR data for compound **105:** MS (FAB) 701.5 MH⁻ 703.5 MH⁺ 725.6 (M+Na)⁺. ¹H NMR (DMSO), ca.1 : 2.5 mixture of rotamers, δ 8.76 & 8.34 (s, 1H), 8.05(b d, J= 7.5Hz, 1H), 7.94 (b d, J= 8Hz, 1H), 7.88 (d, J=8.5Hz, 1H), 7.85-7.78 (m, 2H), 7.59-7.43 (m, 4H), 4.99 (d, J= 12Hz, 1H), 4.89 (d, J= 12Hz, 1H), 4.41-4.05 (m, 5H), 3.82-3.66 (m, 1H), 2.25-2.11 (m, 1H), 2.11-1.98 (m, 1H), 1.90 & 1.84 (s, 3H), 1.78-1.40 (m, 15H), 1.39-0.82 (m, 12H), 0.90 (t, J= 7, 14Hz, 3H).

### EXAMPLE 19

### Synthesis of compounds 103 of Table 1

Following the procedure described for the synthesis of compound **104** of Example 17, the mixtures of 1(*R*), 2(*R*) and 1(*R*),2(*S*) isomers of intermediate compound **10d**, prepared in Example 10, were coupled with compound **2** to give a mixture of isomeric intermediate compounds **19a** and **19b**

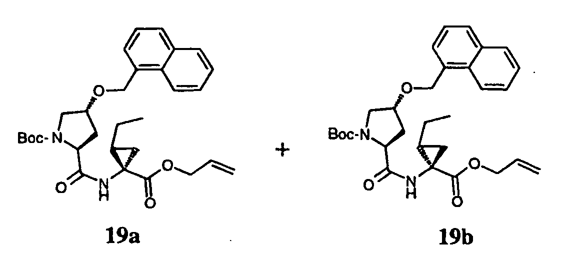

Following the procedures of Example 18, isomeric compounds **19a** and **19b** were separated and transformed into their corresponding compound of formula 1; to isolate the corresponding compound **103** of Table 1.

Spectral data:
Compound **103:** Rotamer population by NMR ca. (1:8.7):
MS (FAB) m/z: 703 (MH+); ¹H-NMR (DMSO-d₆) δ8.21-8.09 (bs, 1H), 8.05 (bd, J = 7.63 Hz, 1H), 7.94 (bd, J = 7.0 Hz, 1H), 7.91-7.83 (m, 2H), 7.83-7.76 (m, 1H), 7.59-7.5 (m, 3H), 7.5-7.43 (m, 1H), 4.99 (d, J = 11.8 Hz, 1H), 4.89 (d, J = 11.8 Hz, 1H), 4.43-4.30 (m, 3H), 4.23-4.16 (m, 1H), 4.13 (bd, J = 10.8 Hz, 1H), 3.71 (dd, J = 11.1, 4 Hz, 1H), 2.2-2.02 (m, 2H), 1.87 and 1.84 (2 x s, 3H), 1.81-1.71 (m, 2H), 1.70-1.40 (m, 12H), 1.26-1.06 (m, 4H), 1.04-0.83 (m, 11H), 0.59 (m, 1H).

### EXAMPLE 20

### Synthesis of compound 108 of Table 1

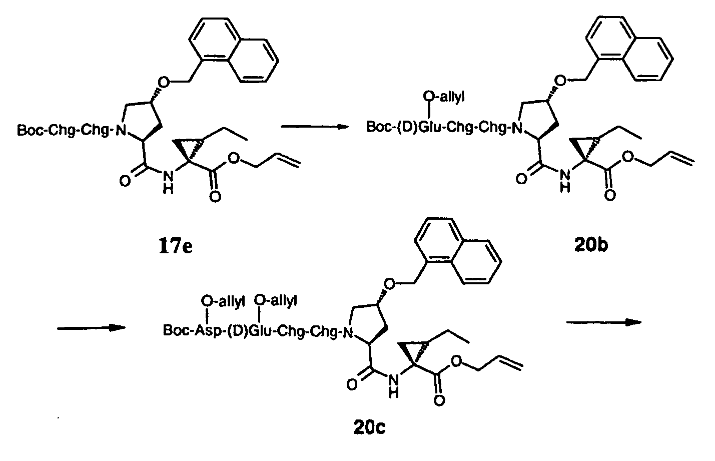

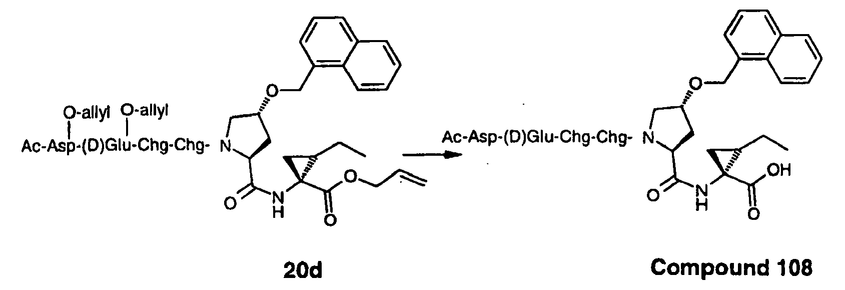

The crude tetrapeptide **17e** from Example 17 (ca.0.963 mmol) was treated with 4N HCl /dioxane solution (5 mL) as described for compound **15c.** The crude hydrochloride salt was coupled to Boc-(D)Glu(O-allyl)-OH (331.9 mg, 1.155 mmol) with NMM (423 µl, 3.850 mmol) and TBTU (370.8 mg, 1.155 mmol) in DCM (5 mL) for 3 h at RT as described for compound **15d**. The crude pentapeptide **20b** was obtained as an ivory foam (ca.933.9 mg, 0.963 mmol). MS (FAB) 968.6 MH⁻ 970.6 MH⁺ 992.5 (M+Na).

¹H NMR (CDCl₃), ca.1 : 4 mixture of rotamers, δ 8.05 (d, J= 8.5Hz, 1H), 7.87 (b d, J= 7.5Hz, 1H), 7.81 (d, J= 8.5Hz, 1H), 7.58-7.34 (m, 5H), 6.77-6.25 (m, 2H), 5.98-5.77 (m, 2H), 5.38-5.21 (m, 4H), 5.16 (dd, J= 1.5, 10.5Hz, 1H), 5.06-4.89 (m, 2H), 4.68-4.13 (m, 7H), 3.96-3.52 (m, 4H), 2.69-2.38 (m, 3H), 2.23-1.87 (m, 2H), 1.78-1.37 (m, 17H), 1.46 & 1.44 (s, 9H), 1.22-0.87 (m, 11H), 0.95 (t, J= 7, 14.5Hz, 3H).

The crude pentapeptide **20b** (ca.0.963 mmol) was treated with 4N HCl /dioxane solution (5 mL) as described for compound **15c.** The crude hydrochloride salt was coupled to Boc-Asp(O-allyl)-OH (315.6 mg, 1.155 mmol) with NMM (423 µl, 3.85 mmol) and TBTU (370.8 mg, 1.155 mmol) in DCM (5 mL) as described for compound **15d**. The crude hexapeptide **20c** was obtained as an ivory foam (ca.1.083 g, 0.963 mmol). MS (FAB) 1147.6 (M+Na)⁺. ¹H NMR (CDCl₃), ca.1:1 mixture of rotamers, δ 8.06 (b d, J= 8Hz, 1H), 7.86 (d, J= 8Hz, 1H), 7.81 (d, J= 8Hz, 1H), 7.59-7.39 (m, 5H), 7.39-6.34 (m, 4H), 5.98-5.76 (m, 3H), 5.38-5.10 (m, 6H), 5.10-4.89 (m, 2H), 4.66-4.05 (m, 10H), 3.87-3.58 (m, 4H), 3.30-2.65 (m, 2H), 2.65-1.89 (m 3H), 1.79-1.33 (m, 19H), 1.47 & 1.45 (s, 9H), 1.33-0.86 (m, 14H).

The crude hexapeptide **20c** (ca.0.963 mmol) was treated with 4N HCl /dioxane solution (5 mL) as described for compound 15c. The crude hydrochloride was acetylated with acetic anhydride (182 µl, 1.193 mmol) and NMM (423.5 µL, 3.850 mmol) in DCM (5 mL) as described for compound **15f** to provide the crude acetylated tetrapeptide. The foam residue was purified by flash chromatography (eluent : 1^{st} hexane : EtOAc 20:80 to 10:90 and 2^{nd} pure EtOAc) to provide the acetylated hexapeptide **20d** as an ivory foam (528 mg, 51% yield over 4 steps). MS (FAB) 1067.6 (MH+) 1089.6 (M+Na).

The acetylated hexapeptide **20d** (528 mg, 0.495 mmol) was dissolved in DCM (3 mL) and treated with a premixed, 15 min stirred solution of tetrakis(triphenylphosphine)-palladium (0) catalyst (90 mg, 0.078 mmol) and pyrrolidine (134 µL, 1.603 mmol) in DCM (3 mL). The reaction mixture was stirred at RT for 48 h after which the solvent was evaporated. The crude product was purified partially by trituration in Et₂O: DCM (85:15), then, purified in two batches by preparatory HPLC. Half of the partially purified material was dissolved in glacial HOAc (5 mL), filtered through a Millipore®: Millex®- HV 0.45µm filter and injected onto an equilibrated Whatman Partisil® 10-ODS-3 (2.2 x50cm) C18 reverse phase column. Purification program : linear gradient at 15 mL/min, 230µm, injected at 5% A; once all HOAc had eluted the program was begun - at 5% A for 10 min, 5-58% A in 70 min; A: 0.06%TFA / CH₃CN; B : 0.06%TFA / H₂O. Fractions were analyzed by analytical HPLC, appropriate fractions from both HPLC purifications were collected and lyophilized to provide the desired hexapeptide compound 108, as a white amorphous solid (218.3 mg, 47% yield).

MS (FAB) 945.5 MH- 947.4 MH+ 969.5 (M+Na)+ 985.4 (M+K)+. ¹H NMR (DMSO), ca.1:9 mixture of rotamers, δ 8.55 & 8.31 (s, 1H), 8.16 (d, J= 7.5Hz, 1H), 8.11 (d, J= 8Hz, 1H), 8.05 (d, J= 8.5Hz, 1H), 7.97-7.85 (m, 2H), 7.88 (d, J= 8.5Hz, 1H), 7.75 (d, J= 9Hz, 1H), 7.59-7.39 (m, 4H), 4.99 (d, J= 12Hz, 1H), 4.89 (d, J= 12Hz, 1H), 4.53 (dd, J= 7, 14Hz, 1H), 4.08-4.45 (m, 6H), 3.77 (b dd, J= 4, 11Hz, 1H), 2.64 (dd, J= 6.5, 16.5Hz, 1H), 2.48-2.41 (m, 1H), 2.25-2.12 (m, 3H), 2.07 & 1.82 (s, 3H), 2.04-1.86 (m, 2H), 1.80-1.35 (m, 14H), 1.32-0.80 (m, 14H), 0.91 (t, J= 7.5, 14.5Hz, 3H).

### EXAMPLE 21

### Synthesis of compound 301 of Table 3

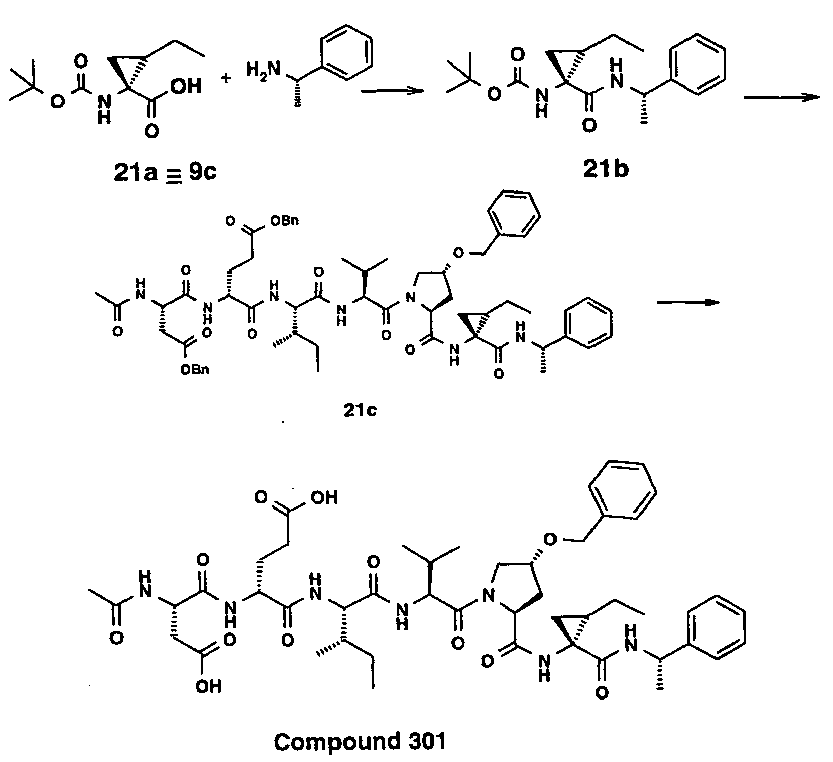

A solution of lithium hydroxide monohydrate (23 mg, 0.56 mmol) in H₂O (4 mL) was added to a solution of the ester compound **21a** (45 mg, 0.185 mmol, described previously as the (*R,R*) isomer **9c**) in MeOH (3.5 mL) and THF (3.5 mL). The resulting solution was stirred vigorously for 16 h and then partitioned between EtOAc (60 mL) and 10% aqueous HCl (20 mL). The organic phase was separated, dried (MgSO₄), filtered and concentrated to give the corresponding acid in quantitative yield.

This material (ca. 0.185 mmol) was combined with (S)-(-)-α-methylbenzylamine (27 mg, 0.22 mmol), HATU (77 mg, 0.20 mmol), and DIPEA (0.11 mL, 0.65 mmol) in DMF (5 mL). After 20 h, the reaction was concentrated. The residue dissolved in EtOAc and the solution was washed sequentially with saturated aqueous NaHCO₃, 10% aqueous HCl, and brine before being dried (MgSO₄), filtered and concentrated *in vacuo*. Purification by flash chromatography (eluent: 35% EtOAc/hexane) gave 11 mg (28%) of the coupled product **21b.** This material (11 mg, 0.033 mmol) was treated with 4N HCl/dioxane for 35 min. The reaction mixture thereafter was concentrated to dryness to give the hydrochloride salt of the corresponding amine. The latter product was coupled with:

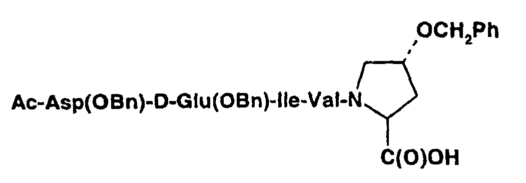

(33 mg, 0.036 mmol, prepared by procedures analogous to those of Example 15 and 20), HATU (14 mg, 0.036 mmol) and DIPEA (0.116 mL, 0.02 mmol) in DMF (4 mL). After the reaction mixture has been stirred 16 h, the mixture was concentrated. The residue was dissolved in EtOAc. The solution was washed sequentially with saturated aqueous NaHCO₃, 10% aqueous HCl and brine, dried (MgSO₄), filtered and concentrated *in vacuo* to give a white solid. This material (ca. 0.033) was dissolved in EtOH (6 mL) and treated with ammonium acetate (7 mg, 0.09 mmol) and 10% Pd/C (10 mg) under an atmosphere of hydrogen gas. After 3 h, the reaction mixture was filtered through diatomaceous earth. The filtrate was concentrated to dryness. The residue was then dissolved in DMSO and purified by preparative HPLC to give a white solid after lyophilization (17.6 mg, 57% yield over two steps).

Spectral data: MS (FAB) ES⁻ 932.6 (M-H)⁻, 954.5 (M-Na)⁻; HRMS calcd for C₄₈H₆₇N₇O₁₂ (MH⁺) 934.49261, found: 934.49010; ¹H-NMR (DMSO,d₆) δ 8.90 (s, 1H), 8.24 (d, J = 7.95 Hz, 1H), 8.14 (d, J = 7.63 Hz, 1H), 7.99 (d, J = 8.26 Hz, 1H), 7.79 (d, J = 8.9 Hz, 1H), 7.75 (d, J = 8.26 Hz, 1H), 7.42-7.17 (m, 10 H), 5.00 (quintet, J = 7.63 Hz, 1H), 4.7 (m, 1H), 4.52 (d, J = 11.76 Hz, 1H), 4.43 (d, J = 11.4 Hz, 1H), 4.33-4.2 (m, 6H), 3.70 (dd, J = 11.4 and 11.1 Hz, 2H), 2.63 (dd, J = 5.7 and 5.7 Hz, 1H), 2.45 (dd, J = 7.95 and 7.95 Hz, 1H), 2.21-2.11 (m, 3H), 2.07-1.97 (m, 1H), 1.93-1.83 (m, 2H), 1.81 (s, 3H), 1.78-1.63 (m, 2H), 1.54-1.41 (m, 2H), 1.39 (d, J = 7.0 Hz, 3H), 1.29 (dd, J = 7.94 and 7.63 Hz, 1H), 1.15 (quintet, J = 7.0 Hz, 1H), 1.05 (m, 1H), 0.90 (d, J = 6.36 Hz, 6H), 0.88-0.83 (m, 1H), 0.71 (m, 9H).

### EXAMPLE 22

Compound **107** of Table 1 was synthesized according to the protocol described in Example 17.

### Rotamer population by NMR (1:7.6)

MS (FAB) m/z: 675 (MH+); 1H-NMR (DMSO-d6) δ 8.35-8.19 (bs, 1H), 8.04 (d, J = 7.63 Hz, 1H), 7.93 (bd, J = 7.31 Hz, 1H), 7.88 (d, J = 8.27 Hz, 1H), 7.86-7.79 (m, 2H), 7.59-7.49 (m, 3H), 7.46 (dd, J = 7.95, 7.95 Hz, 1H), 4.98 (d, J = 11.8 Hz, 1H), 4.89 (d, J = 11.8 Hz, 1H), 4.40-4.34 (m, 1H), 4.32 (bs, 1H), 4.29-4.24 (m, 1H), 4.22-4.15 (m, 1H), 4.09 (d, J = 11.8 Hz, 1H), 3.74 (dd, J = 11.1, 4 Hz, 1H), 2.20-2.12 (m, 1H), 2.05-1.94 (m, 2H), 1.84 (s, 3H), 1.72-1.42 (m, 7H), 1.20-1.13 (m, 1H), 1.08-0.87 (m, 13H), 0.85 (d, J = 6.68 Hz, 6H).

### EXAMPLE 23

Compound **114** of Table 1 was synthesized according to the protocol described in Example 17.

### Rotamer population by NMR (1:7.5):

MS (FAB) m/z: 747 (M+Na⁺); ¹H-NMR (DMSO-d₆) δ 8.40-8.24 (bs, 1H), 8.07-8.01 (m, 1H), 7.96-7.91 (m, 1H), 7.87 (d J = 8.26 Hz, 1H), 7.85-7.78 (m, 2H), 7.58-7.49 (m, 3H), 7.46 (dd, J = 7.95, 7.95 Hz, 1H), 7.30-7.21 (m, 4H), 7.20-7.14 (m, 1H), 4.98 (d, J = 11.8 Hz, 1H), 4.89 (d, J = 11.8 Hz, 1H), 4.40-4.34 (m, 1H), 4.34-4.29 (m, 1H), 4.29-4.25 (m, 1H), 4.22-4.15 (m,1H), 4.09 (d, J = 11.8 Hz, 1H), 3.74 (dd, J = 11.1, 4 Hz, 1H), 2.95-2.79 (m, 2H), 2.21-2.11 (m, 1H), 2.05-1.94 (m, 2H), 1.89-1.83 (2 x s, 3H), 1.63-1.41 (m, 7H), 1.38-1.30 (m, 1H), 1.27-1.22 (m, 1H), 1.12-0.94 (m, 5H), 0.89 (d, J = 6.4 Hz, 3H), 0.84 (d, J = 6.4 Hz, 3H).

### EXAMPLE 24

Compound **118** of Table **1** was synthesized according to the protocol described in Example 17.

### Rotamer population by NMR ca. (1:6.3):

MS (FAB) m/z: 677.4 (MH⁺); ¹H-NMR (DMSO-d₆) δ 8.58 and 8.38 (2 x bs, 1H), 8.04 (d, J = 7.63 Hz, 1H), 7.93 (d, J = 7.63 Hz, 1H), 7.91-7.81 (m, 3H), 7.59-7.49 (m, 3H), 7.49-7.43 (m, 1H), 4.98 (d, J = 12.1 Hz, 1H), 4.89 (d, J = 12.1 Hz, 1H), 4.41-4.29 (m, 2H), 4.29-4.14 (m, 2H), 4.1 (d, J = 10.8 Hz, 1H), 3.74 (bd, J = 7.63 Hz, 1H), 2.21-2.12 (m, 1H), 2.04-1.92 (m, 2H), 1.90 and 1.84 (2 x s, 3H), 1.63-1.41 (m, 9H), 1.39-1.26 (m, 3H), 1.21-1.15 (m, 1H), 1.06-0.92 (m, 5H), 0.92-0.80 (m, 9H).

### EXAMPLE 25

Compound **116** of Table 1 was synthesized according to the protocol described in Example 17.

¹H NMR (DMSO-d₆) δ 8.36 (s, 1H), 8.14 (d, J = 8 Hz, 1H), 8.04 (d, J = 8 Hz, 1H), 7.99 (d, J = 9 Hz, 1 H), 7.79 (d, J = 9 Hz, 1 H), 7.33-7.26 (m, 5 H), 4.54-4.42 (m, 3 H), 4.30-4.21 (m, 5 H), 4.06 (d, J = 11 Hz, 1 H), 3.69 (dd, J = Hz, 1 H), 2.62 (dd, J = 16, 10 Hz, 1 H), 2.47-2.42 (m, 1 H), 2.18-2.14 (m, 3 H), 2.02-1.87 (m, 2 H), 1.82 (s, 3 H), 1.74-1.66 (m, 2 H), 1.54-1.47 (m, 2 H), 1.38-1.27 (m, 2 H), 1.21-1.18 (m, 1 H), 0.97-0.85 (m, 11 H), 0.80-0.70 (m, 7 H).

### EXAMPLE 26

Compound **121** of Table **1** was synthesized according to the protocol described in Example 17.

¹H NMR (DMSO-d₆) δ 9.12 (d, J = 6 Hz, 1 H), 8.64 (s, 1 H), 8.30 (d, J = 8 Hz, 1 H), 8.12 (d, J = 9 Hz, 1 H), 8.05 (dd, J = 8, 7 Hz, 1 H), 7.97 (d, J = 8 Hz, 1 H), 7.80 (dd, J = 8, 7 Hz, 1 H), 7.66 (d, J = 9 Hz, 1 H), 7.54 (d, J = 6 Hz, 1 H), 5.70-5.61 (m, 2 H), 5.26 (d, J = 17 Hz, 1 H), 5.07 (d, J = 12 Hz, 1 H), 4.52 (d, J = 12 Hz, 1 H), 4.39 (dd, J = 9, 8 Hz, 1 H), 4.23-4.12 (m, 2 H), 4.03-3.99 (m, 1 H), 2.66-2.54 (m, 1 H), 2.35-2.28 (m, 1 H), 2.08 (dd, J = 9, 17 Hz, 1 H), 2.01-1.93 (m, 1 H), 1.83 (s, 3 H), 1.65-1.46 (m, 5 H), 1.41-1.38 (m, 1 H), 1.24-1.20 (dd, J = 9, 5 Hz, 1 H), 01.05-0.78 (m, 12 H).

### EXAMPLE 27

Compound **205** of Table 2 was synthesized according to the protocol described in Example 17.

¹H NMR (DMSO-d₆) δ 9.14 (d, J = 6 Hz, 1 H), 8.60 (s, 1 H), 8.32 (d, J = 8 Hz, 1 H), 8.14-8.06 (m, 2 H), 7.98 (d, J = 8 Hz, 1 H), 7.82 (dd, J = 8, 7 Hz, 1 H), 7.66 (d, J = 9 Hz, 1 H), 7.55 (d, J = 8 Hz, 1 H), 5.75-5.66 (m, 2 H), 5.22 (d, J = 17 Hz, 1 H), 5.07 (d, J = 10 Hz, 1 H), 4.50 (d, J = 12 Hz, 1 H), 4.39 (dd, J = 9, 9 Hz, 1 H), 4.23-4.08 (m, 3 H), 2.56-2.50 (m, 1 H), 2.36-2.28 (m, 1 H), 2.04-1.97 (m, 1 H), 1.82 (s, 3 H), 1.62-1.41 (m, 7 H), 1.24 (dd, J = 5, 4 Hz, 1 H), 0.94-0.75 (m, 12 H).

### EXAMPLE 28

Compound **117** of Table 1 was synthesized according to the protocol described in Example 20.

¹H NMR (DMSO-d₆) δ 8.36 (s, 1 H), 8.17 (d, J = 8 Hz, 1 H), 8.09 (d, J = 8 Hz, 1 H), 8.04 (d, J = 8 Hz, 1 H), 7.96-7.92 (m, 2 H), 7.87 (d, J = 8 Hz, 1 H), 7.77 (d, J = 9 Hz, 1 H), 7.56-7.45 (m, 4 H), 4.99 (d, J = 12 Hz, 1 H), 4.89 (d, J = 12 Hz, 1 H), 4.52 (dd, J = 14, 7 Hz, 1 H), 4.37-4.12 (m, 6 H), 3.78-3.73 (m, 1 H), 2.63 (dd, J = 17, 6 Hz, 1 H), 2.47-2.42 (m, 1 H), 2.22-2.16 (m, 3 H), 2.04-1.86 (m, 2 H), 1.82 (s, 3 H), 1.77-1.71 (m, 1 H), 1.69-1.42 (m, 8 H), 1.30 (quint., J = 8 Hz, 1 H), 1.20 (dd, J = 12, 8 Hz, 1 H), 1.10-0.85 (m, 15 H), 0.76-0.72 (m, 1 H).

### EXAMPLE 29

Compound **120** of Table 1 was synthesized according to the protocol described in Example 20.

¹H NMR (DMSO-d₆) δ 8.34 (s, 1 H), 8.12 (d, J = 8 Hz, 1 H), 8.05 (d, J = 8 Hz, 1 H), 7.95-7.87 (m, 3 H), 7.81 (d, J = 9 Hz, 1 H), 7.64-7.52 (m, 4 H), 7.46 (dd, J = 8, 7 Hz, 1 H), 4.99 (d, J = 12 Hz, 1 H), 4.89 (d, J = 12 Hz, 1 H), 4.63 (dd, J = 14, 7 Hz, 1 H), 4.37-4.14 (m, 4 H), 3.74 (dd, J = 11, 4 Hz, 1 H), 3.41-3.35 (m, 2 H), 2.61 (dd, J = 16, 7 Hz, 1 H), 2.44 (dd, J = 16, 8 Hz, 1 H), 2.20-2.15 (m, 1 H), 2.04-1.96 (m, 3 H), 1.82 (s, 3 H), 1.70-1.64 (m, 1 H), 1.56-1.43 (m, 7 H), 1.30 (quint., J = 8 Hz, 1 H), 1.20 (dd, J = 8, 5 Hz, 1 H), 0.99-0.72 (m, 21 H).

### EXAMPLE 30

### Cloning, expression and purification of the recombinant HCV NS3 protease type 1b.

Serum from an HCV-infected patient was obtained through an external collaboration (Bernard Willems MD, Hôpital St-Luc, Montréal, Canada and Dr. Donald Murphy, Laboratoire de Santé Publique du Québec, Ste-Anne de Bellevue, Canada). An engineered full-length cDNA template of the HCV genome was constructed from DNA fragments obtained by reverse transcription-PCR (RT-PCR) of serum RNA and using specific primers selected on the basis of homology between other genotype 1b strains. From the determination of the entire genomic sequence, a genotype 1 b was assigned to the HCV isolate according to the classification of Simmonds et al. (J. Clin. Microbiol., (1993), 31, p.1493-1503). The amino acid sequence of the non-structural region, NS2-NS4B, was shown to be greater than 93% identical to HCV genotype 1 b (BK, JK and 483 isolates) and 88% identical to HCV genotype 1a (HCV-1 isolate). A DNA fragment encoding the polyprotein precursor (NS3/NS4A/NS4B/NS5A/NS5B) was generated by PCR and introduced into eukaryotic expression vectors. After transient transfection, the polyprotein processing mediated by the HCV NS3 protease was demonstrated by the presence of the mature NS3 protein using Western blot analysis. The mature NS3 protein was not observed with expression of a polyprotein precursor containing the mutation S1165A, which inactivates the NS3 protease, confirming the functionality of the HCV NS3 protease.

The DNA fragment encoding the recombinant HCV NS3 protease (amino acid 1027 to 1206) was cloned in the pET11d bacterial expression vector. The NS3 protease expression in *E. coli* BL21 (DE3)pLysS was induced by incubation with 1 mM IPTG for 3 h at 22°C. A typical fermentation (18 L) yielded approximately 100 g of wet cell paste. The cells were resuspended in lysis buffer (3.0 mUg) consisting of 25 mM sodium phosphate, pH 7.5, 10% glycerol (*v*/*v*), 1 mM EDTA, 0.01% NP-40 and stored at -80°C. Cells were thawed and homogenized following the addition of 5 mM DTT. Magnesium chloride and DNase were then added to the homogenate at final concentrations of 20 mM and 20 µg/mL respectively. After a 25 min incubation at 4°C, the homogenate was sonicated and centrifuged at 15000 x *g* for 30 min at 4°C. The pH of the supernatant was then adjusted to 6.5 using a 1M sodium phosphate solution.

An additional gel filtration chromatography step was added to the 2 step purification procedure described in WO 95/22985 (incorporated herein by reference). Briefly, the supernatant from the bacterial extract was loaded on a SP HiTrap column (Pharmacia) previously equilibrated at a flow rate of 2 mL/min in buffer A (50 mM sodium phosphate, pH 6.5, 10% glycerol, 1 mM EDTA, 5 mM DTT, 0.01% NP-40). The column was then washed with buffer A containing 0.15 M NaCl and the protease eluted by applying 10 column volumes of a linear 0.15 to 0.3 M NaCl gradient. NS3 protease-containing fractions were pooled and diluted to a final NaCl concentration of 0.1 M. The enzyme was further purified on a HiTrap Heparin column (Pharmacia) equilibrated in buffer B (25 mM sodium phosphate, pH 7.5, 10% glycerol, 5 mM DTT, 0.01% NP-40). The sample was loaded at a flow rate of 3 mL/min. The column was then washed with buffer B containing 0.15 M NaCl at a flow rate of 1.5 mL/min. Two step washes were performed in the presence of buffer B containing 0.3 or 1 M NaCl. The protease was recovered in the 0.3M NaCl wash, diluted 3-fold with buffer B, reapplied on the HiTrap Heparin column and eluted with buffer B containing 0.4 M NaCl. Finally, the NS3 protease-containing fractions were applied on a Superdex 75 HiLoad 16/60 column (Pharmacia) equilibrated in buffer B containing 0.3 M NaCl. The purity of the HCV NS3 protease obtained from the pooled fractions was judged to be greater than 95% by SDS-PAGE followed by densitometry analysis.

The enzyme was stored at -80°C and was thawed on ice and diluted just prior to use.

### EXAMPLE 31

### Recombinant HCV NS3 protease/NS4A cofactor peptide radiometric assay.

The enzyme was cloned, expressed and prepared according to the protocol described in Example 30. The enzyme was stored at -80°C, thawed on ice and diluted just prior to use in the assay buffer containing the NS4A cofactor peptide.

The substrate used for the NS3 protease/ NS4A cofactor peptide radiometric assay, DDIVPC-SMSYTW, is cleaved between the cysteine and the serine residues by the enzyme. The sequence DDIVPC-SMSYTW corresponds to the NS5A/NS5B natural cleavage site in which the cysteine residue in P2 has been substituted for a proline. The peptide substrate DDIVPC-SMSYTW and the tracer biotin-DDIVPC-SMS[¹²⁵I-Y]TW are incubated with the recombinant NS3 protease and the NS4A peptide cofactor KKGSVVIVGRIILSGRK (molar ratio enzyme: cofactor 1:100) in the absence or presence of inhibitors. The separation of substrate from products is performed by adding avidin-coated agarose beads to the assay mixture followed by filtration. The amount of SMS[¹²⁵I-Y]TW product found in the filtrate allows for the calculation of the percentage of substrate conversion and of the percentage of inhibition.

### A. Reagents

Tris and Tris-HCl (UltraPure) were obtained from Gibco-BRL. Glycerol (UltraPure), MES and BSA were purchased from Sigma. TCEP was obtained from Pierce, DMSO from Aldrich and NaOH from Anachemia.

Assay buffer: 50 mM Tris HCl, pH 7.5, 30% (*w*/*v*) glycerol, 1 mg/mL BSA, 1 mM TCEP (TCEP added just prior to use from a 1 M stock solution in water).

Substrate: DDIVPCSMSYTW, 25 µM final concentration (from a 2 mM stock solution in DMSO stored at -20°C to avoid oxidation).

Tracer: reduced mono iodinated substrate biotin DDIVPC SMS[¹²⁵I Y]TW (∼1 nM final concentration).

HCV NS3 protease type 1b, 25 nM final concentration (from a stock solution in 50 mM sodium phosphate, pH 7.5, 10% glycerol, 300 mM NaCl, 5 mM DTT, 0.01% NP-40).

NS4A Cofactor peptide: KKGSVVIVGRIILSGRK, 2.5 µM final concentration (from a 2 mM stock solution in DMSO stored at -20°C).

### B. Protocol

The assay was performed in a 96-well polypropylene plate from Costar. Each well contained:
■ 20 µL substrate/tracer in assay buffer;
■ 10 µL ± inhibitor in 20% DMSO/assay buffer;
■ 10 µL NS3 protease 1 b/NS4 cofactor peptide (molar ratio 1:100).

Blank (no inhibitor and no enzyme) and control (no inhibitor) were also prepared on the same assay plate.

The enzymatic reaction was initiated by the addition of the enzyme/NS4A peptide solution and the assay mixture was incubated for 40 min at 23°C under gentle agitation. Ten (10) µL of 0.5N NaOH were added and 10 µL 1 M MES, pH 5.8 were added to quench the enzymatic reaction.

Twenty (20) µL of avidin-coated agarose beads (purchased from Pierce) were added in a Millipore MADP N65 filtration plate. The quenched assay mixture was transferred to the filtration plate, and incubated for 60 min at 23°C under gentle agitation.

The plates were filtered using a Millipore MultiScreen Vacuum Manifold Filtration apparatus, and 40 µL of the filtrate was transferred in an opaque 96-well plate containing 60 µL of scintillation fluid per well.

The filtrates were counted on a Packard TopCount instrument using a ¹²⁵I-liquid protocol for 1 minute.

The % inhibition was calculated with the following equation: $100 - [ ( {counts}_{inh} - {counts}_{blank} ) / ( {counts}_{ctl} - {counts}_{blank} ) \times 100 ]$

A non-linear curve fit with the Hill model was applied to the inhibition-concentration data, and the 50% effective concentration (IC₅₀) was calculated by the use of SAS software (Statistical Software System; SAS Institute, Inc. Cary, N.C.).

### EXAMPLE 32

### Full-length NS3-NS4A heterodimer protein assay

The NS2-NS5B-3' non coding region was cloned by RT-PCR into the pCR®3 vector (Invitrogen) using RNA extracted from the serum of an HCV genotype 1b infected individual (provided by Dr. Bernard Willems, Hôpital St-Luc, Montréal, Québec, Canada). The NS3-NS4A DNA region was then subcloned by PCR into the pFastBac™ HTa baculovirus expression vector (Gibco/BRL). The vector sequence includes a region encoding a 28-residue N-terminal sequence which contains a hexahistidine tag. The Bac-to-Bac™ baculovirus expression system (Gibco/BRL) was used to produce the recombinant baculovirus. The full length mature NS3 and NS4A heterodimer protein (His-NS3-NS4AFL) was expressed by infecting 10⁶ Sf21 cells/mL with the recombinant baculovirus at a multiplicity of infection of 0.1-0.2 at 27°C. The infected culture was harvested 48 to 64 h later by centrifugation at 4°C. The cell pellet was homogenized in 50mM NaPO₄, pH 7.5, 40% glycerol (w/v), 2mM β-mercaptoethanol, in presence of a cocktail of protease inhibitors. His-NS3-NS4AFL was then extracted from the cell lysate with 1.5% NP-40, 0.5% Triton X-100, 0.5M NaCl, and a DNase treatment. After ultracentrifugation, the soluble extract was diluted 4-fold and bound on a Pharmacia Hi-Trap Ni-chelating column. The His-NS3-NS4AFL was eluted in a >90% pure form (as judged by SDS-PAGE), using a 50 to 400 mM imidazole gradient. The His-NS3-NS4AFL was stored at -80°C in 50 mM sodium phosphate, pH 7.5, 10% (w/v) glycerol, 0.5 M NaCl, 0.25 M imidazole, 0.1 % NP-40. It was thawed on ice and diluted just prior to use.

The protease activity of His-NS3-NS4AFL was assayed in 50 mM Tris-HCl, pH 8.0, 0.25 M sodium citrate, 0.01% (w/v) n-dodecyl-β-D-maltoside, 1 mM TCEP. Five (5) µM of the internally quenched substrate anthranilyl-DDIVPAbu[C(O)-O]-AMY(3-NO₂)TW-OH in presence of various concentrations of inhibitor were incubated with 1.5 nM of His-NS3-NS4AFL for 45 min at 23°C. The final DMSO concentration did not exceed 5.25%. The reaction was terminated with the addition of 1M MES, pH 5.8. Fluorescence of the N-terminal product was monitored on a Perkin-Elmer LS-50B fluorometer equipped with a 96-well plate reader (excitation wavelength: 325 nm; emission wavelength: 423 nm). A non-linear curve fit using the Hill model was then applied to the % inhibition-concentration data and 50% effective concentration (IC₅₀) was calculated through the use of SAS (Statistical Software System, SAS Institute Inc., Cary, N.C.).

### EXAMPLE 33

### NS3 Protease Cell-based assay

This assay was done with Huh-7 cells, a human cell line derived from a hepatoma, co-transfected with 2 DNA constructs:
- one expressing a polyprotein comprising the HCV non-structural proteins fused to tTA in the following order: NS3-NS4A-NS4B-NS5A-tTA (called NS3);
- the other expressing the reporter protein, secreted alkaline phosphatase, under the control of tTA (called SEAP).

The polyprotein must be cleaved by the NS3 protease for the mature proteins to be released. Upon release of the mature proteins, it is believed that the viral proteins will form a complex at the membrane of the endoplasmic reticulum while tTA will migrate to the nucleus and transactivate the SEAP gene. Therefore, reduction of NS3 proteolytic activity should lead to reduction of mature tTA levels and concomitant decrease in SEAP activity.

To control for other effects of the compounds, a parallel transfection was done where a construct expressing tTA alone (called tTA) was co-transfected with the SEAP construct such that SEAP activity is independent of NS3 proteolytic activity.

Protocol of the assay: Huh-7 cells, grown in CHO-SFMII + 10% FCS (fetal calf serum), were co-transfected with either NS3 and SEAP or tTA and SEAP, using the FuGene protocol (Boehringer Mannheim). After 5 h at 37°, the cells were washed, trypsinized and plated (at 80 000 cells/well) in 96-well plates containing a range of concentrations of the compounds to be tested. After a 24-h incubation period, an aliquot of the medium was drawn and the SEAP activity in this aliquot was measured with the Phospha-Light kit (Tropix).

Analysis of the percent inhibition of SEAP activity with respect to compound concentration was performed with the SAS software to obtain the EC₅₀.

The toxicity of the compound (TC₅₀) was then assessed using the **MTT** assay as follows:

20µL of a MTT solution (5mg/ml medium) was added per well and incubated at 37° for 4 hrs;
the medium was removed and 50 µl of 0.01 N HCl + 10% Triton X-100 was added;
after shaking at RT for at least 1 hr, the OD of each well was read at 595 nm wavelength.

The TC₅₀ was calculated in the same way as the EC₅₀.

### EXAMPLE 34

### Specificity assays

The specificity of the compounds was determined against a variety of serine proteases: human leukocyte elastase, porcine pancreatic elastase and bovine pancreatic α-chymotrypsin and one cysteine protease: human liver cathepsin B. In all cases a 96-well plate format protocol using a colorimetric p-nitroaniline (pNA) substrate specific for each enzyme was used. Each assay included a 1 h enzyme-inhibitor pre-incubation at 30°C followed by addition of substrate and hydrolysis to ≈30% conversion as measured on a UV Thermomax® microplate reader. Substrate concentrations were kept as low as possible compared to K_{M} to reduce substrate competition. Compound concentrations varied from 300 to 0.06 µM depending on their potency. The final conditions for each assay were as follows:
50 mM Tris-HCl pH 8, 0.5 M Na₂SO₄, 50 mM NaCl, 0.1 mM EDTA, 3% DMSO, 0.01% Tween® 20 with;
[100 µM Succ-AAPF-pNA and 250 pM α-chymotrypsin], [133 µM Succ-AAA-pNA and 8 nM porcine elastase], [133 µM Succ-AAV-pNA and 8 nM leukocyte elastase]; or
[100 mM NaHPO₄ pH 6, 0.1 mM EDTA, 3% DMSO, 1 mM TCEP, 0.01% Tween® 20, 30 µM Z-FR-pNA and 5 nM cathepsin B (the stock enzyme was activated in buffer containing 20 mM TCEP before use)).

A representative example is summarized below for porcine pancreatic elastase:
In a polystyrene flat-bottom 96-well plate were added using a Biomek liquid handler (Beckman):
- 40 µL of assay buffer (50 mM Tris-HCl pH 8, 50 mM NaCl, 0.1 mM EDTA);
- 20 µL of enzyme solution (50 mM Tris-HCl pH 8, 50 mM NaCl, 0.1 mM EDTA. 0.02% Tween® 20, 40 nM porcine pancreatic elastase); and
- 20 µL of inhibitor solution (50 mM Tris-HCl, pH 8, 50 mM NaCl, 0.1 mM EDTA, 0.02% Tween® 20, 1.5 mM-0.3 µM inhibitor, 15% v/v DMSO).

After 60 min pre-incubation at 30°C, 20 µL of substrate solution (50 mM Tris/HCl, pH 8, 0.5 M Na₂SO₄. 50 mM NaCl, 0.1 mM EDTA, 665 µM Succ-AAA-pNA) were added to each well and the reaction was further incubated at 30°C for 60 min after which time the absorbance was read on the UV Thermomax® plate reader. Rows of wells were allocated for controls (no inhibitor) and for blanks (no inhibitor and no enzyme).

The sequential 2-fold dilutions of the inhibitor solution were performed on a separate plate by the liquid handler using 50 mM Tris-HCl pH 8, 50 mM NaCl, 0.1 mM EDTA, 0.02% Tween® 20, 15% DMSO. All other specificity assays were performed in a similar fashion.

The percentage of inhibition was calculated using the formula: $[ 1 - ( ( U {V}_{inh} - U {V}_{blank} ) / ( U {V}_{ctl} - U {V}_{blank} ) ) ] \times 100$

A non-linear curve fit with the Hill model was applied to the inhibition-concentration data, and the 50% effective concentration (IC₅₀) was calculated by the use of SAS software (Statistical Software System; SAS Institute, Inc., Cary, N.C.).

### TABLES OF COMPOUNDS

Compounds of the invention were assayed either in one or both of the assays of Examples 31 and 32 and were found to be active with IC₅₀ below 50µM *(**A**);* below 5µM *(**B**)* or below 0.5µM *(**C**).*

### Activity in cells and specificity:

Representative compounds of the invention were also tested in the surrogate cell-based assay of Example 33, and in one or several assays of Example 34. For example, compound **233** from Table 2 was found to have an IC₅₀ of 1 nM in the assay of Example 32. The EC₅₀ as determined by the assay of Example 33 is 5.4 µM whereas other effects (tTA) were not detectable at concentrations up to 120µM. Compound **233** has also been tested in the MTT assay and its TC₅₀ was determined to be greater than 120µM, indicating that this compound is non toxic at its effective concentration. In the specificity assays of Example 34, the same compound was found to have the following activity: HLE >75µM; PPE >75µM; α-Chym. >75 µM; Cat. B >75µM.

These results indicate that this family of compounds is highly specific for the NS3 protease.

The following tables list representative of the invention. he following abbreviations are used: MS: Mass spectrometric data; Ac: acetyl; Bn: benzyl; Chg: cyclohexylglycine (2-amino-2-cyclohexyl-acetic acid); Dnl: Dansyl; O-Bn: benzyloxy; Pip: pipecolic acid; Tbg: *tert*-butylglycine.

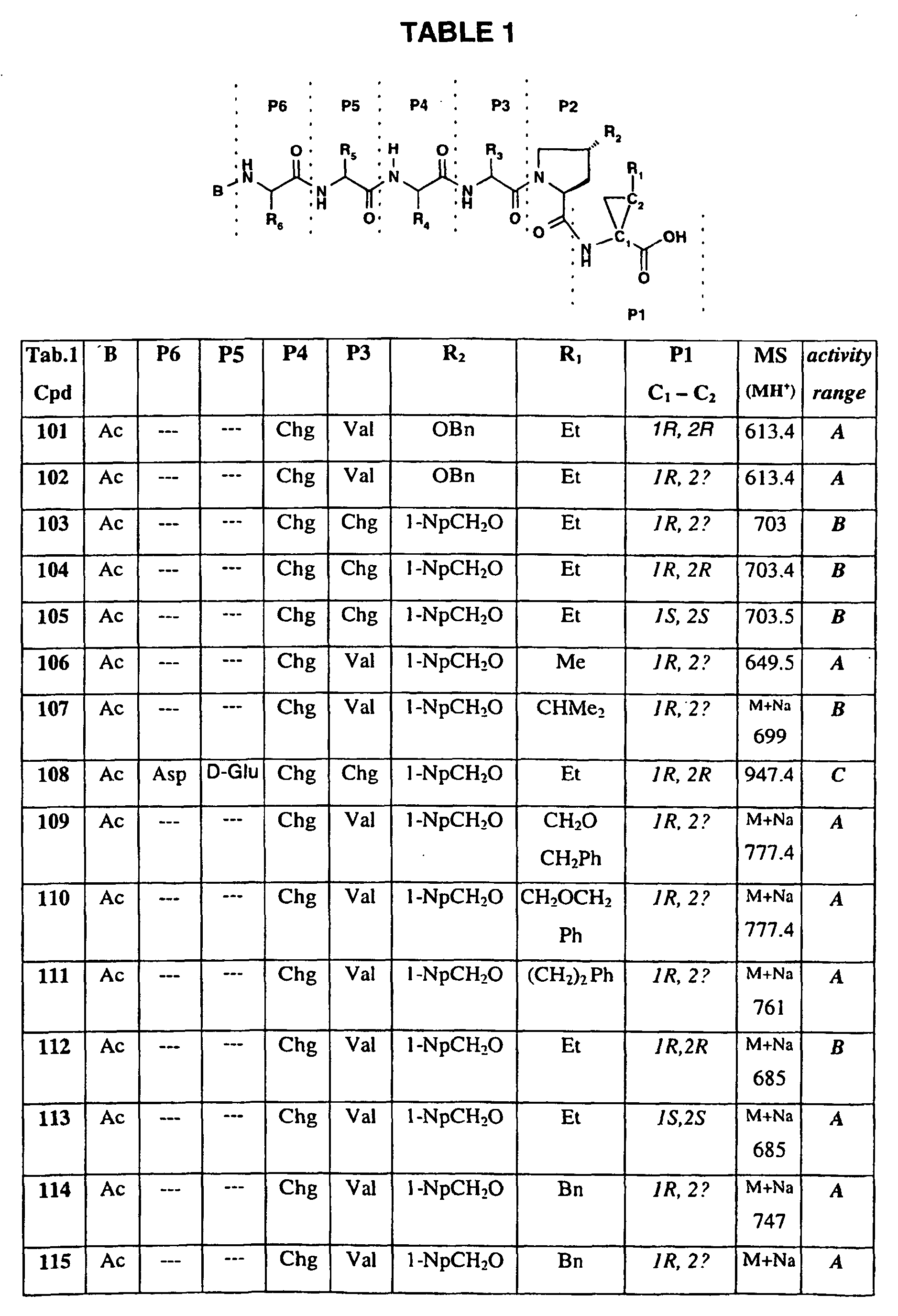

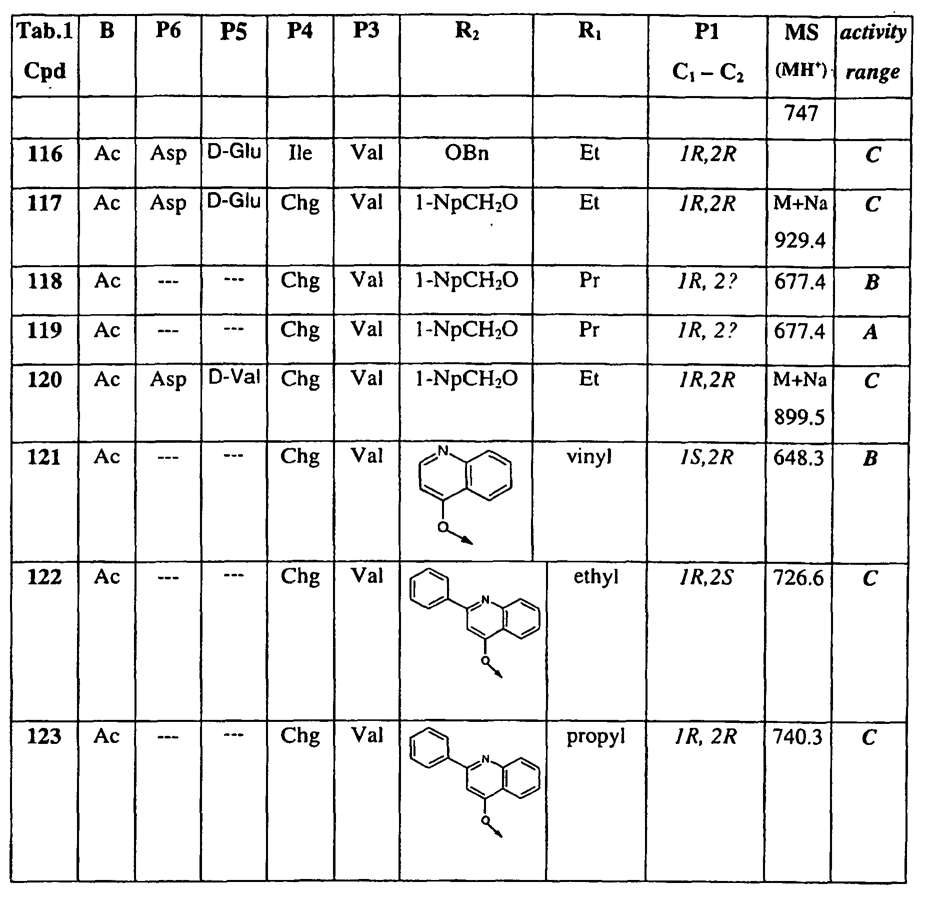

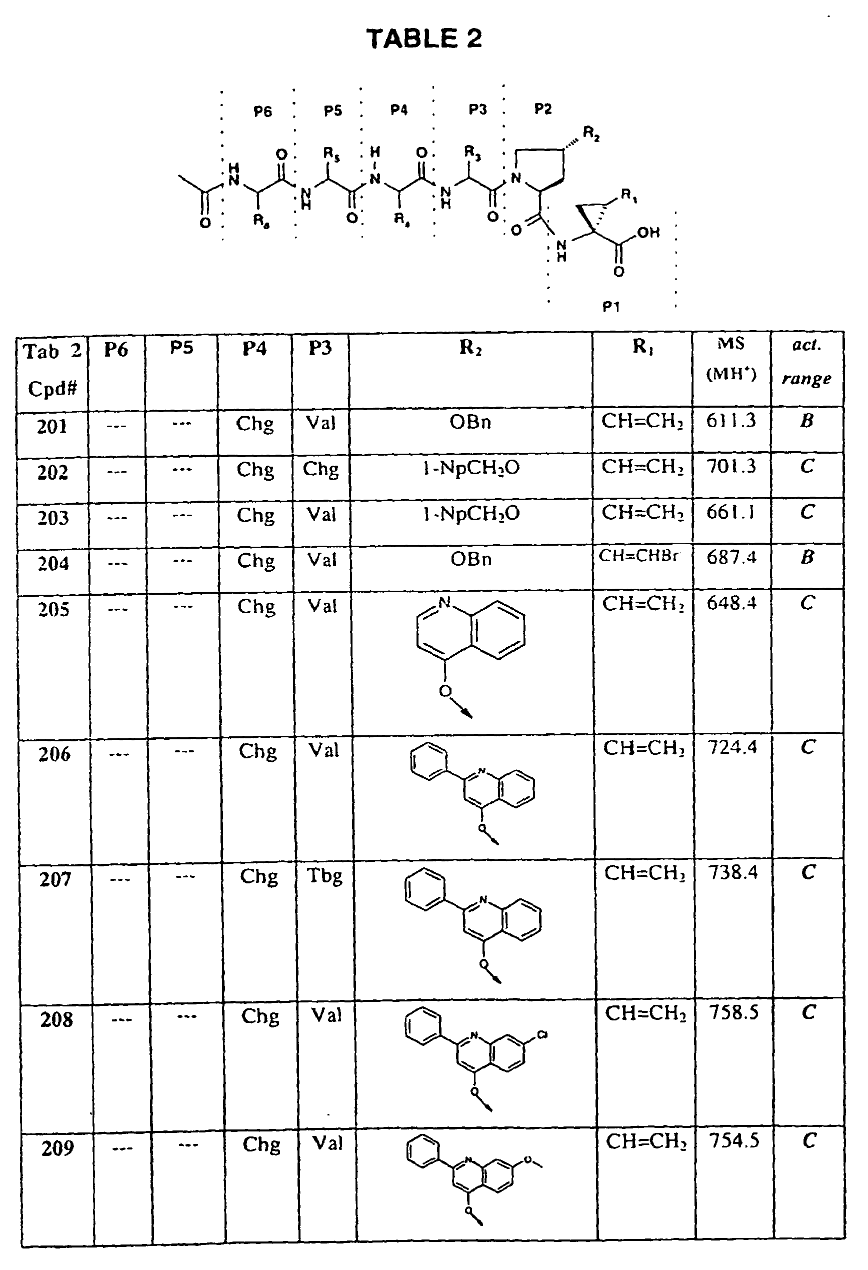

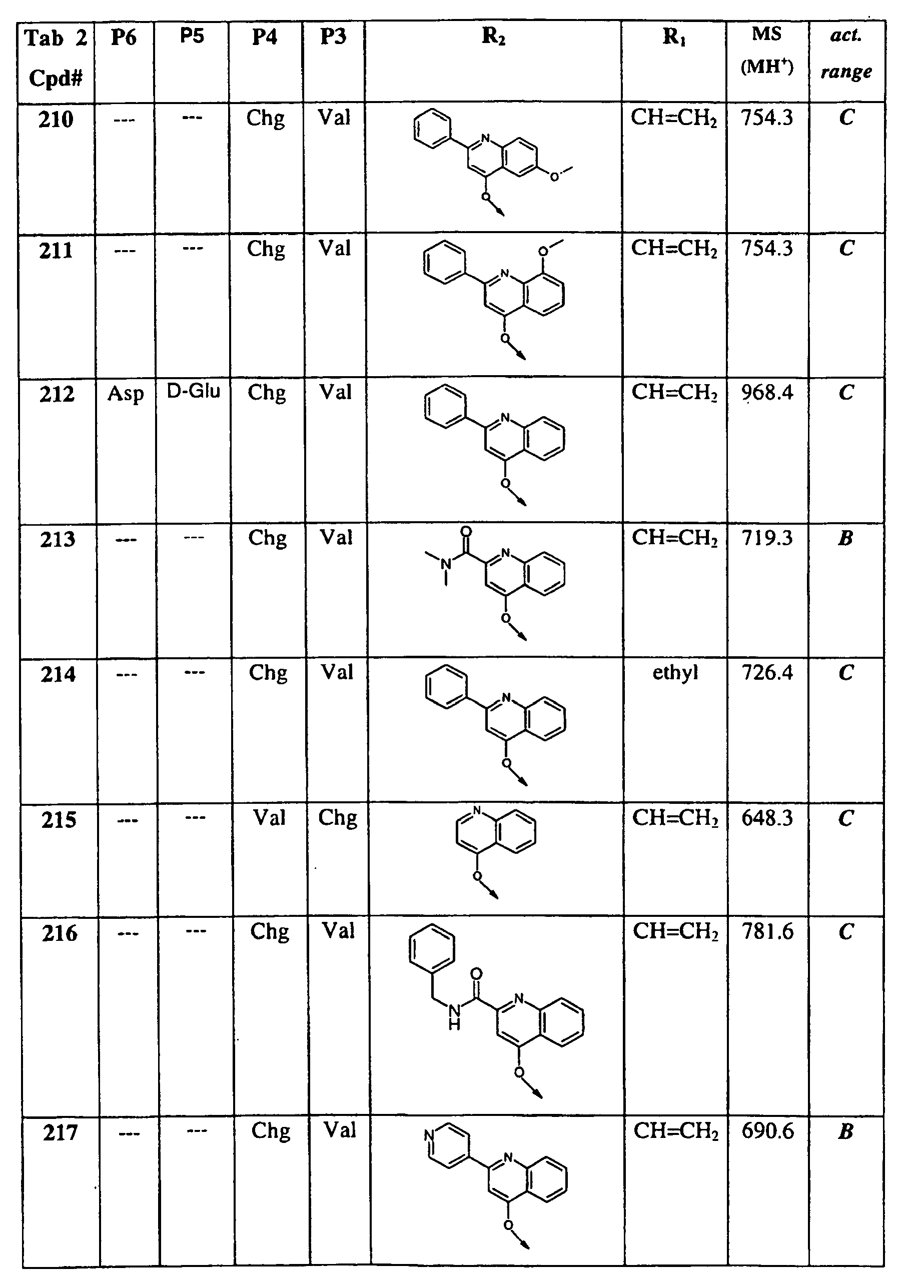

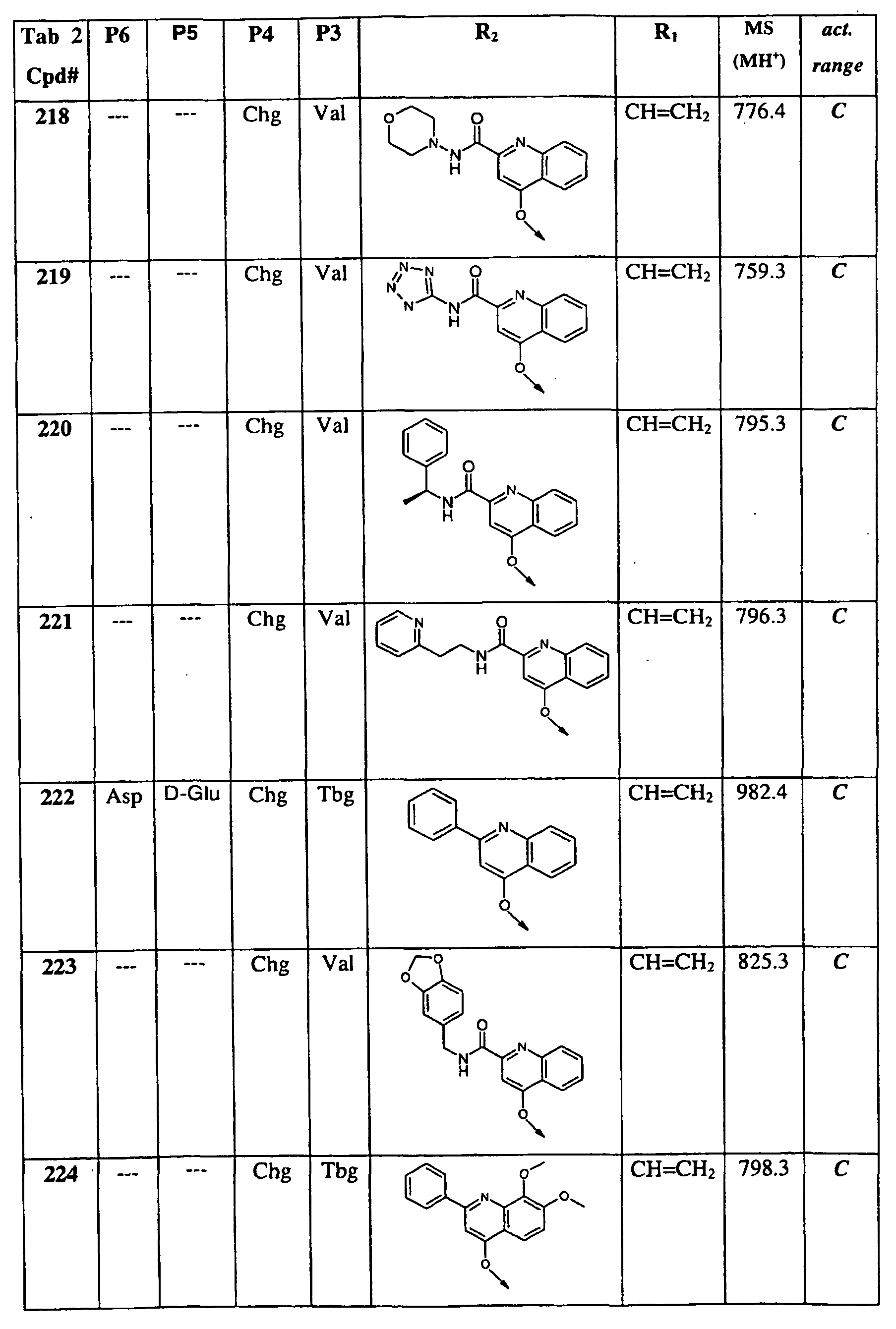

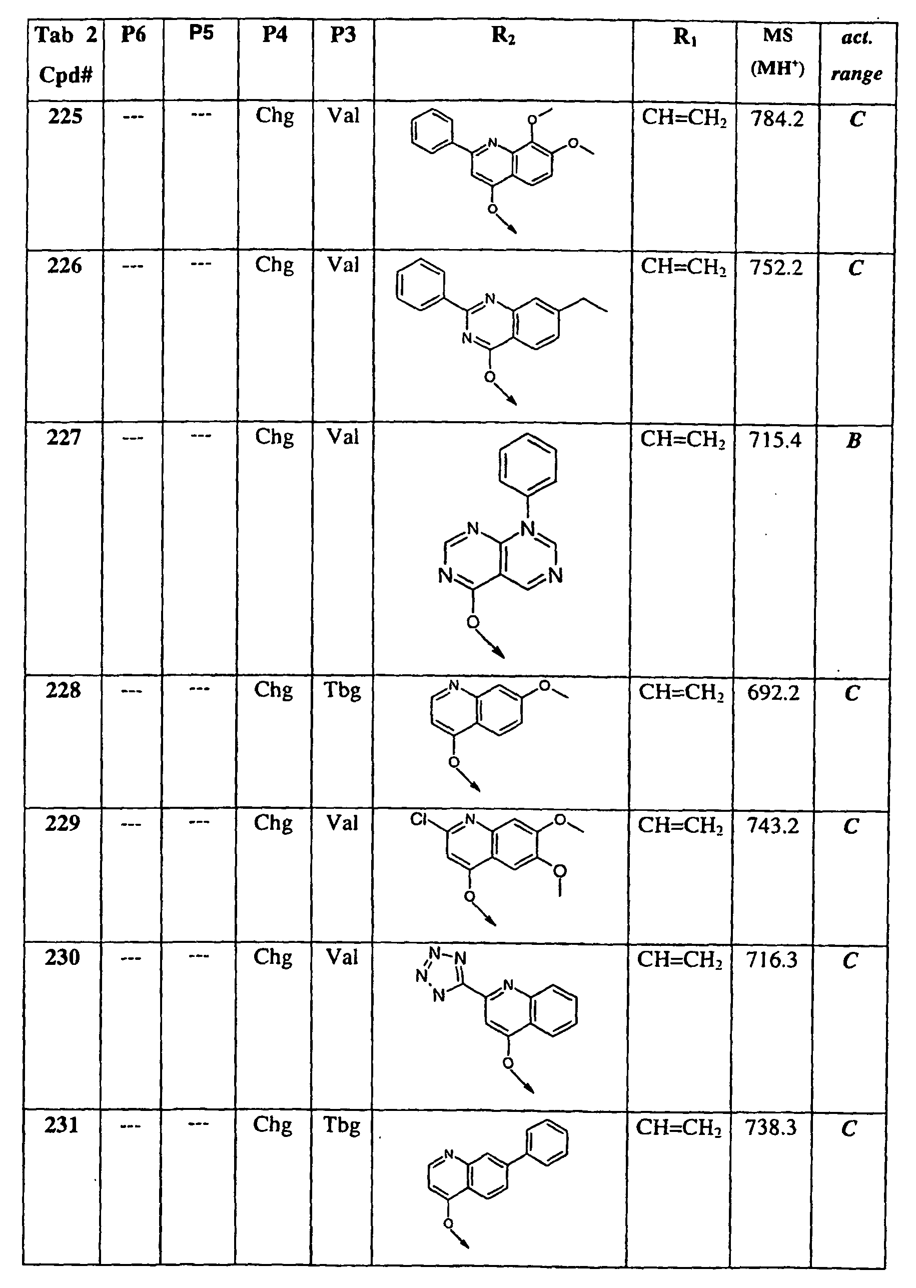

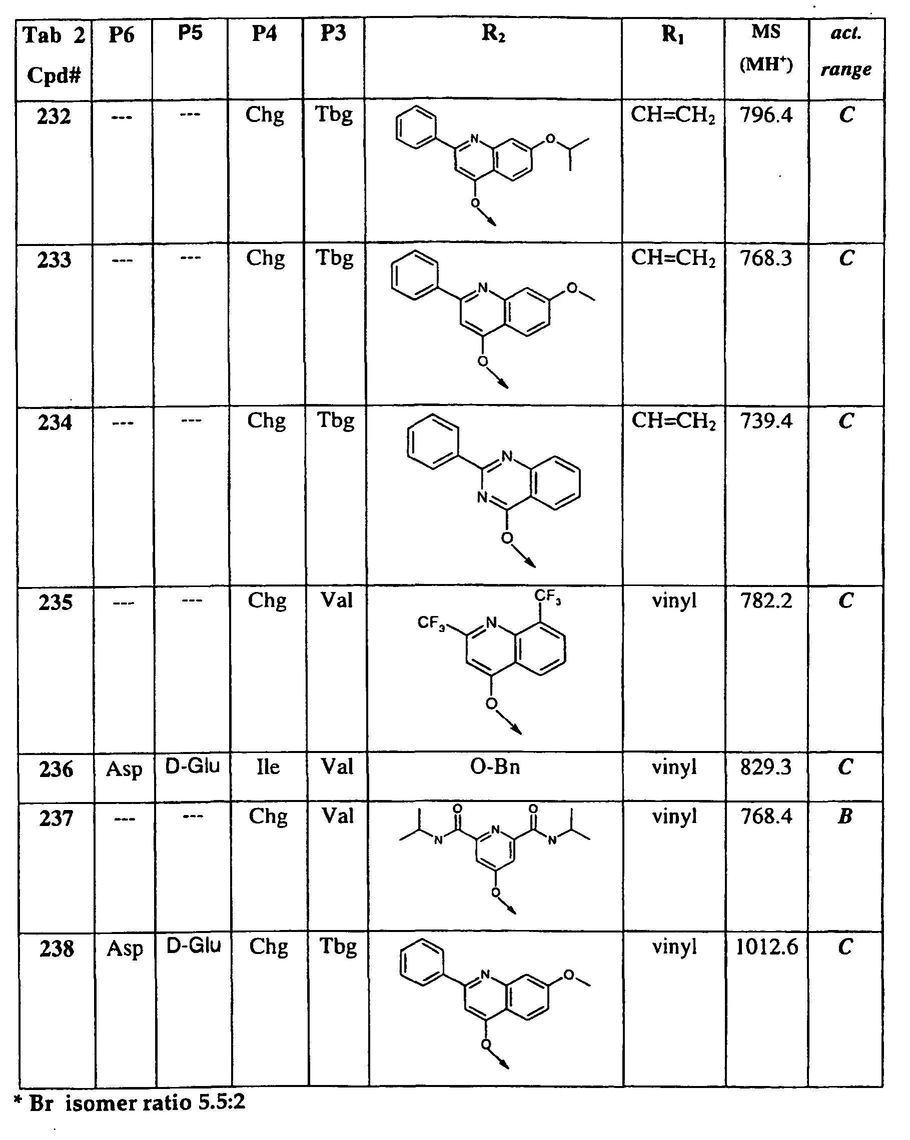

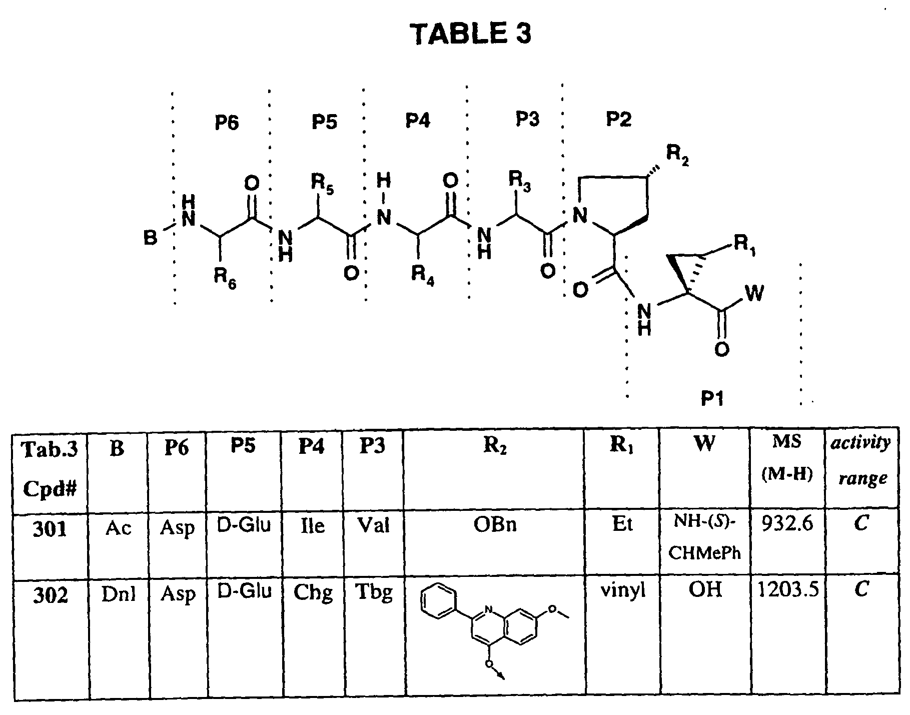

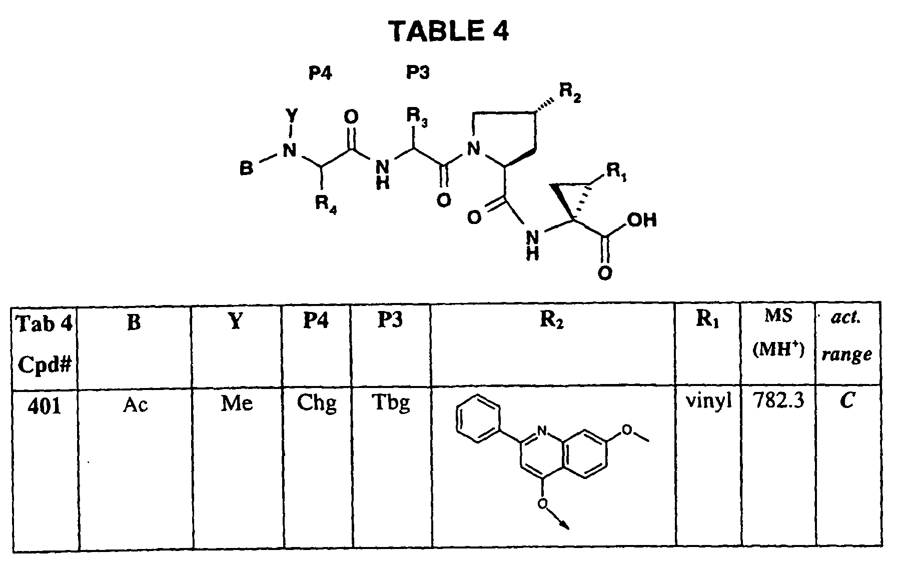

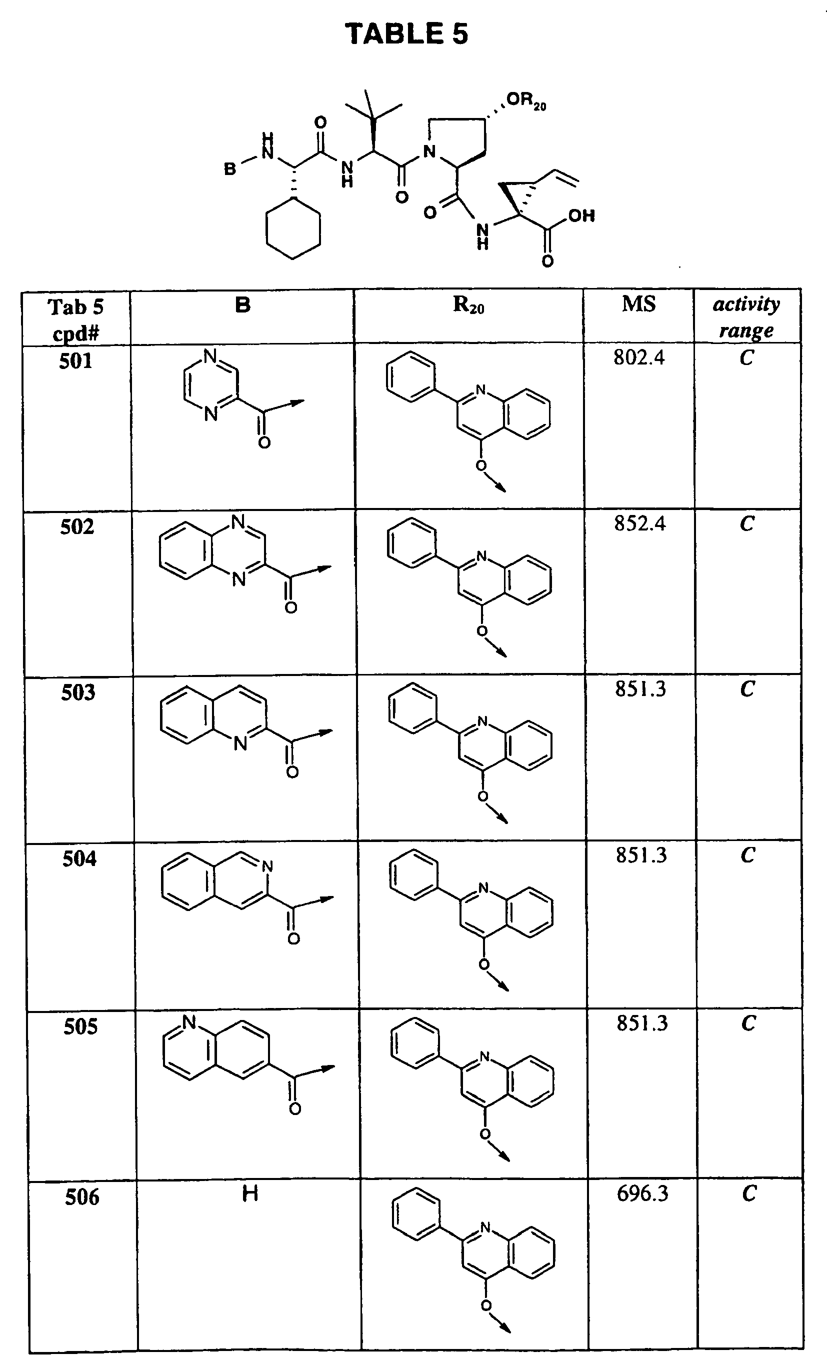

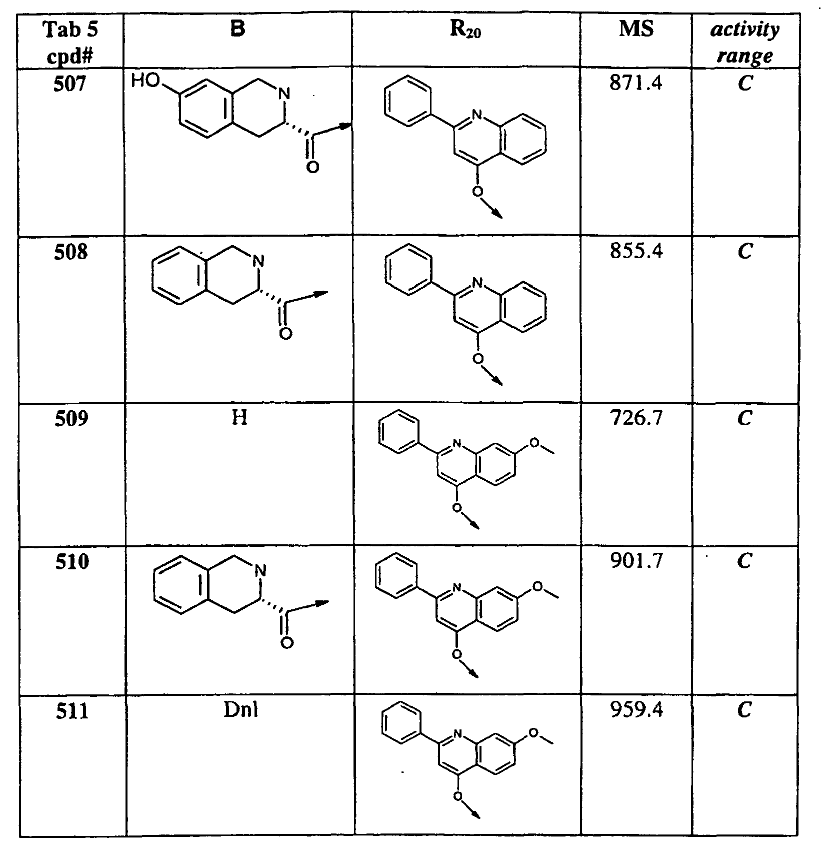

## Claims

1. A compound of formula (I), including racemates, diastereoisomers and optical isomers: 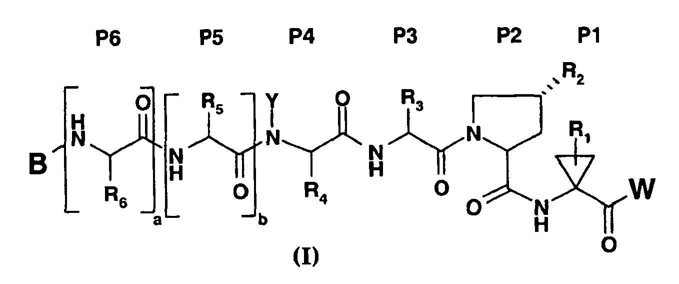
wherein
**a** is 0 or 1; **b** is 0 or 1; **Y** is H or C₁₋₆ alkyl;
**B** is H, an acyl derivative of formula **R**₇-C(O)- or a sulfonyl of formula **R**₇-SO₂ wherein
**R**₇ is (i) C₁₋₁₀ alkyl optionally substituted with carboxyl, C₁₋₆ alkanoyloxy or C₁₋₆ alkoxy;
(ii) C₃₋₇ cycloalkyl optionally substituted with carboxyl, (C₁₋₆ alkoxy)carbonyl or phenylmethoxycarbonyl;
(iii) C₆ or C₁₀ aryl or C₇₋₁₆ aralkyl optionally substituted with C₁₋₆ alkyl, hydroxy, or amino optionally substituted with C₁₋₆ alkyl; or
(iv) Het optionally substituted with C₁₋₆ alkyl, hydroxy, amino optionally substituted with C₁₋₆alkyl, or amido optionally substituted with C₁₋₆ alkyl;
**R**_{**6**} is C₁₋₆ alkyl substituted with carboxyl;
**R**_{**5**} is C₁₋₆ alkyl optionally substituted with carboxyl;
**R**_{**4**} is C₁₋₁₀ alkyl, C₃₋₇ cycloalkyl or C₄₋₁₀ (alkylcycloalkyl);
**R**_{**3**} is C₁₋₁₀ alkyl, C₃₋₇ cycloalkyl or C₄₋₁₀ (alkylcycloalkyl);
**R**_{**2**} is CH₂-**R**_{**20**}, NH-**R**_{**20**}, O-**R**_{**20**} or S-**R**_{**20**}, wherein **R**_{**20**} is a saturated or unsaturated C₃₋₇ cycloalkyl or C₄₋₁₀ (alkyl cycloalkyl) being optionally mono-, di- or tri-substituted with **R**_{**21**}, or **R**_{**20**} is a C₆ or C₁₀ aryl or C₇₋₁₆ aralkyl optionally mono-, di- or tri-substituted with **R**_{**21**}, or **R**_{**20**} is Het or (lower alkyl)-Het optionally mono-, di- or tri-substituted with **R**_{**21**},
wherein each **R**_{**21**} is independently C₁₋₆ alkyl; C₁₋₆alkoxy; amino optionally mono- or di-substituted with C₁₋₆ alkyl; sulfonyl; NO₂; OH; SH; halo; haloalkyl; amido optionally mono-substituted with C₁₋₆ alkyl, C₆ or C₁₀ aryl, C₇₋₁₆ aralkyl, Het or (lower alkyl)-Het; carboxyl; carboxy(lower alkyl); C₆ or C₁₀ aryl, C₇₋₁₆ aralkyl or Het, said aryl, aralkyl or Het being optionally substituted with **R**_{**22**};
wherein **R**_{**22**} is C₁₋₆alkyl; C₁₋₆ alkoxy; amino optionally mono- or di-substituted with C₁₋₆ alkyl; sulfonyl; NO₂; OH; SH; halo; haloalkyl; carboxyl; amide or (lower alkyl)amide;
**R**_{**1**} is C₁₋₆ alkyl or C₂₋₆ alkenyl optionally substituted with halogen; and
**W** is hydroxy or a N-substituted amino;
or a pharmaceutically acceptable salt or ester thereof, with the proviso that the compound of formula (I) is excluded,
wherein
B is acetyl,
a and b are 0,
P4 represents the amino acid cyclohexylglycine (Chg);
P3 represents the amino acid valine (Val);
R₂ represents benzyloxy (O-Bn);
P1 represents a racemic group of formula 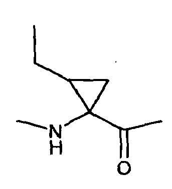
and W represents OH.

2. The compound according to claim 1, wherein **B** is H or an acyl derivative of formula **R**_{**7**}C(O)-, wherein **R**_{**7**} is C₁₋₆ alkyl; C₁₋₆ alkoxy; C₃₋₇ cycloalkyl optionally substituted with hydroxy; amido optionally substituted with C₁₋₆ alkyl or Het; C₆ or C₁₀ aryl, C₇₋₁₆ aralkyl or Het all optionally substituted with C₁₋₆ alkyl or hydroxy.

3. The compound according to claim 2, wherein **R**_{**7**} is C₁₋₆ alkyl or Het.

4. The compound according to claim 3, wherein said Het is selected from the group consisting of:

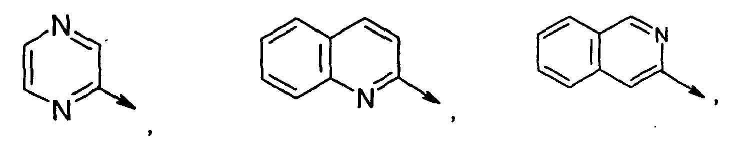

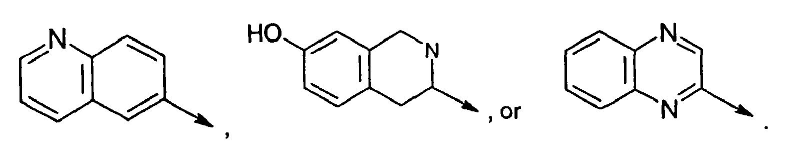

5. The compound according to claim 2, wherein **B** is selected from the group consisting of: H. acetyl;

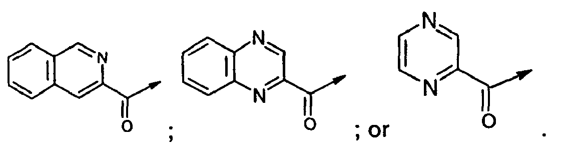

6. The compound according to claim 5, wherein **B** is acetyl.

7. The compound according to claim 1, wherein **B** is **R**_{**7**}-SO₂ and **R**_{**7**} is **C**_{**6**} or C₁₀ aryl, a C₇₋₁₆ aralkyl or Het, all optionally substituted with C₁₋₆ alkyl.

8. The compound according to claim 1, wherein **R**_{**6**} when present, is the side chain of Asp or Glu.

9. The compound according to claim 8, wherein **R**_{**6**}**,** when present, is the side chain of Asp.

10. The compound according to claim 1, wherein a is 0 and then **R**_{**6**} is absent.

11. The compound according to claim 1, wherein **R**_{**5**}**,** when present, is the side chain of an amino acid selected from the group consisting of: D-Asp, L-Asp, D-Glu, L-Glu, D-Val, L-Val, D-tert-butylglycine (Tbg), and L-Tbg.

12. The compound according to claim 11, wherein **R**_{**5**}**,** when present, is the side chain of D-Asp, D-Val, or D-Glu.

13. The compound according to claim 12, wherein **R**_{**5**}**,** when present, is the side chain of D-Glu.

14. The compound according to claim 1, wherein **R**_{**4**} is the side chain of an amino acid selected from the group consisting of: Val, cyclohexylglycine (Chg), Tbg, Ile or Leu.

15. The compound according to claim 14, wherein **R**_{**4**} is the side chain of Chg or Ile.

16. The compound according to claim 15, wherein **R**_{**4**} is the side chain of Chg.

17. The compound according to claim 1, wherein **Y** is H, or Me.

18. The compound according to claim 17, wherein **Y** is H.

19. The compound according to claim 1, wherein **R**_{**3**} is the side chain of an amino acid selected from the group consisting of: Ile, Chg, Val or Tbg.

20. The compound according to claim 19, wherein **R**_{**3**} is the side chain of Val, Chg or Tbg.

21. The compound according to claim 20, wherein **R**_{**3**} is the side chain of Val or Tbg.

22. The compound according to claim 1, wherein **R**_{**2**} is S-**R**₂₀, O-**R**₂₀ wherein **R**_{**20**} is C₆ or C₁₀ aryl, C₇₋₁₆ aralkyl, Het or -CH₂-Het, all optionally mono-, di- or tri-substituted with **R**_{**21**};
wherein **R**_{**21**} is C₁₋₆ alkyl, C₁₋₆ alkoxy; amino, mono- or di-(lower alkyl)amino; amido optionally mono-substituted with C₁₋₆ alkyl, C₆ or C₁₀ aryl, C₇₋₁₆ aralkyl, Het or (lower alkyl)-Het; NO₂; OH; halo; trifluoromethyl; carboxyl; C₆ or C₁₀ aryl, C₇₋₁₆ aralkyl, or Het, said aryl aralkyl or Het being optionally substituted with **R**_{**22**};
wherein **R**_{**22**} is C₁₋₆ alkyl; C₁₋₆ alkoxy; amino; mono- or di-(lower alkyl)amino; (lower alkyl)amide; NO₂; OH; halo; trifluoromethyl; or carboxyl.

23. The compound according to claim 22, wherein **R**_{**21**} is C₁₋₆ alkyl; C₁₋₆ alkoxy; amino; di(lower alkyl)amino; (lower alkyl)amide; C₆ or C₁₀ aryl, or Het, said aryl or Het optionally substituted with **R**_{**22**}**,** wherein **R**_{**22**} is C₁₋₆ alkoxy; amino; di(lower alkyl)amino; (lower alkyl)amide; halo or trifluoromethyl.

24. The compound according to claim 22, wherein **R**_{**2**} is 1-naphthylmethoxy; 2-naphthylmethoxy; benzyloxy, 1-naphthyloxy; 2-naphtyloxy; or quinolinoxy unsubstituted, mono- or di-substituted with **R**_{**21**} wherein **R**_{**21**} is as defined in claim 23.

25. The compound according to claim 22, wherein **R**_{**2**} is 1-naphtylmethoxy; or quinolinoxy unsubstituted, mono- or di-substituted with **R**_{**21**} wherein **R**_{**21**} is as defined in claim 23.

26. The compound according to claim 25, wherein **R**_{**2**} is : 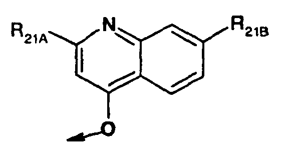
wherein **R**_{**21A**} is amido optionally substituted with C₁₋₆ alkyl, C₆ or C₁₀ aryl, C₇₋₁₆ aralkyl or Het; C₆ or C₁₀ aryl or Het optionally substituted with **R**_{**22**}**,** and **R**_{**22**} is amino, di(lower alkyl)amino; or (lower alkyl)amide; and **R**_{**21B**} is C₁₋₆ alkyl; C₁₋₆alkoxy; amino; di(lower alkyl)amino; (lower alkyl)amide; NO₂; OH; halo; trifluoromethyl; or carboxyl.

27. The compound according to claim 26, wherein **R**_{**21A**} is C₆ or C₁₀ aryl or Het, all optionally substituted with **R**_{**22**} and **R**_{**22**} is amino, dimethylamino, or acetamido.

28. The compound according to claim 26, wherein **R**_{**21B**} is C₁₋₆ alkoxy or di(lower alkyl)amino.

29. The compound according to claim 28, wherein **R**_{**21B**} is methoxy.

30. The compound according to claim 1, wherein the asymmetric carbon at position 1 has the *R* configuration, represented by the following absolute configurations: 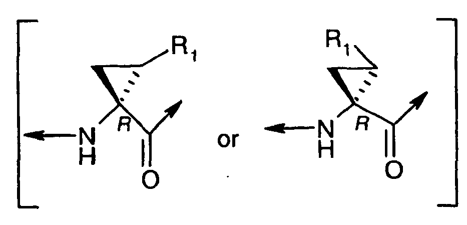
wherein **R**_{**1**} is as defined in claim 1.

31. The compound according to claim 30, wherein the **R**_{**1**} substituent on P1 is oriented *syn* to the carbonyl group as represented by the following absolute configuration: 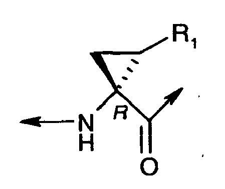
wherein **R**_{**1**} is methyl, ethyl, propyl, vinyl, all of which optionally substituted with halo.

32. The compound according to claim 31, wherein **R**_{**1**} is ethyl, vinyl or bromovinyl.

33. The compound according to claim 32, wherein **R**_{**1**} is vinyl.

34. The compound according to claim 1, wherein **W** is hydroxy or a pharmaceutically acceptable salt or ester thereof; or (lower alkyl)amino, di(lower alkyl)amino or amino aralkyl.

35. The compound according to claim 32, wherein **W** is hydroxy, or N(**R**₁₃ₐ)**R**_{13b} wherein **R**_{**13a**} and **R**_{**13b**} are independently H, aryl or C₁₋₆ alkyl optionally substituted with hydroxy or phenyl; or a pharmaceutically acceptable salt thereof.

36. The compound according to claim 35, wherein **W** is -OH, -NH-benzyl or -NH-CH(Me)Ph.

37. The compound according to claim 36, wherein **W** is -OH or -NH-(*S*)CH(Me)-phenyl.

38. The compound according to claim 37, wherein when **W** is an ester, said ester is selected from the group consisting of: C₁₋₆ alkoxy, phenoxy, or aryl(C₁₋₆ alkoxy).

39. The compound according to claim 38, wherein said ester is methoxy, ethoxy, phenoxy, benzyloxy, or PhCH(Me)-O-.

40. The compound of formula I according to claim 1, wherein **B** is H, lower alkyl-C(O)- or Het-C(O)-;
**R**_{**6**}**,** when present, is the side chain of Asp or Glu;
**R**_{**5**}**,** when present, is the side chain of D- or L-: Asp, Glu, Val, or Tbg;
**Y** is H or methyl;
**R**_{**4**} is the side chain of Val, Chg, Tbg, Ile or Leu;
**R**_{**3**} is hydrogen or the side chain of Ile, Chg, Val, or Tbg;
**R**_{**2**} is 1-naphthylmethoxy, 2-naphthylmethoxy, O-Bn, 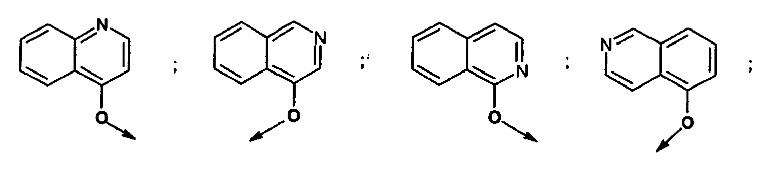 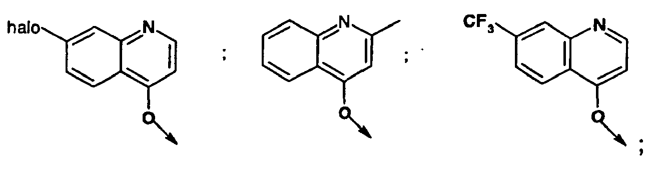 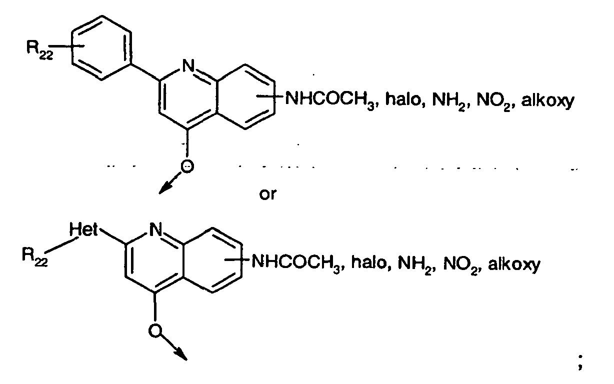
wherein **R**_{**22**} is amino, di(lower alkyl)amino, (lower alkyl)amide, NO₂, OH, halo, CF₃, or COOH;
P1 is a cyclopropyl ring system of formula 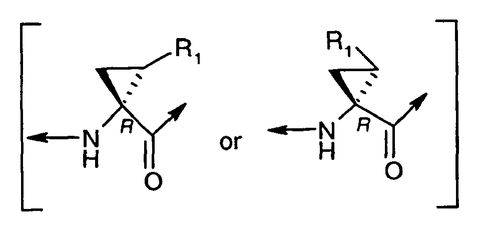
wherein **R**_{**1**} is ethyl, vinyl or bromovinyl; and
**W** is hydroxy or **N(R**_{**13a**}**)R**_{**13b**} wherein **R**_{**13a**} and **R**_{**13b**} are independently H, aryl or C₁₋₆ alkyl optionally substituted with hydroxy or phenyl; or a pharmaceutically acceptable salt or ester thereof.

41. The compound of formula I according to claim 1, wherein **B** is H, acetyl or Het-C(O)-; **R**_{**6**}**,** when present, is the side chain of Asp; **R**_{**5**}**,** when present, is the side chain of D-Asp, D-Glu or D-Val; Y is H; **R**_{**4**} is the side chain of Chg or Ile; **R**_{**3**} is the side chain of Val, Chg or Tbg; **R**_{**2**} is 1-naphthylmethoxy, benzyloxy, 4-quinolinoxy, or 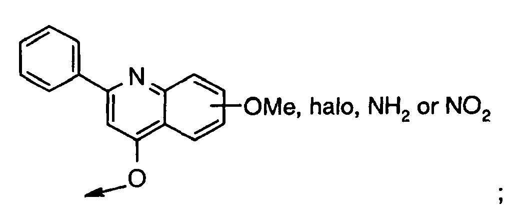
**P1** is a cyclopropyl ring system of formula 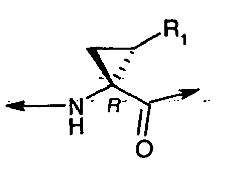
wherein **R**_{**1**} is Et or CH=CH₂ or CH=CHBr; and
**W** is hydroxy or NH-(*S*)-CHMePh, or a pharmaceutically acceptable salt thereof.

42. The compound of formula I according to claim 1, wherein **B** is acetyl; **R**_{**6**}, when present, is the side chain of Asp; **R**_{**5**}, when present, is the side chain of D-Glu; **Y** is H; **R**_{**4**} is the side chain of Chg; **R**_{**3**} is the side chain of Val or Tbg; **R**_{**2**} is: 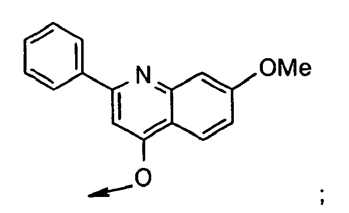
**P1** is: 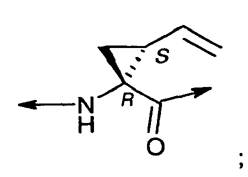
and
**W** is hydroxy, or a pharmaceutically acceptable salt or ester thereof.

43. The compound according to claim 40 represented by formula: 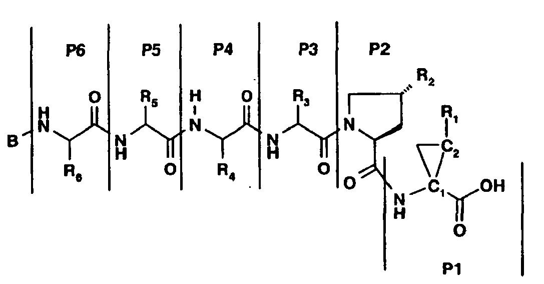
wherein **B, P6, P5, P4, P3, R**_{**2**}**,** and **R**_{**1**} are as defined below:

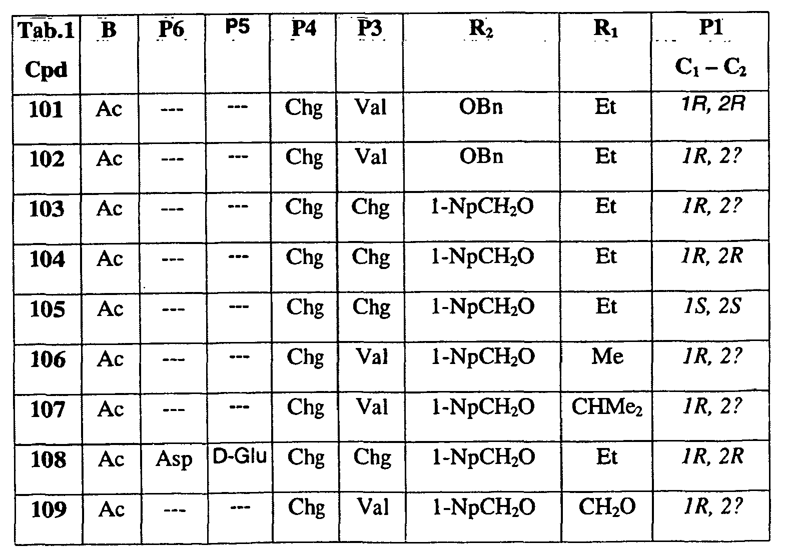

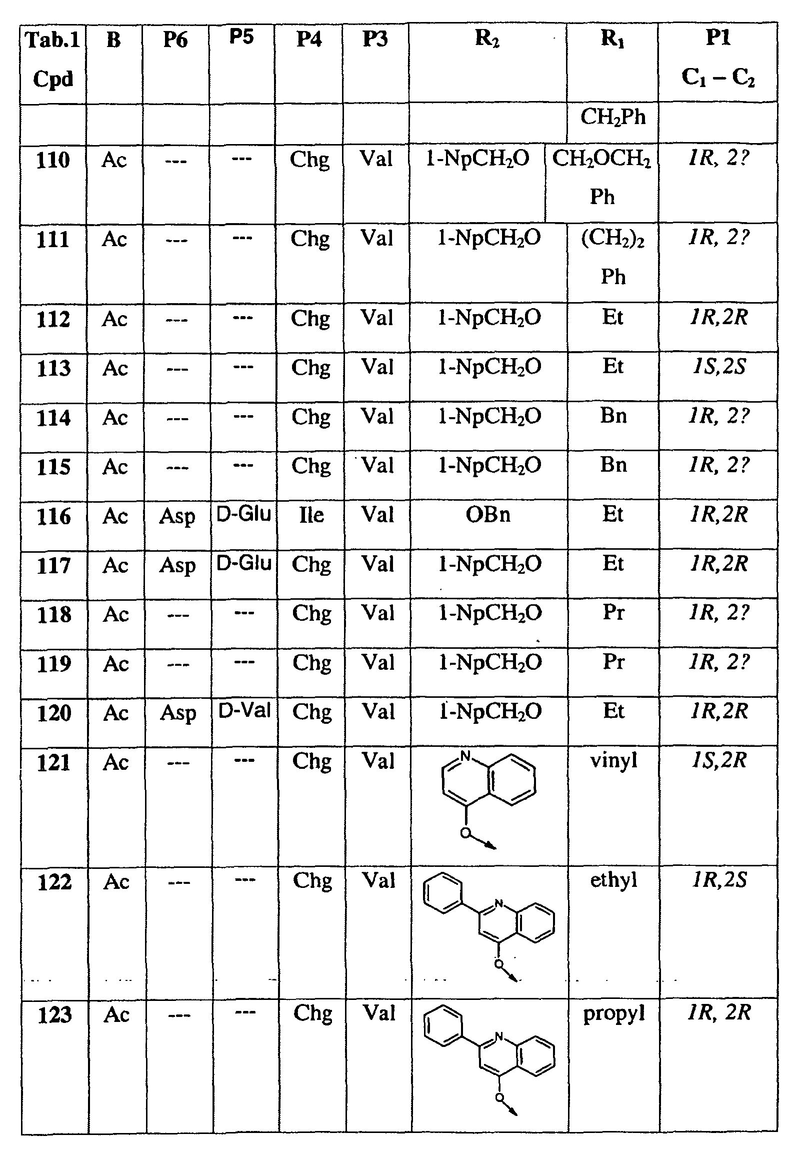

44. The compound according to claim 40 represented by formula: 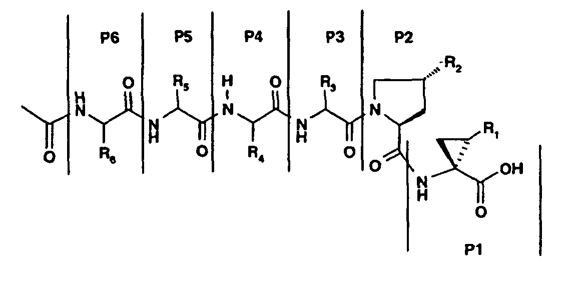
wherein **P6, P5, P4, P3, R**_{**2**}**,** and **R**_{**1**} are as defined below:

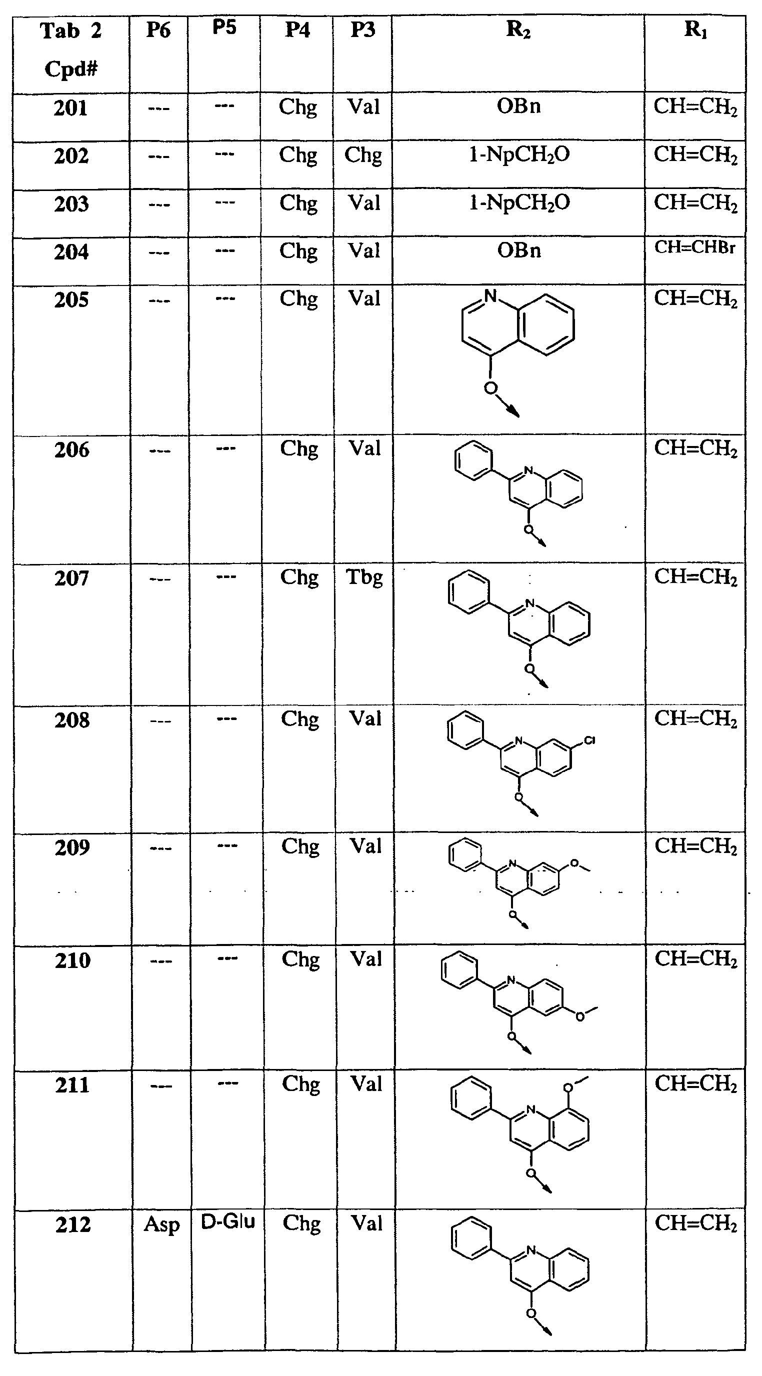

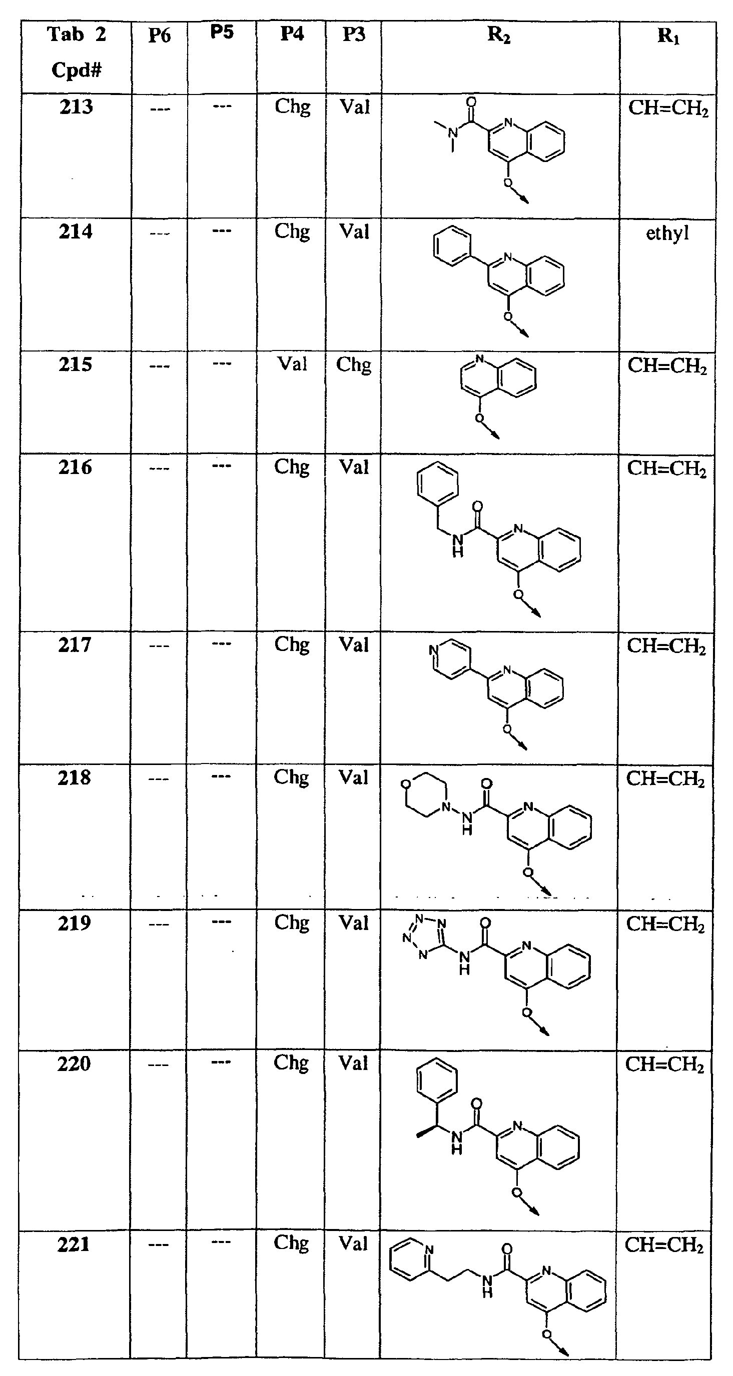

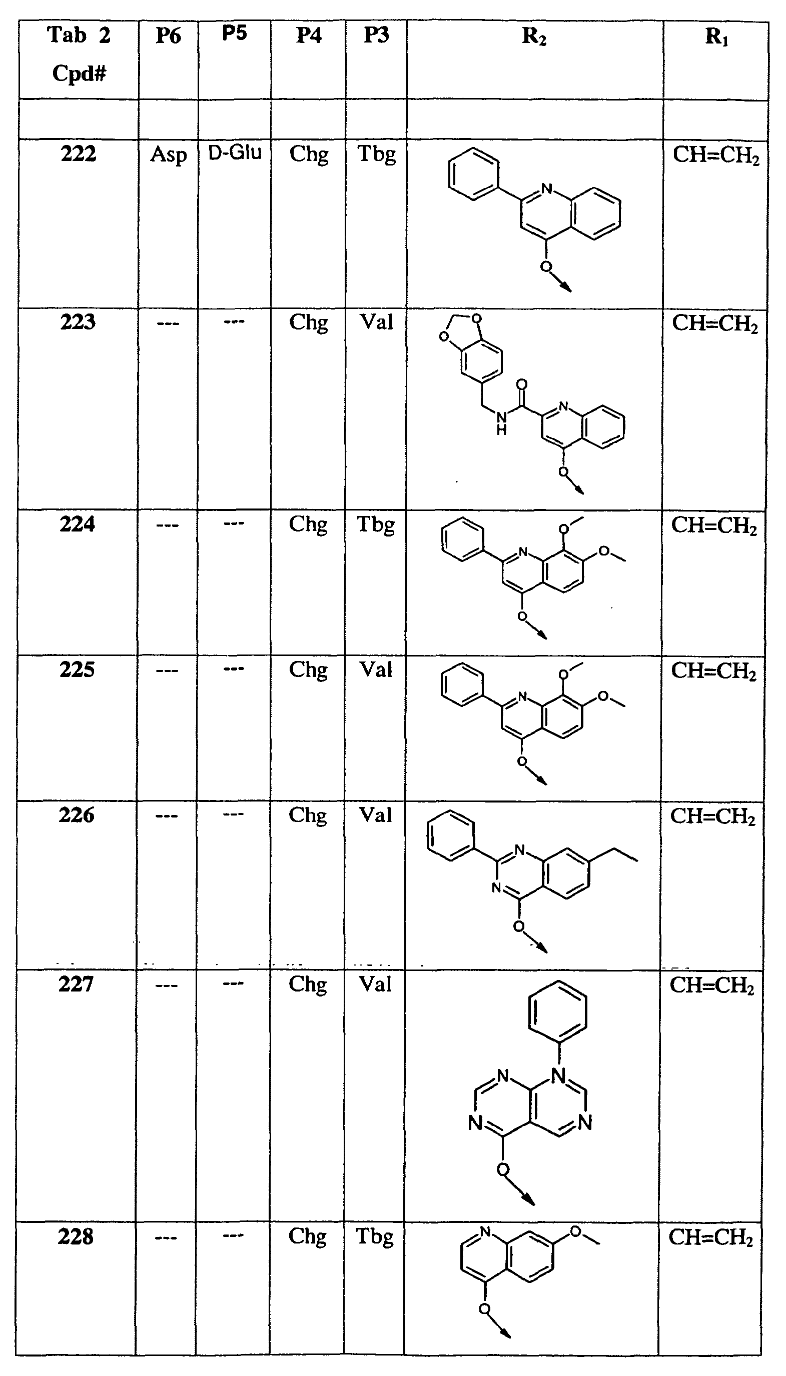

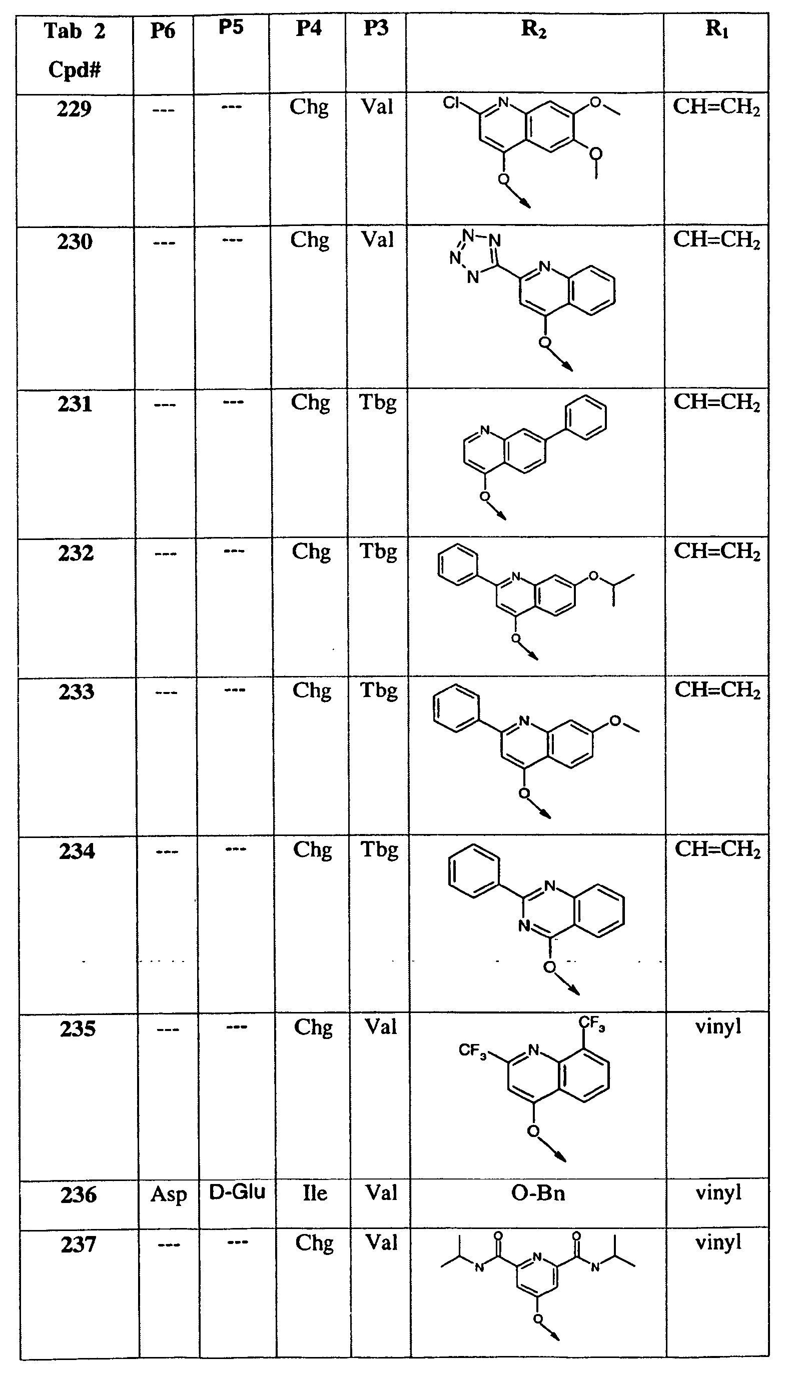

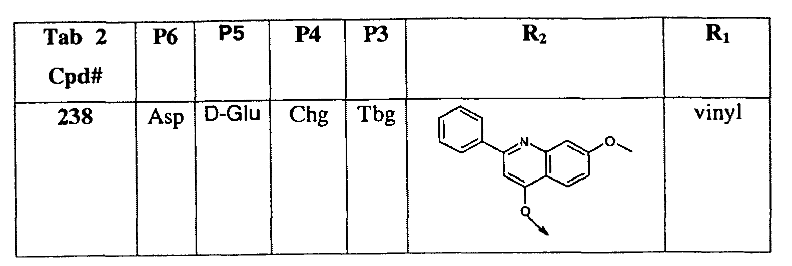

45. The compound according to claim 40 represented by formula: 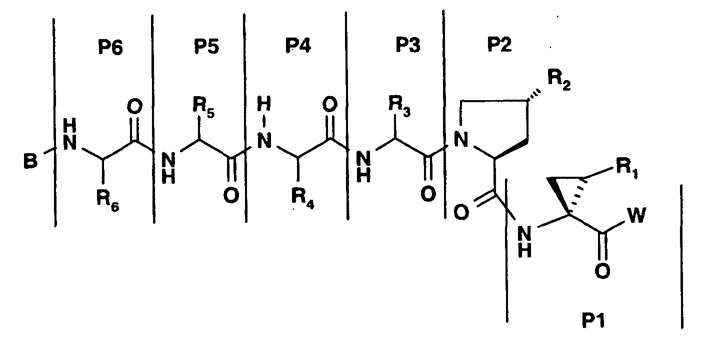
wherein **B, P6, P5, P4, P3, R**_{**2**}**, R**_{**1**} and **W** are as defined below:

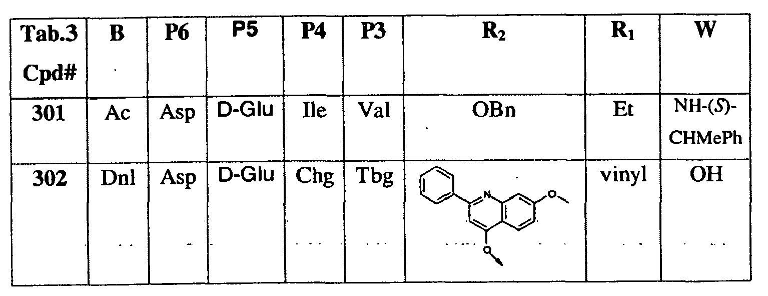

46. The compound according to claim 40 represented by formula: 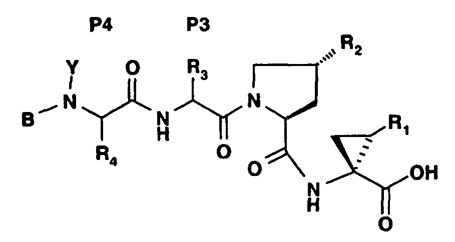
wherein **B, Y, P4, P3, R**_{**2**}**,** and **R**_{**1**} are as defined below:

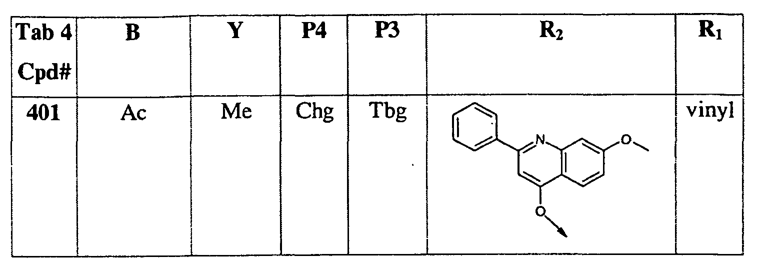

47. The compound according to claim 40 represented by formula: 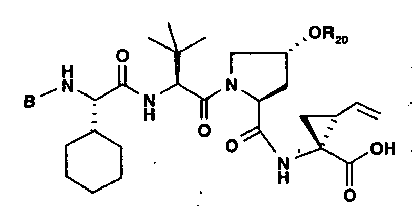
wherein **B**, and **R**_{**20**}, are as defined below:

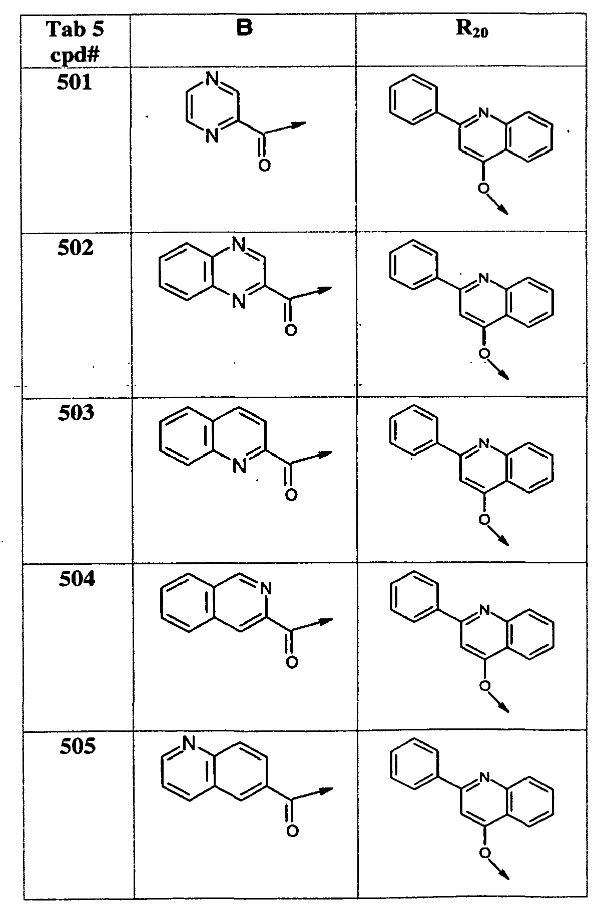

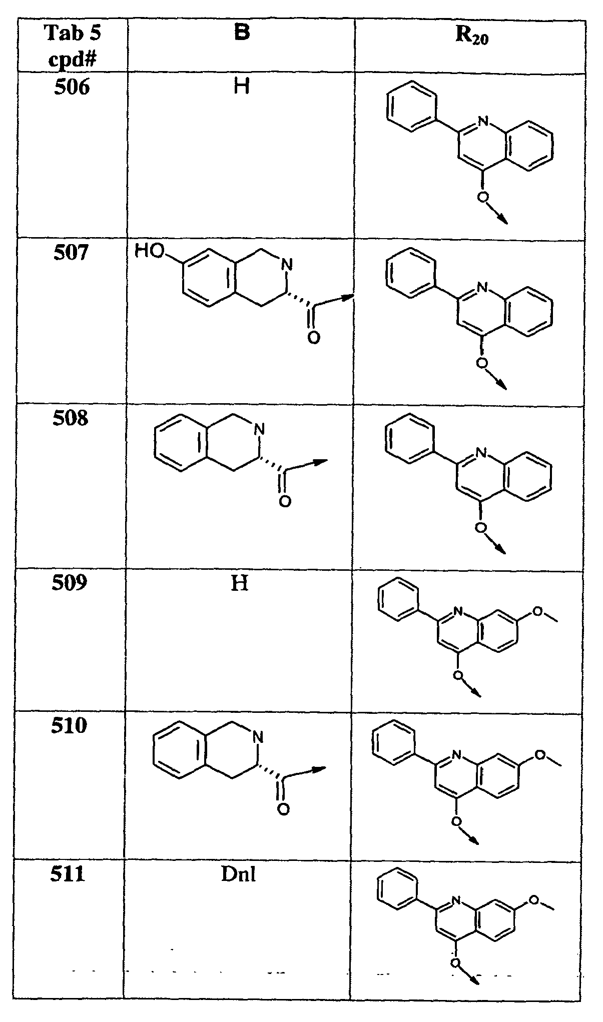

48. A hexapeptide of formula I according to claim 43, selected from the group consisting of compound #:108; 116; 117; and 120.

49. A hexapeptide of formula I according to claim 44, selected from the group consisting of compound #: 212; 222; 236; and 238.

50. A hexapeptide of formula I according to claim 45, selected from the group consisting of compound #: 301 and 302.

51. A tetrapeptide of formula I according to claim 43 selected from the group consisting of compound #: 122; and 123.

52. A tetrapeptide of formula I according to claim, 44 selected from the group consisting of compound #: 202; 203; 205; 206; 207; 208; 209; 210; 211; 214; 215; 216; 218; 219; 220; 221; 223; 224; 225; 226; 228; 229; 230; 231; 232; 233; 234; 235.

53. A tetrapeptide of formula I according to claim, 46 selected from the group consisting of compound #: 401.

54. A tetrapeptide of formula I according to claim 47, selected from the group consisting of compound #: 501; 502; 503; 504; 505; 506; 507; 508; 509; 510; and 511.

55. A compound according to any one of claims 1 to 54 as medicament.

56. A pharmaceutical composition comprising an anti-hepatitis C virally effective amount of a compound of formula I according to claim 1, or a therapeutically acceptable salt or ester thereof, in admixture with a pharmaceutically acceptable carrier medium or auxiliary agent.

57. The use of a compound of formula I according to claim 1, or a therapeutically acceptable salt or ester thereof for the preparation of a medicament for the treatment of a hepatitis C viral infection.

58. An *in vitro* method of inhibiting the replication of hepatitis C virus by exposing the virus to a hepatitis C viral NS3 protease inhibiting amount of the compound of formula I according to claim 1, or a therapeutically acceptable salt or ester thereof or a composition according to claim 56.

59. The use of a combination of the compound of formula I according to claim 1, or a therapeutically acceptable salt or ester thereof, and an interferon for the preparation of a medicament for the treatment of a hepatitis C viral infection..

60. The pharmaceutical composition according to claim 56, further comprising a second antiviral agent.

61. The pharmaceutical composition according to claim 60, wherein said second antiviral agent is ribavirin or amantadine.

62. The pharmaceutical composition according to claim 56, further comprising other inhibitors of HCV protease.

63. The pharmaceutical composition according to claim 56, further comprising an inhibitor of other targets in the HCV life cycle, selected from: helicase, polymerase, metalloprotease or IRES.

64. A process for the preparation of a peptide analog of formula (I) according to claim 1, wherein P1 is a substituted aminocyclopropyl carboxylic acid residue, comprising the step of:
■ coupling a peptide selected from the group consisting of: APG-P6-P5-P4-P3-P2; APG-P5-P4-P3-P2; APG-P4-P3-P2; APG-P3-P2; and APG-P2;
■ with a P1 intermediate of formula: 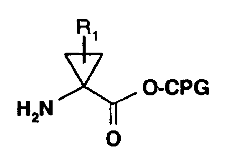
wherein **R**_{**1**} is C₁₋₆ alkyl or C₂₋₆ alkenyl optionally substituted with halogen, CPG is a carboxyl protecting group and P6 to P2 are as defined in claim 1.

65. A process for the preparation of a peptide analog of formula (I) according to claim 1, wherein P1 is a substituted aminocyclopropyl carboxylic acid residue, comprising the step of:
■ coupling a peptide selected from the group consisting of: APG-P6-P5-P4-P3-P2; APG-P5-P4-P3-P2; APG-P4-P3-P2; APG-P3-P2; and APG-P2;
■ with a P1 intermediate of formula: 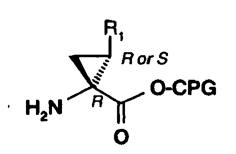
wherein **R**_{**1**} is ethyl, vinyl or bromovinyl, CPG is a carboxyl protecting group and P6 to P2 are as defined in claim 1.

66. A process for the preparation of a peptide analog of formula (I) according to claim 1, wherein P1 is a substituted aminocyclopropyl carboxylic acid residue, comprising the step of:
■ coupling a peptide selected from the group consisting of: APG-P6-P5-P4-P3-P2; APG-P5-P4-P3-P2; APG-P4-P3-P2; APG-P3-P2; and APG-P2;
■ with a P1 intermediate of formula: 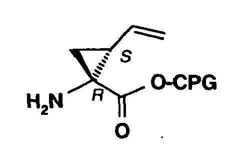
wherein CPG is a carboxyl protecting group and P6 to P2 are as defined in claim 1.

67. The process according to claim 64, 65 or 66 wherein said carboxyl protecting group (CPG) is selected from the group consisting of:
alkyl esters, aralkyl esters, and esters being cleavable by mild base treatment or mild reductive means.

68. Use of an amino acid analog of formula: 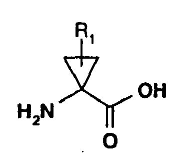
wherein **R**_{**1**} is C₁₋₆ alkyl or C₂₋₆ alkenyl optionally substituted with halogen, for the preparation of a compound of formula I according to claim 1.

69. Use of an amino acid analog of formula: 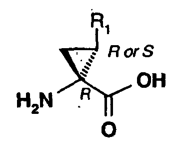
wherein **R**_{**1**} is ethyl, vinyl or bromovinyl, for the preparation of a compound of formula I according to claim 1.

70. Use of an amino acid analog of formula: 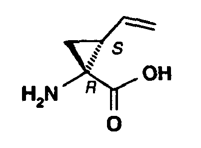
for the preparation of a compound of formula I according to claim 1.

## Patentansprüche

1. Verbindung der Formel (I), einschließlich Racemate, Diastereoisomere und optische Isomere: 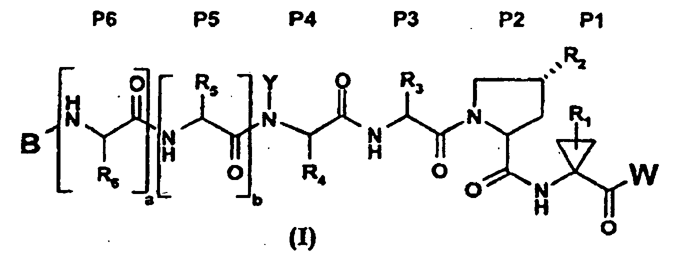
wobei
a 0 oder 1 ist; b 0 oder 1 ist; Y H oder C₁₋₆-Alkyl bedeutet;
B H, ein Acylderivat der Formel R₇-C(O)- oder ein Sulfonyl der Formel R₇-SO₂ bedeutet, wobei
R₇ folgende Bedeutungen hat:
(i) C₁₋₁₀-Alkyl, gegebenenfalls substituiert mit Carboxyl, C₁₋₆-Alkanoyloxy oder C₁₋₆-Alkoxy;
(ii) C₃₋₇-Cycloalkyl, gegebenenfalls substituiert mit Carboxyl, (C₁₋₆-Alkoxy)-carbonyl oder Phenylmethoxycarbonyl;
(iii) C₆- oder C₁₀-Aryl oder C₇₋₁₆-Aralkyl, gegebenenfalls substituiert mit C₁₋₆₋Alkyl, Hydroxy oder Amino, das gegebenenfalls mit C₁₋₆-Alkyl substituiert ist; oder
(iv) Het, gegebenenfalls substituiert mit C₁₋₆-Alkyl, Hydroxy, Amino, das gegebenenfalls mit C₁₋₆-Alkyl substituiert ist, oder Amido, das gegebenenfalls mit C₁₋₆-Alkyl substituiert ist;
R₆ C₁₋₆-Alkyl, das mit Carboxyl substituiert ist, bedeutet;
R₅ C₁₋₆-Alkyl, das gegebenenfalls mit Carboxyl substituiert ist, bedeutet;
R₄ C₁₋₁₀-Alkyl, C₃₋₇-Cycloalkyl oder C₄₋₁₀-(Alkylcycloalkyl) bedeutet;
R₃ C₁₋₁₀-Alkyl, C₃₋₇-Cycloalkyl oder C₄₋₁₀-(Alkylcycloalkyl) bedeutet;
R₂ CH₂-R₂₀, NH-R₂₀, O-R₂₀ oder S-R₂₀ bedeutet, wobei R₂₀ gesättigtes oder ungesättigtes C₃₋₇-Cycloalkyl oder C₄₋₁₀-(Alkylcycloalkyl), die gegebenenfalls mit R₂₁ mono-, di- oder trisubstituiert sind, bedeutet oder R₂₀ C₆- oder C₁₀-Aryl oder C₇₋₁₆-Aralkyl, die gegebenenfalls mit R₂₁ mono-, di- oder trisubstituiert sind, bedeutet, oder R₂₀ Het oder (Niederalkyl)-Het, die gegebenenfalls mit R₂₁ mono-, di- oder trisubstituiert sind, bedeutet,
wobei jedes R₂₁ unabhängig voneinander C₁₋₆-Alkyl; C₁₋₆-Alkoxy; Amino, das gegebenenfalls mit C₁₋₆-Alkyl mono- oder disubstituiert ist; Sulfonyl; NO₂; OH; SH; Halogen; Halogenalkyl; Amido, das gegebenenfalls mit C₁₋₆-Alkyl, C₆- oder C₁₀₋Aryl, C₇₋₁₆-Aralkyl, Het oder (Niederalkyl)-Het monosubstituiert ist; Carboxyl; Carboxy-(niederalkyl); C₆- oder C₁₀-Aryl, C₇₋₁₆-Aralkyl oder Het, wobei das Aryl, Aralkyl oder Het gegebenenfalls mit R₂₂ substituiert ist;
wobei R₂₂ C₁₋₆-Alkyl; C₁₋₆-Alkoxy; Amino, das gegebenenfalls mit C₁₋₆-Alkyl mono- oder disubstituiert ist; Sulfonyl; NO₂; OH; SH; Halogen; Halogenalkyl; Carboxyl; Amid oder (Niederalkyl)-amid bedeutet;
R₁ C₁₋₆-Alkyl oder C₂₋₆-Alkenyl, die gegebenenfalls mit Halogen substituiert sind, bedeutet; und
W Hydroxy oder N-substituiertes Amino bedeutet;
oder ein pharmazeutisch verträgliches Salz oder Ester davon, mit der Maßgabe, dass die Verbindung der Formel (I) ausgenommen ist, in der
B Acetyl bedeutet,
a und b 0 sind,
P4 die Aminosäure Cyclohexylglycin (Chg) bedeutet;
P3 die Aminosäure Valin (Val) bedeutet;
R₂ Benzyloxy (O-Bn) bedeutet;
P₁ eine racemische Gruppe der Formel 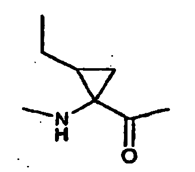
bedeutet
und W OH bedeutet.

2. Verbindung nach Anspruch 1, wobei B H oder ein Acylderivat der Formel R₇C(O)- bedeutet, wobei R₇ C₁₋₆-Alkyl; C₁₋₆-Alkoxy; C₃₋₇-Cycloalkyl, das gegebenenfalls mit Hydroxy substituiert ist; Amido, das gegebenenfalls mit C₁₋₆₋Alkyl oder Het substituiert ist; C₆- oder C₁₀-Aryl, C₇₋₁₆-Aralkyl- oder Het, die alle gegebenenfalls mit C₁₋₆-Alkyl oder Hydroxy substituiert sind, bedeutet.

3. Verbindung nach Anspruch 2, wobei R₇ C₁₋₆-Alkyl oder Het bedeutet.

4. Verbindung nach Anspruch 3, wobei Het aus der folgenden Gruppe ausgewählt ist:

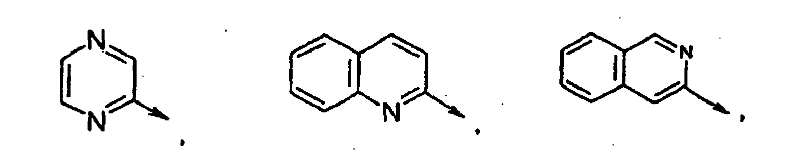

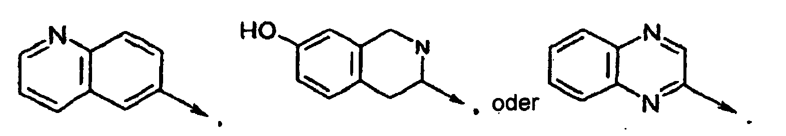

5. Verbindung nach Anspruch 2, wobei B aus der folgenden Gruppe ausgewählt ist: H; Acetyl;

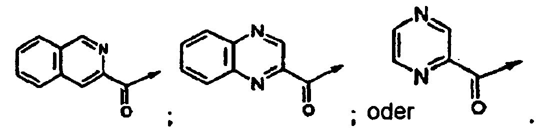

6. Verbindung nach Anspruch 5, wobei B Acetyl bedeutet.

7. Verbindung nach Anspruch 1, wobei B R₇-SO₂ bedeutet und R₇ C₆- oder C₁₀-Aryl, C₇₋₁₆-Aralkyl oder Het bedeutet, die alle gegebenenfalls mit C₁₋₆-Alkyl substituiert sind.

8. Verbindung nach Anspruch 1, wobei R₆, falls vorhanden, die Seitenkette von Asp oder Glu bedeutet.

9. Verbindung nach Anspruch 8, wobei R₆, falls vorhanden, die Seitenkette von Asp bedeutet.

10. Verbindung nach Anspruch 1, wobei a den Wert 0 hat und A₆ fehlt.

11. Verbindung nach Anspruch 1, wobei R₅, falls vorhanden, die Seitenkette einer Aminosäure bedeutet, die aus der folgenden Gruppe ausgewählt ist: D-Asp, L-Asp, D-Glu, L-Glu, D-Val, L-Val, D-tert.-Butylglycin (Tbg) und L-Tbg.

12. Verbindung nach Anspruch 11, wobei R₅, falls vorhanden, die Seitenkette von D-Asp, D-Val oder D-Glu bedeutet.

13. Verbindung nach Anspruch 12, wobei R₅, falls vorhanden, die Seitenkette von D-Glu bedeutet.

14. Verbindung nach Anspruch 1, wobei R₄ die Seitenkette einer Aminosäure bedeutet, die aus der Gruppe Val, Cyclohexylglycin (Chg), Tbg, Ile oder Leu ausgewählt ist.

15. Verbindung nach Anspruch 14, wobei R₄ die Seitenkette von Chg oder Ile bedeutet.

16. Verbindung nach Anspruch 15, wobei R₄ die Seitenkette von Chg bedeutet.

17. Verbindung nach Anspruch 1, wobei Y H oder Me bedeutet.

18. Verbindung nach Anspruch 17, wobei Y H bedeutet.

19. Verbindung nach Anspruch 1, wobei R₃ die Seitenkette einer Aminosäure bedeutet, die aus der Gruppe IIe, Chg, Val oder Tbg ausgewählt ist.

20. Verbindung nach Anspruch 19, wobei R₃ die Seitenkette von Val, Chg oder Tbg bedeutet.

21. Verbindung nach Anspruch 20, wobei R₃ die Seitenkette von Val oder Tbg bedeutet.

22. Verbindung nach Anspruch 1, wobei R₂ S-R₂₀ oder O-R₂₀ bedeutet, wobei R₂₀ C₆- oder C₁₀-Aryl, C₇₋₁₆-Aralkyl, Het oder -CH₂-Het bedeutet, die alle gegebenenfalls mit R₂₁ mono-, di- oder trisubstituiert sind;
wobei R₂₁ C₁₋₆-Alkyl; C₁₋₆-Alkoxy; Amino; Mono- oder Di-(niederalkyl)-amino; Amido, gegebenenfalls monosubstituiert mit C₁₋₆-Alkyl, C₆- oder C₁₀-Aryl, C₇₋₁₆₋Aralkyl, Het oder (Niederalkyl)-Het; NO₂; OH; Halogen; Trifluormethyl; Carboxyl; C₆- oder C₁₀-Aryl, C₇₋₁₆-Aralkyl oder Het, wobei das Aryl, Aralkyl oder Het gegebenenfalls durch R₂₂ substituiert sind, bedeutet;
wobei R₂₂ C₁₋₆-Alkyl; C₁₋₆-Alkoxy, Amino; Mono- oder Di-(niederalkyl)-amino; (Niederalkyl)-amid; NO₂; OH; Halogen; Trifluormethyl; oder Carboxyl bedeutet.

23. Verbindung nach Anspruch 22, wobei R₂₁ C₁₋₆-Alkyl; C₁₋₆-Alkoxy; Amino; Di-(niederalkyl)-amino; (Niederalkyl)-amid; C₆- oder C₁₀-Aryl oder Het bedeutet, wobei das Aryl oder Het gegebenenfalls mit R₂₂ substituiert sind; wobei R₂₂ C₁₋₆-Alkoxy; Amino; Di-(niederalkyl)-amino; (Niederalkyl)-amid; Halogen oder Trifluormethyl bedeutet.

24. Verbindung nach Anspruch 22, wobei R₂ 1-Naphthylmethoxy; 2-Naphthylmethoxy; Benzyloxy, 1-Naphthyloxy; 2-Naphthyloxy oder Chinolinoxy, das unsubstituiert oder mit R₂₁ mono- oder disubstituiert ist, wobei R₂₁ die in Anspruch 22 definierte Bedeutung hat, bedeutet.

25. Verbindung nach Anspruch 22, wobei R₂ 1-Naphthylmethoxy; oder Chinolinoxy, das unsubstituiert oder mit R₂₁ mono- oder disubstituiert ist, wobei R₂₁ die in Anspruch 22 definierte Bedeutung hat, bedeutet.

26. Verbindung nach Anspruch 25, wobei R₂ die folgende Bedeutung hat: 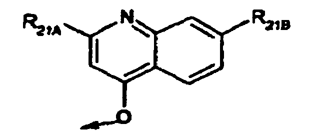
wobei R_{21A} Amido, das gegebenenfalls mit C₁₋₆-Alkyl, C₆- oder C₁₀-Aryl, C₇₋₁₆-Aralkyl oder Het substituiert ist; C₆- oder C₁₀-Aryl oder Het, das gegebenenfalls mit R₂₂ substituiert ist, bedeutet und R₂₂ Amino; Di-(niederalkyl)-amino; oder (Niederalkyl)-amid bedeutet; und R_{21B} C₁₋₆-Alkyl; C₁₋₆-Alkoxy; Amino; Di-(niederalkyl)-amino; (Niederalkyl)-amid; NO₂; OH; Halogen; Trifluormethyl; oder Carboxyl bedeutet.

27. Verbindung nach Anspruch 26, wobei R_{21A} C₆- oder C₁₀-Aryl oder Het bedeutet, die alle gegebenenfalls mit R₂₂ substituiert sind, und R₂₂ Amino, Dimethylamino oder Acetamido bedeutet.

28. Verbindung nach Anspruch 26, wobei R_{21B} C₁₋₆-Alkoxy oder Di-(niederalkyl)-amino bedeutet.

29. Verbindung nach Anspruch 28, wobei R_{21 B} Methoxy bedeutet.

30. Verbindung nach Anspruch 1, wobei der asymmetrische Kohlenstoff in Position 1 die R-Konfiguration aufweist, die durch die folgenden absoluten Konfigurationen dargestellt ist: 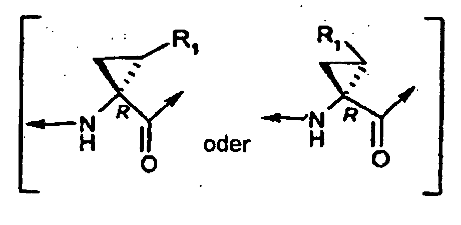
wobei R₁ die in Anspruch 1 definierte Bedeutung hat.

31. Verbindung nach Anspruch 30, wobei der R₁-Substituent an P1 in syn-Stellung zur Carbonylgruppe gemäß der folgenden absoluten Konfiguration orientiert ist: 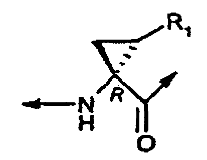
wobei R₁ Methyl, Ethyl, Propyl oder Vinyl bedeutet, die alle gegebenenfalls mit Halogen substituiert sind.

32. Verbindung nach Anspruch 31, wobei R₁ Ethyl, Vinyl oder Bromvinyl bedeutet.

33. Verbindung nach Anspruch 32, wobei R₁ Vinyl bedeutet.

34. Verbindung nach Anspruch 1, wobei W Hydroxy oder ein pharmazeutisch verträgliches Salz oder einen Ester davon; oder (Niederalkyl)-amino, Di-(niederalkyl)-amino oder Aminoaralkyl bedeutet.

35. Verbindung nach Anspruch 32, wobei W Hydroxy oder N(R₁₃ₐ)R_{13b}, wobei R₁₃ₐ und R_{13b} unabhängig voneinander H, Aryl oder C₁₋₆-Alkyl bedeutet, die gegebenenfalls mit Hydroxy oder Phenyl substituiert sind; oder ein pharmazeutisch verträgliches Salz davon bedeutet.

36. Verbindung nach Anspruch 35, wobei W -OH, -NH-Benzyl oder -NH-CH(Me)Ph bedeutet.

37. Verbindung nach Anspruch 36, wobei W -OH oder -NH-(S)CH(Me)-phenyl bedeutet.

38. Verbindung nach Anspruch 37, wobei W einen Ester bedeutet, wobei der Ester aus der Gruppe C₁₋₆-Alkoxy, Phenoxy oder Aryl-(C₁₋₆-alkoxy) ausgewählt ist.

39. Verbindung nach Anspruch 38, wobei es sich beim Ester um Methoxy, Ethoxy, Phenoxy, Benzyloxy oder PhCH(Me)-O- handelt.

40. Verbindung der Formel I nach Anspruch 1, wobei B H, Niederalkyl-C(O)- oder Het-C(O)- bedeutet;
R₆, falls vorhanden, die Seitenkette von Asp oder Glu bedeutet;
R₅, falls vorhanden, die Seitenkette von D- oder L-Asp, -Glu, -Val oder -Tbg bedeutet;
Y H oder Methyl bedeutet;
R₄ die Seitenkette von Val, Chg, Tbg, Ile oder Leu bedeutet;
R₃ Wasserstoff oder die Seitenkette von Ile, Chg, Val oder Tbg bedeutet;
R₂ 1-Naphthylmethoxy, 2-Naphthylmethoxy, O-Bn, 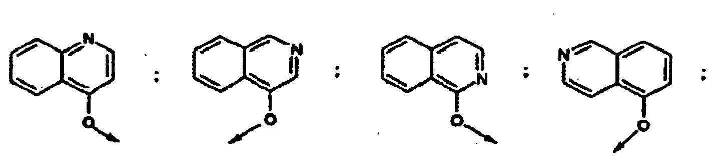 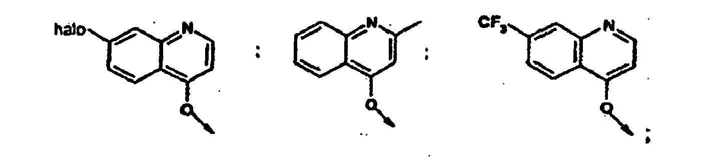 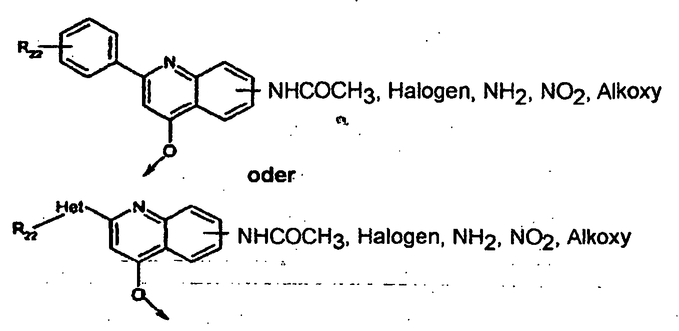
bedeutet, wobei R₂₂ Amino, Di-(niederalkyl)-amino, (Niederalkyl)-amid, NO₂, OH, Halogen, CF₃ oder COOH bedeutet;
P1 ein Cyclopropyl-Ringsystem der Formel 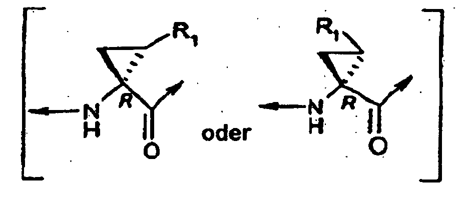
bedeutet,
wobei R₁ Ethyl, Vinyl oder Bromvinyl bedeutet; und
W Hydroxy oder N(R₁₃ₐ)R_{13b} bedeutet, wobei R₁₃ₐ und R_{13b} unabhängig voneinander H, Aryl oder C₁₋₆-Alkyl, gegebenenfalls substituiert mit Hydroxy oder Phenyl; oder ein pharmazeutisch verträgliches Salz oder Ester davon bedeutet.

41. Verbindung der Formel I nach Anspruch 1, wobei B H, Acetyl oder Het-C(O)- bedeutet;
R₆, falls vorhanden, die Seitenkette von Asp bedeutet; R₅, falls vorhanden, die Seitenkette von D-Asp, D-Glu oder D-Val bedeutet; Y H bedeutet; R₄ die Seitenkette von Chg oder Ile bedeutet; R₃ die Seitenkette von Val, Chg oder Tbg bedeutet; R₂ 1-Naphthylmethoxy, Benzyloxy, 4-Chinolinoxy oder 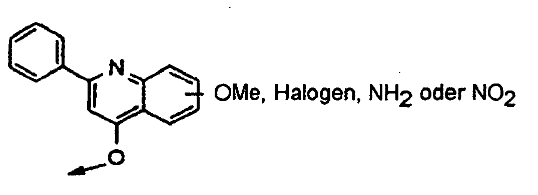
bedeutet,
P1 ein Cyclopropyl-Ringsystem der folgenden Formel bedeutet: 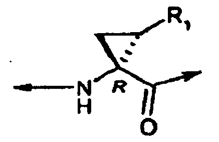
wobei R₁ Et oder CH=CH₂ oder CH=CHBr bedeutet; und
W Hydroxy oder NH-(S)-CHMePh oder ein pharmazeutisch verträgliches Salz davon bedeutet.

42. Verbindung der Formel I nach Anspruch 1, wobei B Acetyl bedeutet; R₆, falls vorhanden, die Seitenkette von Asp bedeutet; R₅, falls vorhanden, die Seitenkette von D-Glu bedeutet; Y H bedeutet; R₄ die Seitenkette von Chg bedeutet; R₃ die Seitenkette von Val oder Tbg bedeutet; R₂ die folgende Bedeutung hat: 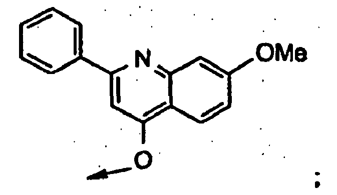
**P1** die folgende Bedeutung hat: 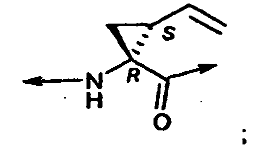
und
W Hydroxy oder ein pharmazeutisch verträgliches Salz oder Ester davon bedeutet.

43. Verbindung nach Anspruch 40 der folgenden Formel: 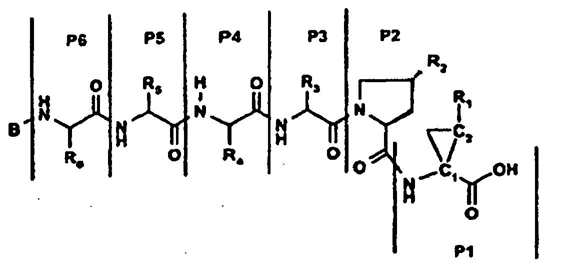
wobei B, P6, P5, P4, P3, R₂ und R₁ die nachstehend definierten Bedeutungen haben:

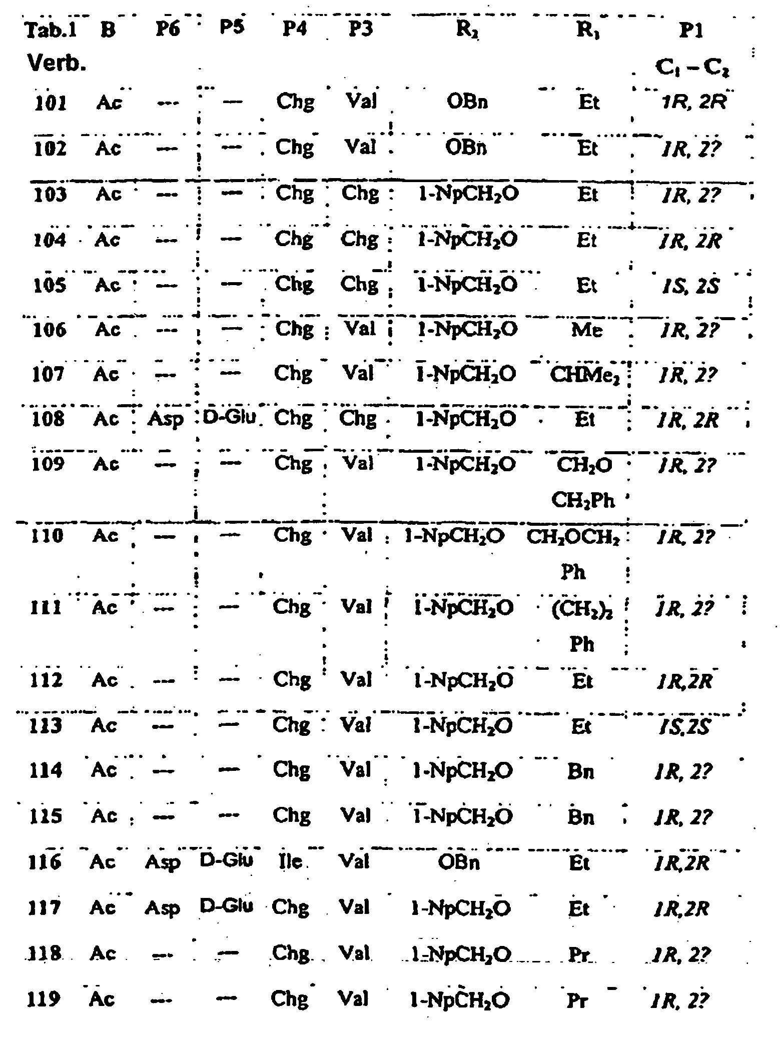

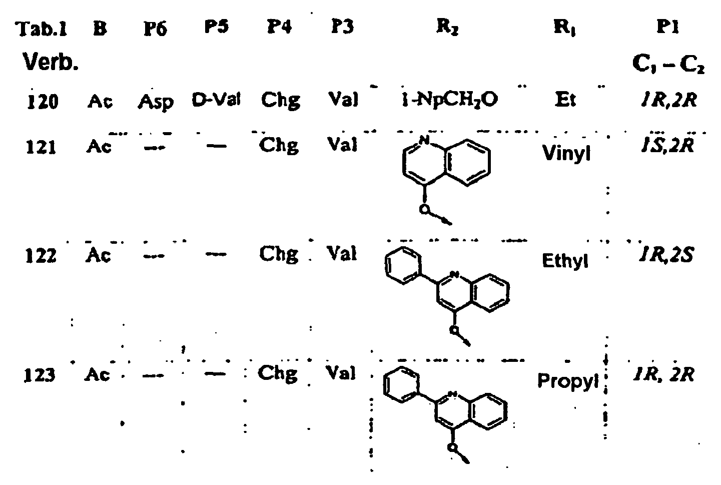

44. Verbindung nach Anspruch 40 der Formel: 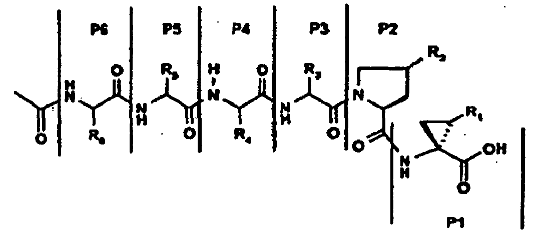
wobei P6, P5, P4, P3, R₂ und R₁ die nachstehend definierten Bedeutungen haben:

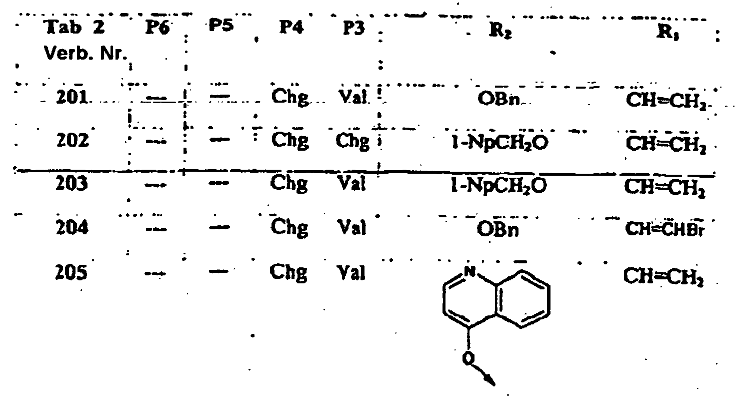

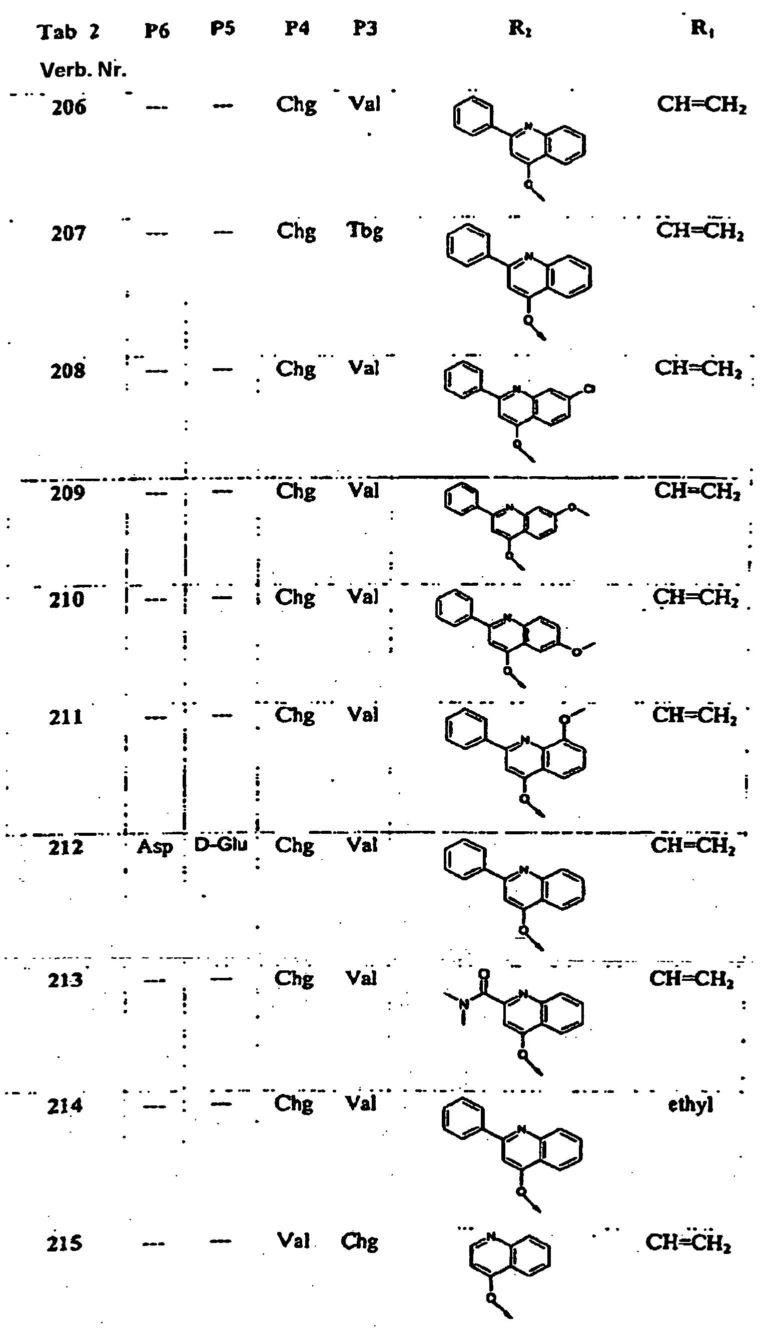

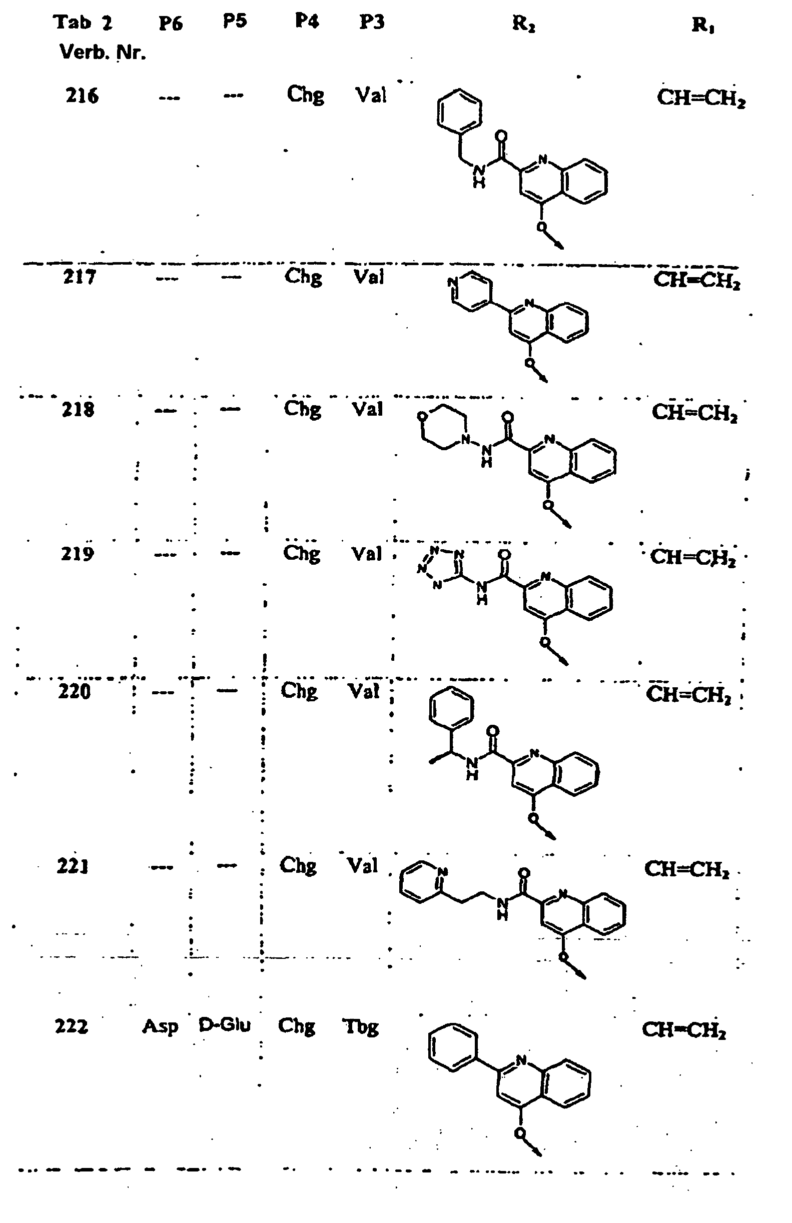

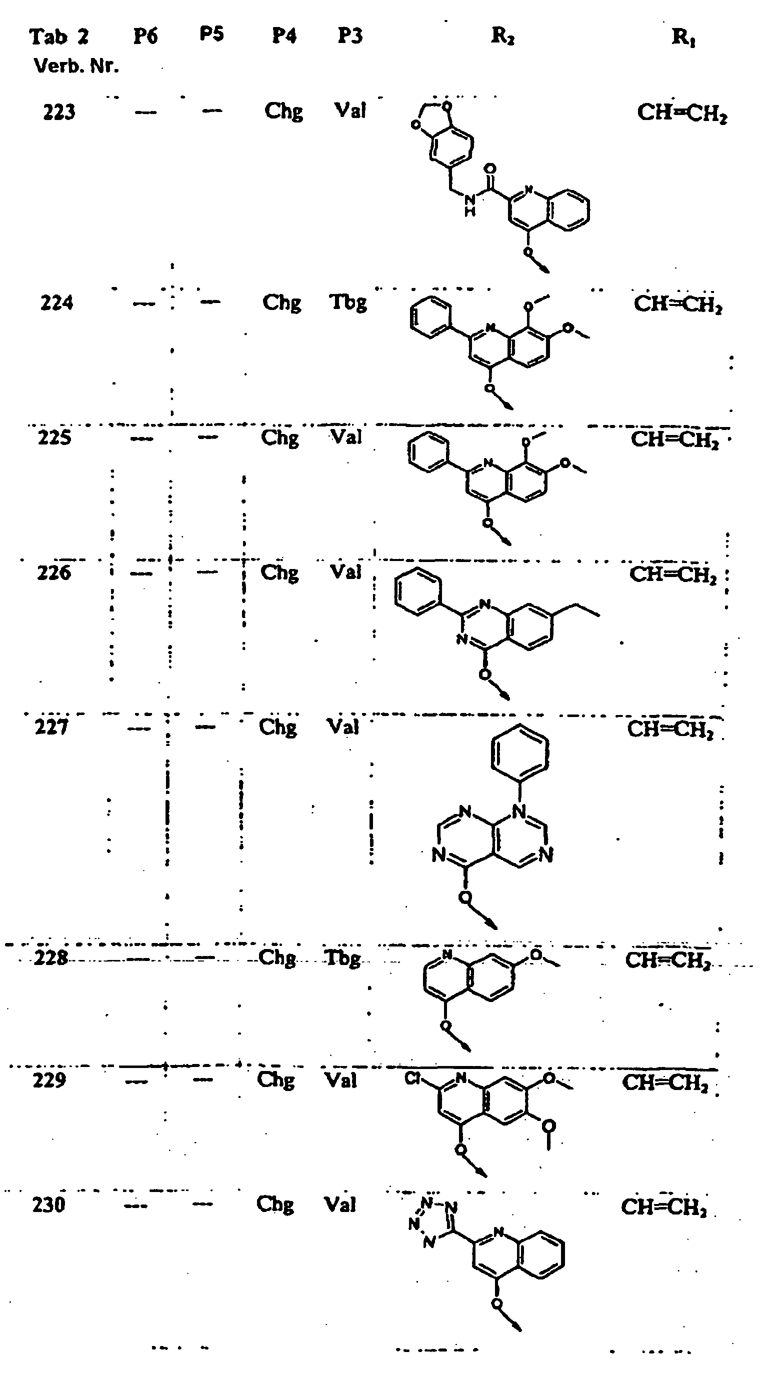

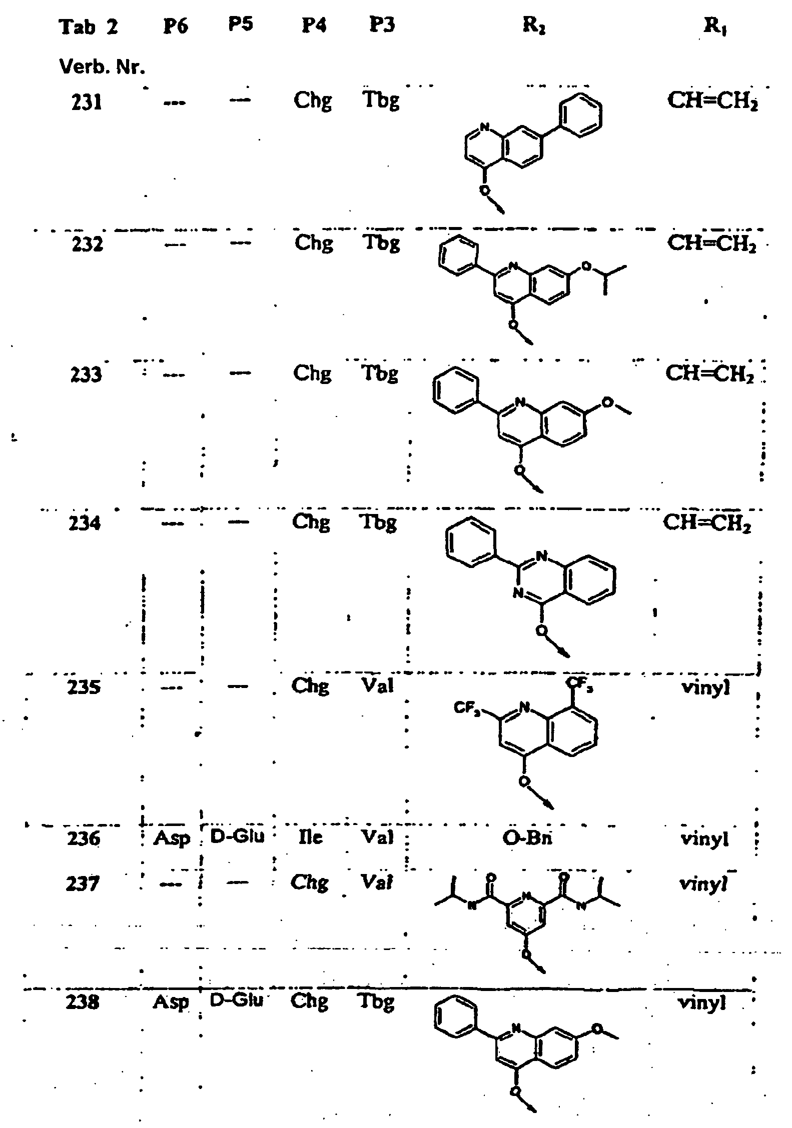

45. Verbindung nach Anspruch 40 der Formel: 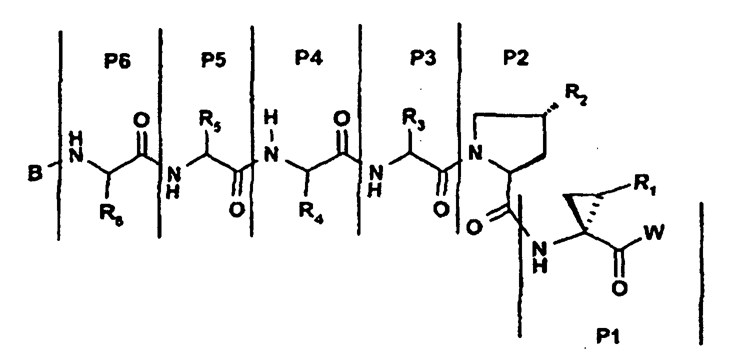
wobei B, P6, P5, P4, P3, R₂, R₁ und W die nachstehend definierten Bedeutungen haben:

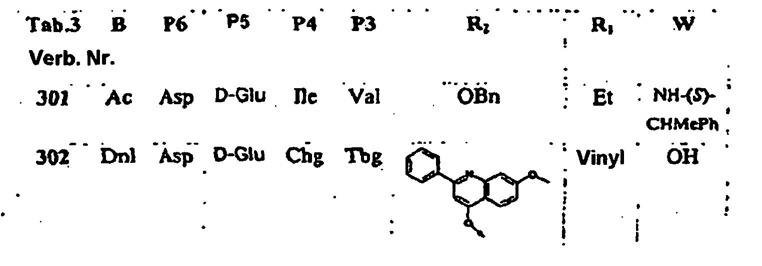

46. Verbindung nach Anspruch 40 der Formel: 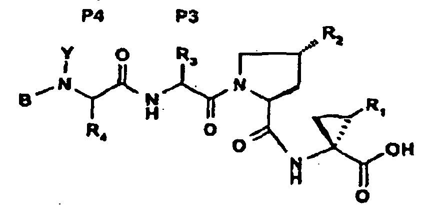
wobei B, Y, P4, P3, R₂ und R₁ die nachstehend definierten Bedeutungen haben:

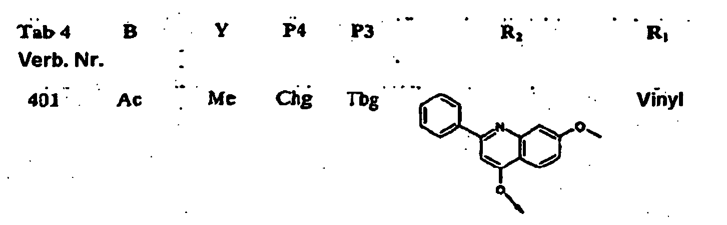

47. Verbindung nach Anspruch 40 der Formel: 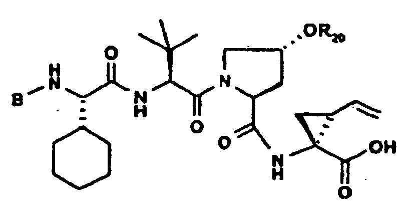
wobei B und R₂₀ die nachstehend definierten Bedeutungen haben:

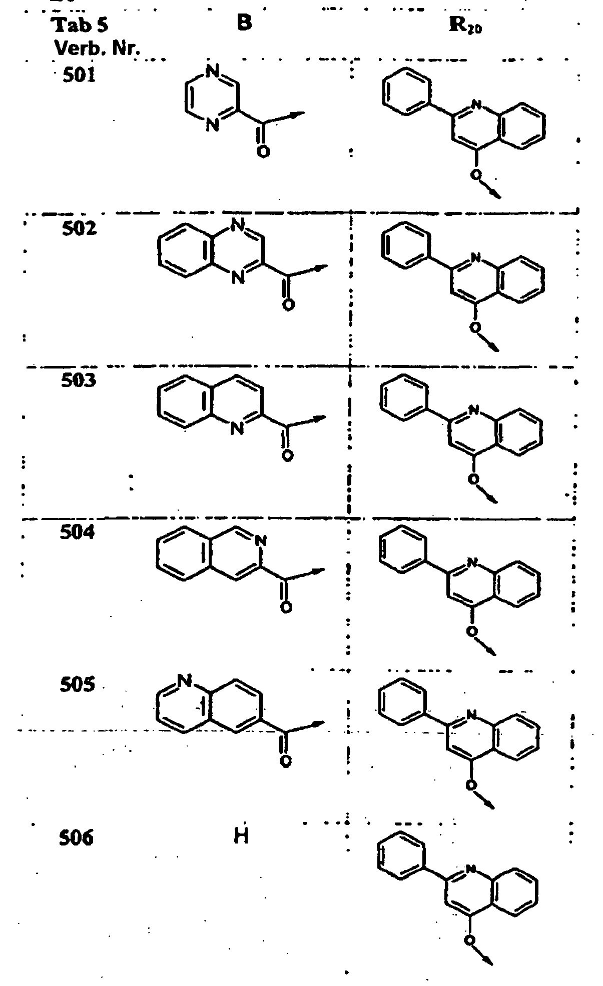

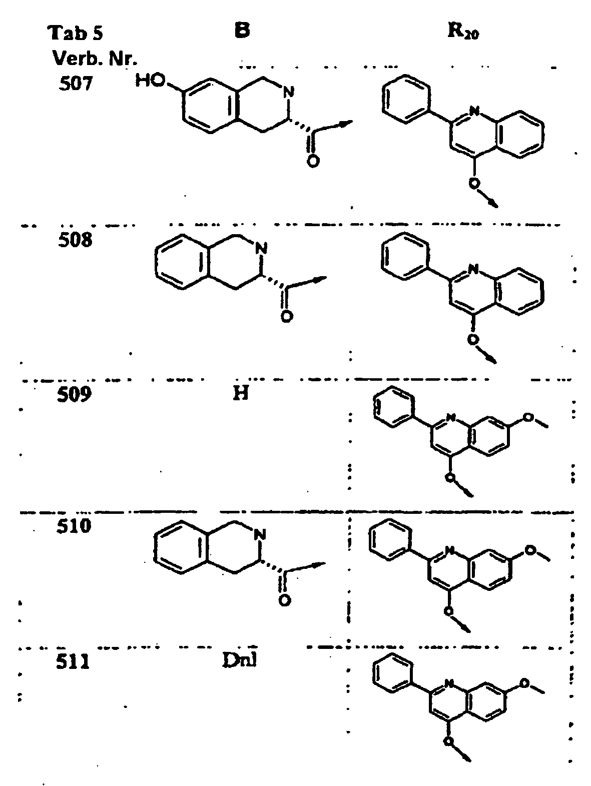

48. Hexapeptid der Formel I nach Anspruch 43, ausgewählt aus der Gruppe der Verbindungen Nr. 108, 116, 117 und 120.

49. Hexapeptid der Formel I nach Anspruch 44, ausgewählt aus der Gruppe der Verbindungen Nr. 212, 222, 236 und 238.

50. Hexapeptid der Formel I nach Anspruch 45, ausgewählt aus der Gruppe der Verbindungen Nr. 301 und 302.

51. Tetrapeptid der Formel I nach Anspruch 43, ausgewählt aus der Gruppe der Verbindungen Nr. 122 und 123.

52. Tetrapeptid der Formel I nach Anspruch 44, ausgewählt aus der Gruppe der Verbindungen Nr. 202, 203, 205, 206, 207, 208, 209, 210, 211, 214, 215, 216, 218, 219, 220, 221, 223, 224, 225, 226, 228, 229, 230, 231, 232, 233, 234, 235.

53. Tetrapeptid der Formel I nach Anspruch 46, ausgewählt aus der Gruppe der Verbindung Nr. 401.

54. Tetrapeptid der Formel I nach Anspruch 47, ausgewählt aus der Gruppe der Verbindungen Nr. 501, 502, 503, 504, 505, 506, 507, 508, 509, 510 und 511.

55. Verbindung nach einem der Ansprüche 1 bis 54 als Medikament.

56. Pharmazeutische Zusammensetzung, umfassend eine gegen Hepatitis C-Virus wirksame Menge einer Verbindung der Formel I nach Anspruch 1 oder eines therapeutisch verträglichen Salzes oder Esters davon im Gemisch mit einem pharmazeutisch verträglichen Trägermedium oder Hilfsstoff.

57. Verwendung einer Verbindung der Formel I nach Anspruch 1 oder eines therapeutisch verträglichen Salzes oder Esters davon bei der Herstellung eines Arzneimittels zur Behandlung einer viralen Hepatitis C-Infektion.

58. In vitro-Verfahren zur Hemmung der Replikation von Hepatitis C-Virus, bei dem man das Virus einer die virale Hepatitis C-NS3-protease hemmenden Menge der Verbindung der Formel I nach Anspruch 1 oder eines therapeutisch verträglichen Salzes oder Esters davon oder einer Zusammensetzung nach Anspruch 56 aussetzt.

59. Verwendung einer Kombination der Verbindung der Formel I nach Anspruch 1 oder eines therapeutisch annehmbaren Salzes oder Esters davon und eines Interferons bei der Herstellung eines Arzneimittels zur Behandlung einer viralen Hepatitis C-Infektion.

60. Pharmazeutische Zusammensetzung nach Anspruch 56, ferner umfassend ein zweites antivirales Mittel.

61. Pharmazeutische Zusammensetzung nach Anspruch 60, wobei es sich beim zweiten antiviralen Mittel um Ribavirin oder Amantadin handelt.

62. Pharmazeutische Zusammensetzung nach Anspruch 56, ferner umfassend weitere Inhibitoren von HCV-Protease.

63. Pharmazeutische Zusammensetzung nach Anspruch 56, ferner umfassend einen Inhibitor von anderen Zielen im HCV-Lebenszyklus, ausgewählt aus Helicase, Polymerase, Metalloprotease oder IRES.

64. Verfahren zur Herstellung eines Peptidanalogen der Formel (I) nach Anspruch 1, wobei P1 einen substituierten Aminocyclopropylcarbonsäurerest bedeutet, umfassend die folgenden Stufen:
• Kupplung eines Peptids, das aus der Gruppe APG-P6-P5-P4-P3-P2; APG-P5-P4-P3-P2; APG-P4-P3-P2; APG-P3-P2 und APG-P2 ausgewählt ist;
• mit einem P1-Zwischenprodukt der Formel: 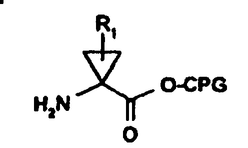
wobei R₁ C₁₋₆-Alkyl oder C₂₋₆-Alkenyl, gegebenenfalls substituiert mit Halogen, bedeutet, CPG eine Carboxylschutzgruppe bedeutet und P6 bis P2 die in Anspruch 1 definierten Bedeutungen haben.

65. Verfahren zur Herstellung eines Peptidanalogen der Formel (I) nach Anspruch 1, wobei P1 einen substituierten Aminocyclopropylcarbonsäurerest bedeutet, umfassend die folgende Stufe:
• Kupplung eines Peptids, das aus der Gruppe APG-P6-P5-P4-P3-P2; APG-P5-P4-P3-P2; APG-P4-P3-P2; APG-P3-P2 und APG-P2 ausgewählt ist;
• mit einem P1-Zwischenprodukt der Formel 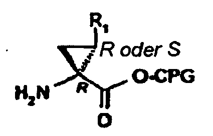
wobei R₁ Ethyl, Vinyl oder Bromvinyl bedeutet, CPG eine Carboxylschutzgruppe bedeutet und P6 bis P2 die in Anspruch 1 definierten Bedeutungen haben.

66. Verfahren zur Herstellung eines Peptidanalogen der Formel (I) nach Anspruch 1, wobei P1 einen substituierten Aminocyclopropylcarbonsäurerest bedeutet, umfassend die folgende Stufe:
• Kupplung eines Peptids, das aus der Gruppe APG-P6-P5-P4-P3-P2; APG-P5-P4-P3-P2; APG-P4-P3-P2; APG-P3-P2 und APG-P2 ausgewählt ist;
• mit einem P1-Zwischenprodukt der Formel 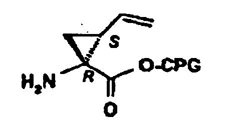
wobei CPG eine Carboxylschutzgruppe bedeutet und P6 bis P2 die in Anspruch 1 definierten Bedeutungen haben.

67. Verfahren nach Anspruch 64, 65 oder 66, wobei die Carboxylschutzgruppe (CPG) aus der folgenden Gruppe ausgewählt ist:
Alkylester, Aralkylester und Ester, die durch milde Basenbehandlung oder milde reduktive Maßnahmen spaltbar sind.

68. Verwendung eines Aminosäureanalogen der Formel: 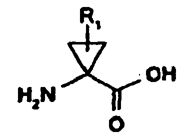
wobei R₁ C₁₋₆-Alkyl oder C₂₋₆-Alkenyl, gegebenenfalls substituiert mit Halogen, bedeutet, zur Herstellung einer Verbindung der Formel I nach Anspruch 1.

69. Verwendung eines Aminosäureanalogen der Formel: 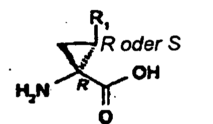
wobei R₁ Ethyl, Vinyl oder Bromvinyl bedeutet, zur Herstellung einer Verbindung der Formel I nach Anspruch 1.

70. Verwendung eines Aminosäureanalogen der Formel: 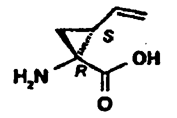
zur Herstellung einer Verbindung der Formel I nach Anspruch 1.

## Revendications

1. Composé de formule (I), y compris les racémates, diastéréoisomères et isomères optiques : 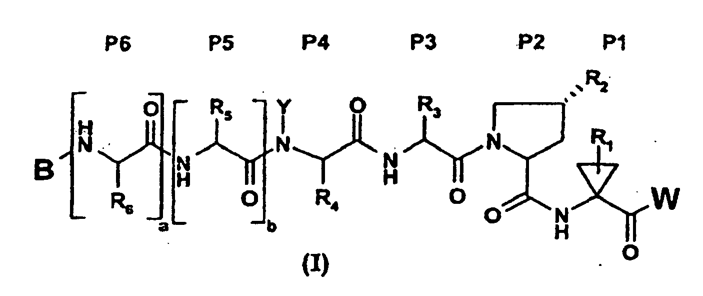
où
**a** est 0 ou 1 ; **b** est 0 ou 1 ; **Y** est H ou C₁₋₆ alkyle ;
**B** est H, un dérivé acyle de formule **R**_{**7**}-C(O)- ou un sulfonyle de formule **R**_{**7**}-SO₂ où
**R**_{**7**} est
(i) C₁₋₁₀ alkyle éventuellement substitué avec carboxyle, C₁₋₆ alcanoyloxy ou C₁₋₆ alcoxy ;
(ii) C₃₋₇ cycloalkyle éventuellement substitué avec carboxyle, (C₁₋₆ alcoxy)carbonyle ou phénylméthoxycarbonyle ;
(iii) C₆ ou C₁₀ aryle ou C₇₋₁₆ aralkyle éventuellement substitué avec C₁₋₆ alkyle, hydroxy, ou amino éventuellement substitué avec C₁₋₆ alkyle ; ou
(iv) Het éventuellement substitué avec C₁₋₆ alkyle, hydroxy, amino éventuellement substitué avec C₁₋₆ alkyle, ou amido éventuellement substitué avec C₁₋₆ alkyle ;
**R**_{**6**} est C₁₋₆ alkyle substitué avec carboxyle ;
**R**_{**5**} est C₁₋₆ alkyle éventuellement substitué avec carboxyle ;
**R**_{**4**} est C₁₋₁₀ alkyle, C₃₋₇ cycloalkyle ou C₄₋₁₀ (alkylcycloalkyle) ;
**R**_{**3**} est C₁₋₁₀ alkyle, C₃₋₇ cycloallcyle ou C₄₋₁₀ (alkylcycloalkyle) ;
**R**_{**2**} est CH₂-**R**_{**20**}, NH-**R**_{**20**}, O-**R**_{**20**} ou S-**R**_{**20**}, où **R**_{**20**} est un C₃₋₇ cycloalkyle ou C₄₋₁₀ (alkylcycloalkyle) saturé ou insaturé qui est éventuellement mono-, di- ou tri-substitué avec **R**_{**21**}**,** ou **R**_{**20**} est un C₆ ou C₁₀ aryle ou C₇₋₁₆ aralkyle éventuellement mono-, di- ou tri-substitué avec **R**_{**21**}**,**
ou **R**_{**20**} est Het ou (alkyle inférieur)-Het éventuellement mono-, di- ou tri-substitué avec **R**_{**21**}**,**
où chaque **R**_{**21**} est indépendamment C₁₋₆ alkyle ; C₁₋₁₆ alcoxy ; amino éventuellement mono- ou di-substitué avec C₁₋₆ alkyle ; sulfonyle ; NO₂; OH ; SH ; halogéno ; halogénoalkyle ; amido éventuellement mono-substitué avec C₁₋₆ alkyle, C₆ ou C₁₀ aryle, C₇₋₁₆ aralkyle, Het ou (alkyle inférieur)-Het ; carboxyle ; carboxy(alkyle inférieur) ; C₆ ou C₁₀ aryle, C₇₋₁₆ aralkyle ou Het, ledit aryle, aralkyle ou Het étant éventuellement substitué avec **R**_{**22**} **;**
où **R**_{**22**} est C₁₋₆ alkyle ; C₁₋₆ alcoxy ; amino éventuellement mono- ou di-substitué avec C₁₋₆ alkyle ; sulfonyle ; NO₂ ; OH ; SH ; halogéno ; halogénoalkyle ; carboxyle ; amide ou (alkyle inférieur)amide ;
**R**_{**1**} est C₁₋₆ alkyle ou C₂₋₆ alcényle éventuellement substitué avec halogène ; et
**W** est hydroxy ou un amino N-substitué ;
ou sel ou ester pharmaceutiquement acceptable de celui-ci, avec la condition que le composé de formule (I) où
B est acétyle,
a et b sont 0,
P4 représente l'aminoacide cyclohexylglycine (Chg) ;
P3 représente l'aminoacide valine (Val) ;
R₂ représente benzyoxy (O-Bn) ;
P1 représente un groupe racémique de formule 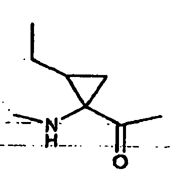
et W représente OH est exclu.

2. Composé selon la revendication 1 où **B** est H ou un dérivé acyle de formule **R**_{**7**}C(O)- où **R**_{**7**} est C₁₋₆ alkyle ; C₁₋₆ alcoxy; C₃₋₇ cycloalkyle éventuellement substitué avec hydroxy ; amido éventuellement substitué avec C₁₋₆ alkyle ou Het; C₆ ou C₁₀ aryle, C₇₋₁₆ aralkyle ou Het tous éventuellement substitués avec C₁₋₆ alkyle ou hydroxy.

3. Composé selon la revendication 2 où **R**_{**7**} est C₁₋₆ alkyle ou Het.

4. Composé selon la revendication 3 où ledit Het est choisi dans le groupe consistant en :

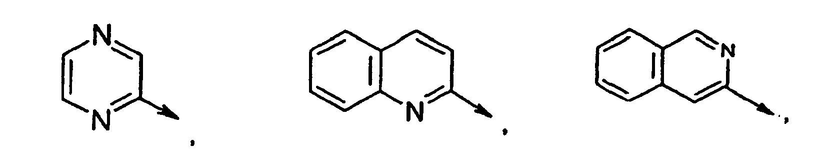

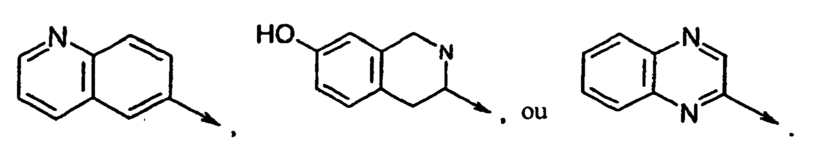

5. Composé selon la revendication 2 où **B** est choisi dans le groupe consistant en :
H, acétyle ;

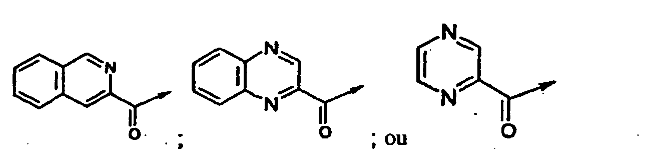

6. Composé selon la revendication 5 où **B** est acétyle.

7. Composé selon la revendication 1 où **B** est **R**_{**7**}-SO₂ et **R**_{**7**} est C₆ ou C₁₀ aryle, C₇₋₁₆ aralkyle ou Het, tous éventuellement substitués avec C₁₋₆ alkyle.

8. Composé selon la revendication 1 où **R**_{**6**}, quand il est présent, est la chaîne latérale de Asp ou Glu.

9. Composé selon la revendication 8 où **R**_{**6**}**,** quand il est présent, est la chaîne latérale de Asp.

10. Composé selon la revendication 1 où a est 0 et alors **R**_{**6**} est absent.

11. Composé selon la revendication 1 où **R**_{**5**}**,** quand il est présent, est la chaîne latérale d'un aminoacide choisi dans le groupe consistant en : D-Asp, L-Asp, D-Glu, L-Glu, D-Val, L-Val, D-tert-butylglycine (Tbg) et L-Tbg.

12. Composé selon la revendication 11 où **R**_{**5**}**,** quand il est présent, est la chaîne latérale de D-Asp, D-Val ou D-Glu.

13. Composé selon la revendication 12 où **R**_{**5**}**,** quand il est présent, est la chaîne latérale de D-Glu.

14. Composé selon la revendication 1 où **R**_{**4**} est la chaîne latérale d'un aminoacide choisi dans le groupe consistant en : Val, la cyclohexylglycine (Chg), Tbg, Ile ou Leu.

15. Composé selon la revendication 14 où **R**_{**4**} est la chaîne latérale de Chg ou Ile.

16. Composé selon la revendication 15 où **R**_{**4**} est la chaîne latérale de Chg.

17. Composé selon la revendication 1 où **Y** est H ou Me.

18. Composé selon la revendication 17 où **Y** est H.

19. Composé selon la revendication 1 où **R**_{**3**} est la chaîne latérale d'un aminoacide choisi dans le groupe consistant en : Ile, Chg, Val ou Tbg.

20. Composé selon la revendication 19 où **R**_{**3**} est la chaîne latérale de Val, Chg ou Tbg.

21. Composé selon la revendication 20 où **R**_{**3**} est la chaîne latérale de Val ou Tbg.

22. Composé selon la revendication 1 où **R**_{**2**} est S-**R**_{**20**}, O-**R**_{**20**} où **R**_{**20**} est C₆ ou C₁₀ aryle, C₇₋₁₆ aralkyle, Het ou -CH₂-Het, tous éventuellement mono-, di- ou tri-substitués avec **R**_{**21**}**;**
où **R**_{**21**} est C₁₋₆ alkyle, C₁₋₆ alcoxy ; amino, mono- ou di-(alkyle inférieur)amino ; amido éventuellement mono-substitué avec C₁₋₆ alkyle, C₆ ou C₁₀ aryle, C₇₋₁₆ aralkyle, Het ou (alkyle inférieur)-Het ; NO₂ ; OH ; halogéno ; trifluorométhyle ; carboxyle ; C₆ ou C₁₀ aryle, C₇₋₁₆ aralkyle ou Het, ledit aryle, aralkyle ou Het étant éventuellement substitué avec **R**_{**22**} ;
où **R**_{**22**} est C₁₋₆ alkyle ; C₁₋₆ alcoxy ; amino ; mono- ou di-(alkyle inférieur)amino ; (alkyle inférieur)amide ; NO₂ ; OH ; halogéno ; trifluorométhyle ; ou carboxyle.

23. Composé selon la revendication 22 où **R**_{**21**} est C₁₋₆ alkyle ; C₁₋₆ alcoxy ; amino ; di-(alkyle inférieur)amino ; (alkyle inférieur)amide ; C₆ ou C₁₀) aryle, ou Het ; ledit aryle ou Het éventuellement substitué avec **R**_{**22**}**,** où **R**_{**22**} est C₁₋₆ alcoxy ; amino ; di-(alkyle inférieur)amino ; (alkyle inférieur)amide ; halogéno ou trifluorométhyle.

24. Composé selon la revendication 22 où **R**_{**2**} est 1-naphtylméthoxy ; 2-naphtylméthoxy ; benzyloxy, 1-naphtyloxy ; 2-naphtyloxy ; ou quinolinoxy non substitué, mono- ou disubstitué avec **R**_{**21**} où **R**_{**21**} est défini comme dans la revendication 23.

25. Composé selon la revendication 22 où **R**_{**2**} est 1-naphtylméthoxy ; ou quinolinoxy non substitué, mono- ou disubstitué avec **R**_{**21**} où **R**_{**21**} est défini comme dans la revendication 23.

26. Composé selon la revendication 25 où **R**_{**2**} est : 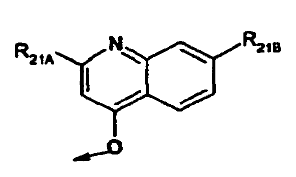
où **R**_{**21A**} est amido éventuellement substitué avec C₁₋₆ alkyle, C₆ ou C₁₀ aryle, C₇₋₁₆ aralkyle ou Het ; C₆ ou C₁₀ aryle ou Het éventuellement substitué avec **R**_{**22**}**,** et **R**_{**22**} est amino, di(alkyle inférieur)amino ; ou (alkyle inférieur)amide ; et **R**_{**21B**} est C₁₋₆ alkyle ; C₁₋₆ alcoxy ; amino ; di(alkyle inférieur)amino ; (alkyle inférieur)amide ; NO₂ ; OH ; halogéno ; trifluorométhyle ; ou carboxyle.

27. Composé selon la revendication 26 où **R**_{**21A**} est C₆ ou C₁₀ aryle ou Het, tous éventuellement substitués avec **R**_{**22**} et **R**_{**22**} est amino, diméthylamino ou acétamido.

28. Composé selon la revendication 26 où **R**_{**21B**} est C₁₋₆ alcoxy ou di(alkyle inférieur)amino.

29. Composé selon la revendication 28 où **R**_{**21B**} est méthoxy.

30. Composé selon la revendication 1 où le carbone asymétrique à la position 1 a la configuration *R*, représentée par les configurations absolues suivantes : 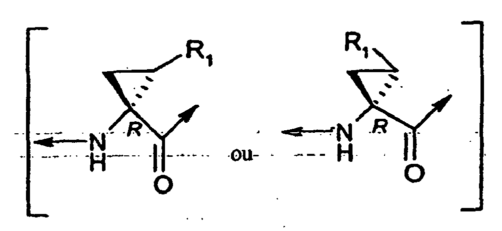
20 où **R**_{**1**} est défini comme dans la revendication 1.

31. Composé selon la revendication 30 où le substituant **R**_{**1**} sur **P1** est orienté *syn* par rapport au groupe carbonyle comme représenté par la configuration absolue suivante : 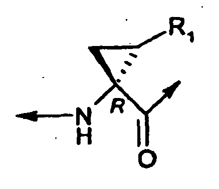
où **R**_{**1**} est méthyle, éthyle, propyle, vinyle, tous éventuellement substitués avec halogéno.

32. Composé selon la revendication 31 où **R**_{**1**} est éthyle, vinyle ou bromovinyle.

33. Composé selon la revendication 32 où **R**_{**1**} est vinyle.

34. Composé selon la revendication 1 où **W** est hydroxy ou un sel ou ester pharmaceutiquement acceptable de celui-ci ; ou (alkyle inférieur)amino, di(alkyle inférieur)amino ou amino aralkyle.

35. Composé selon la revendication 32 où W est hydroxy, ou **N(R**_{**13a**}**)R**_{**13b**} où **R**_{**13a**} et **R**_{**13b**} sont indépendamment H, aryle ou C₁₋₆ alkyle éventuellement substitué avec hydroxy ou phényle ; ou sel pharmaceutiquement acceptable de celui-ci.

36. Composé selon la revendication 35 où **W** est -OH, -NH-benzyle ou -NH-CH(Me)Ph.

37. Composé selon la revendication 36 où **W** est -OH ou -NH-(*S*)CH(Me)-phényle.

38. Composé selon la revendication 37 où **W** est un ester, ledit ester est choisi dans le groupe consistant en : C₁₋₆ alcoxy, phénoxy ou aryl(C₁₋₆ alcoxy).

39. Composé selon la revendication 38 où ledit ester est méthoxy, éthoxy, phénoxy, benzyloxy ou PhCH(Me)-O-.

40. Composé de formule I selon la revendication 1 où **B** est H, alkyle inférieur-C(O)- ou Het-C(O)- ;
**R**_{**6**}**,** quand il est présent, est la chaîne latérale de Asp ou Glu ;
**R**_{**5**}**,** quand il est présent, est la chaîne latérale de D- ou L- : Asp, Glu, Val ou Tbg ;
**Y** est H ou méthyle ;
**R**_{**4**} est la chaîne latérale de Val, Chg, Tbg, Ile ou Leu ;
**R**_{**3**} est l'hydrogène ou la chaîne latérale de Ile, Chg, Val ou Tbg ;
**R**_{**2**} est 1-naphtylméthoxy, 2-naphtylméthoxy, O-Bn, 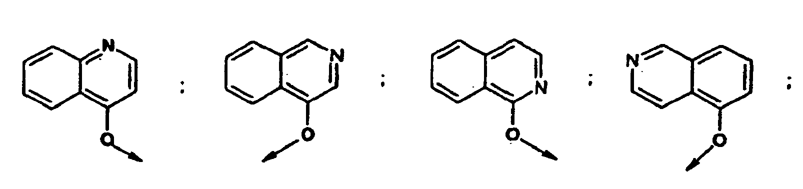 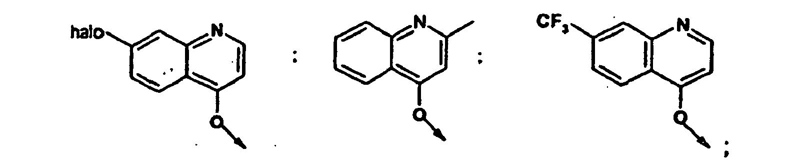 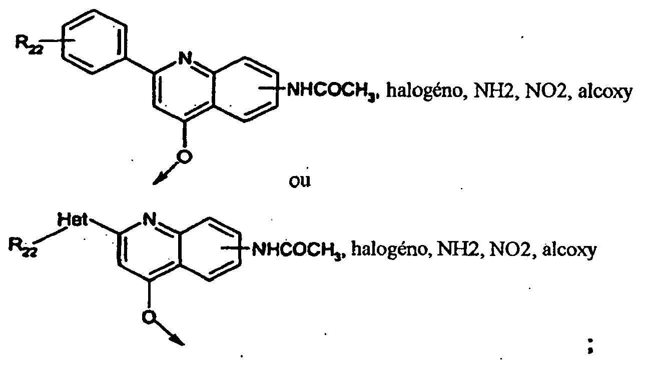
où **R**_{**22**} est amino, di(alkyle inférieur)amino, (alkyle inférieur)amide, NO₂, OH, halogéno, CF₃ ou COOH ;
**P1** est un système cyclique cyclopropyle de formule 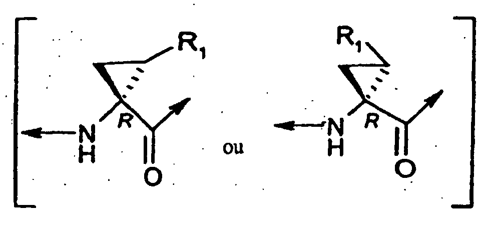
où **R**_{**1**} est éthyle, vinyle ou bromovinyle ; et
W est hydroxy ou **N(R**_{**13a**}**)R**_{**13b**} où **R**_{**13a**} et **R**_{**13b**} sont indépendamment H, aryle ou C₁₋₆ alkyle éventuellement substitué avec hydroxy ou phényle ; ou sel ou ester pharmaceutiquement acceptable de celui-ci.

41. Composé de formule I selon la revendication 1 où **B** est H, acétyle ou Het-C(O)- ; **R**_{**6**}**,** quand il est présent, est la chaîne latérale de Asp ; **R**_{**5**}**,** quand il est présent, est la chaîne latérale de D-Asp, D-Glu ou D-Val ; **Y** est H ; **R**_{**4**} est la chaîne latérale de Chg ou Ile ; **R**_{**3**} est la chaîne latérale de Val, Chg ou Tbg ; **R**_{**2**} est 1-naphtylméthoxy, benzyloxy, 4-quinolinoxy, ou 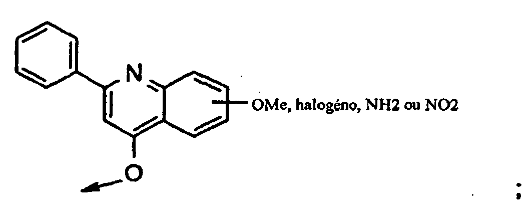
**P1** est un système cyclique cyclopropyle de formule 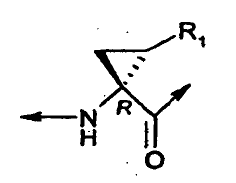
où **R**_{**1**} est Et ou CH=CH₂ ou CH=CHBr ; et
**W** est hydroxy ou NH-(*S*)-CHMePh, ou sel pharmaceutiquement acceptable de celui-ci.

42. Composé de formule I selon la revendication 1 où **B** est acétyle ; **R**_{**6**}**,** quand il est présent, est la chaîne latérale de Asp ; **R**_{**5**}**,** quand il est présent, est la chaîne latérale de D-Glu ; **Y** est H ; **R**_{**4**} est la chaîne latérale de Chg ; **R**_{**3**} est la chaîne latérale de Val ou Tbg ; **R**_{**2**} est : 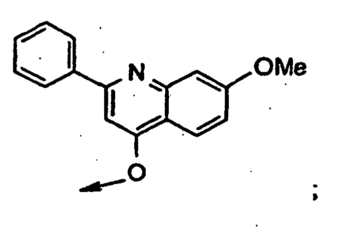
**P1** est: 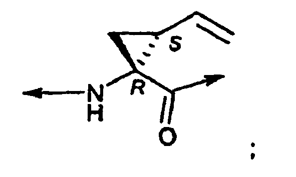
et
**W** est hydroxy, ou sel ou ester pharmaceutiquement acceptable de celui-ci.

43. Composé selon la revendication 40 représenté par la formule : 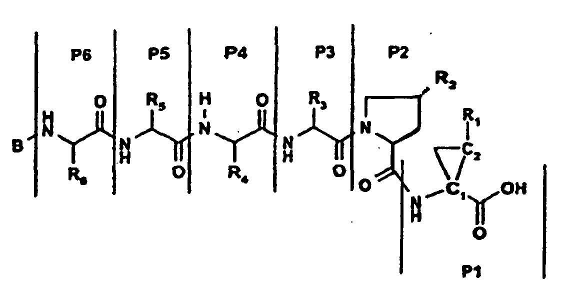
où **B, P6, P5, P4, P3, R**_{**2**} et **R**_{**1**} sont définis comme ci-dessous :

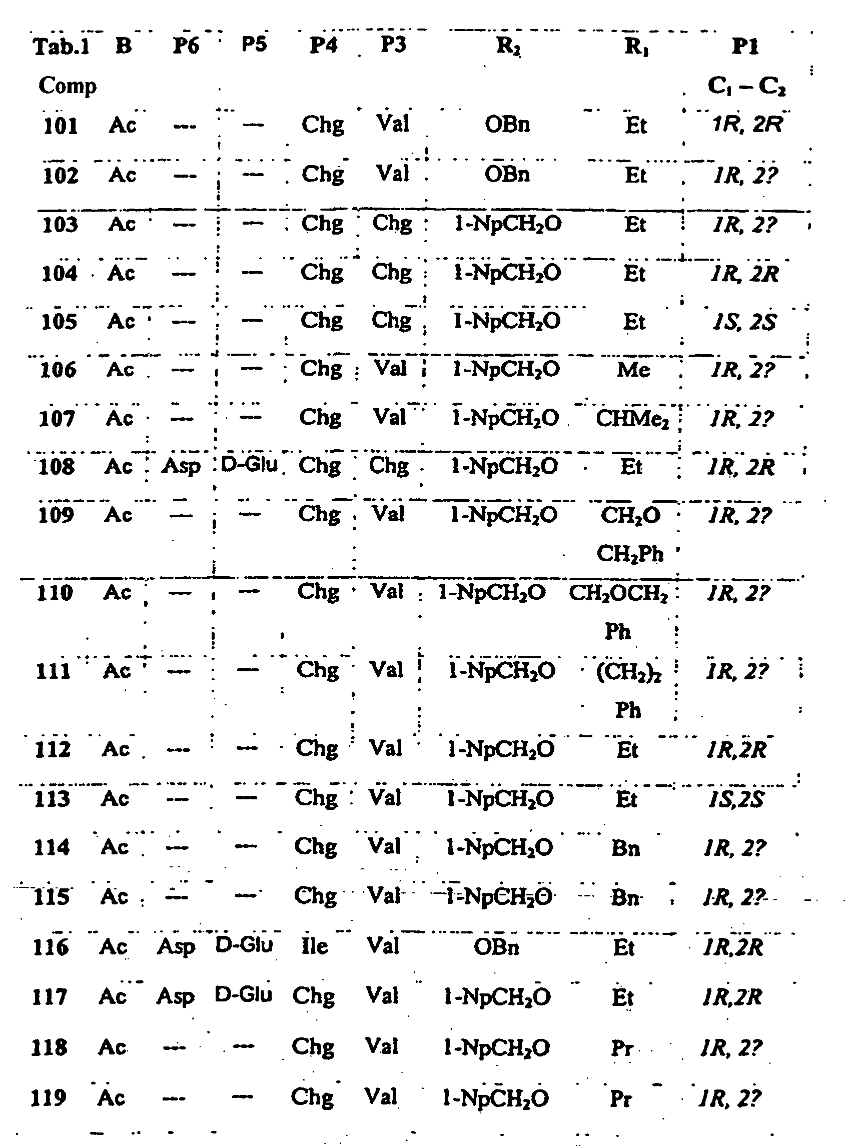

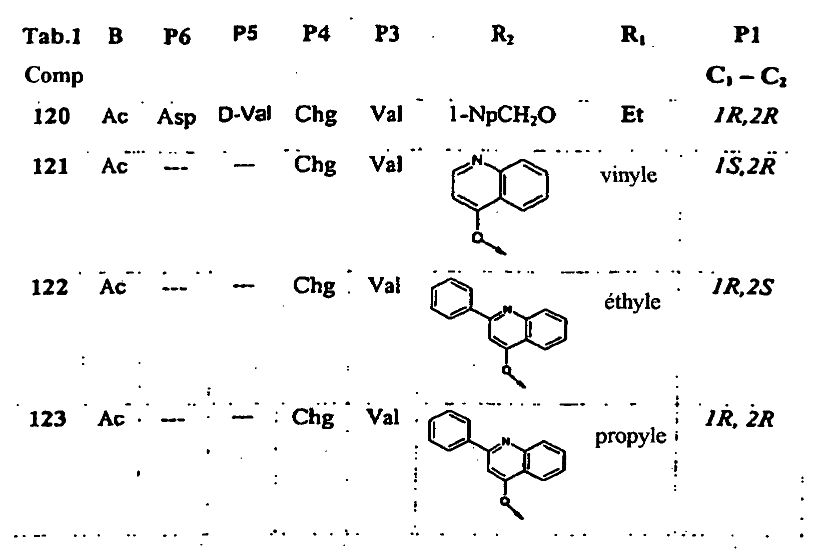

44. Composé selon la revendication 40 représenté par la formule : 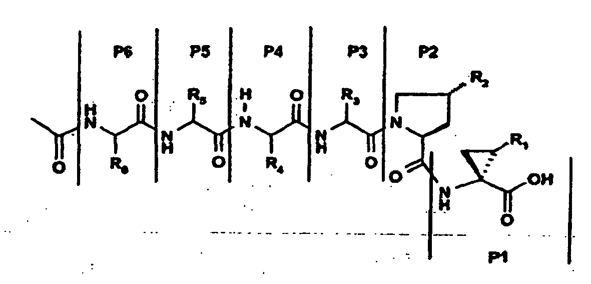
où **P6, P5, P4, P3, R**_{**2**} et **R**_{**1**} sont définis comme ci-dessous :

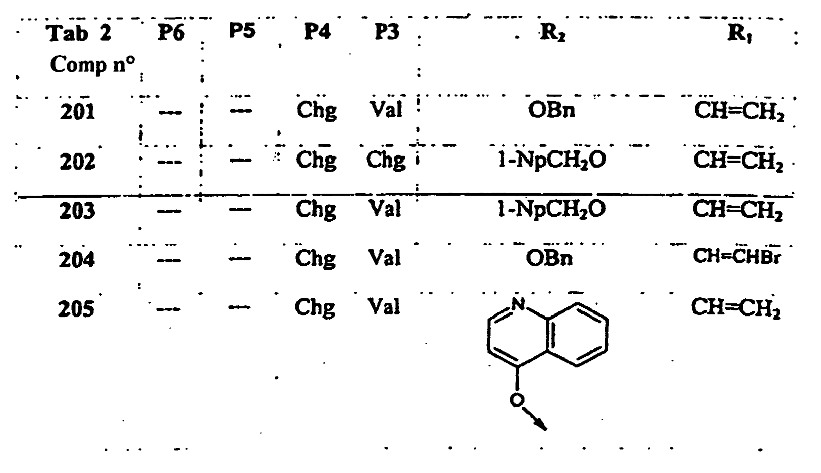

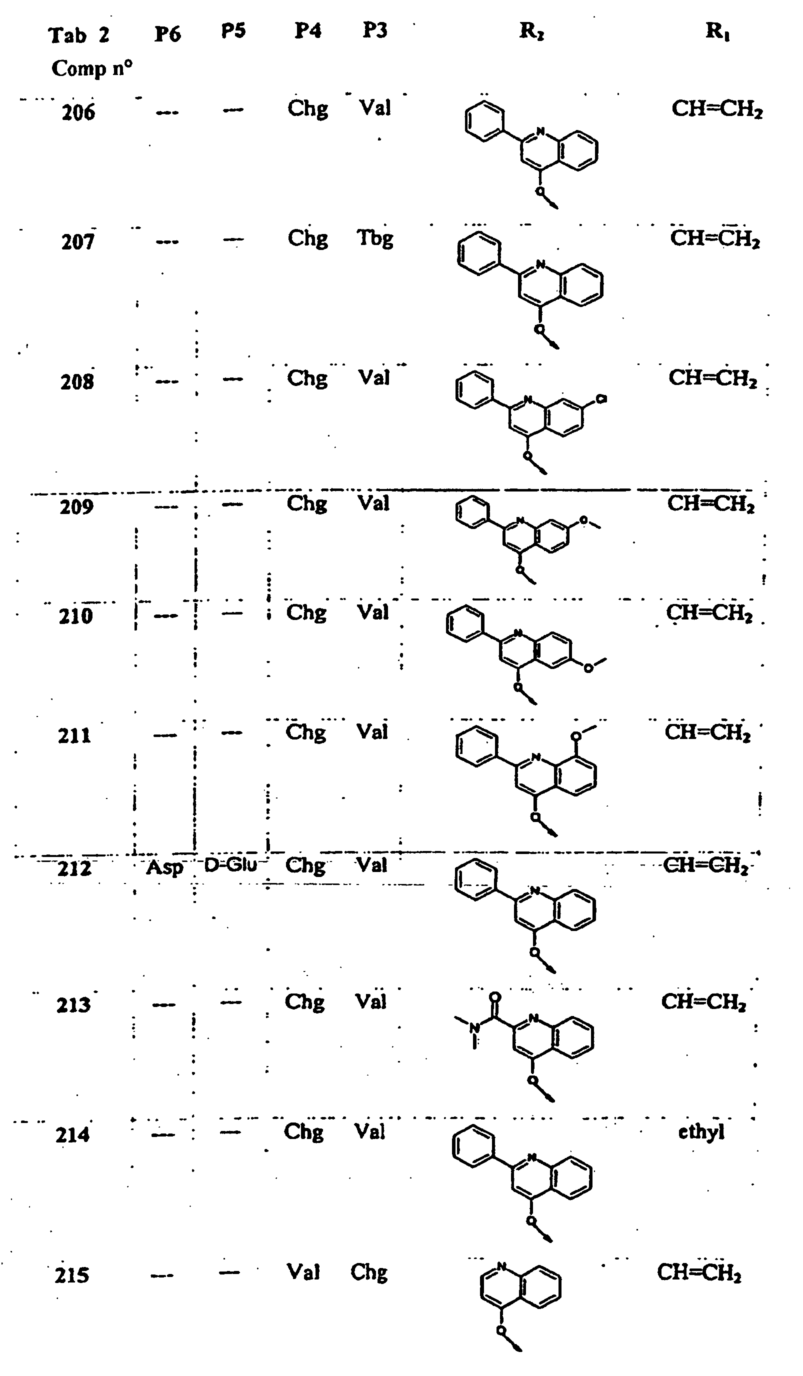

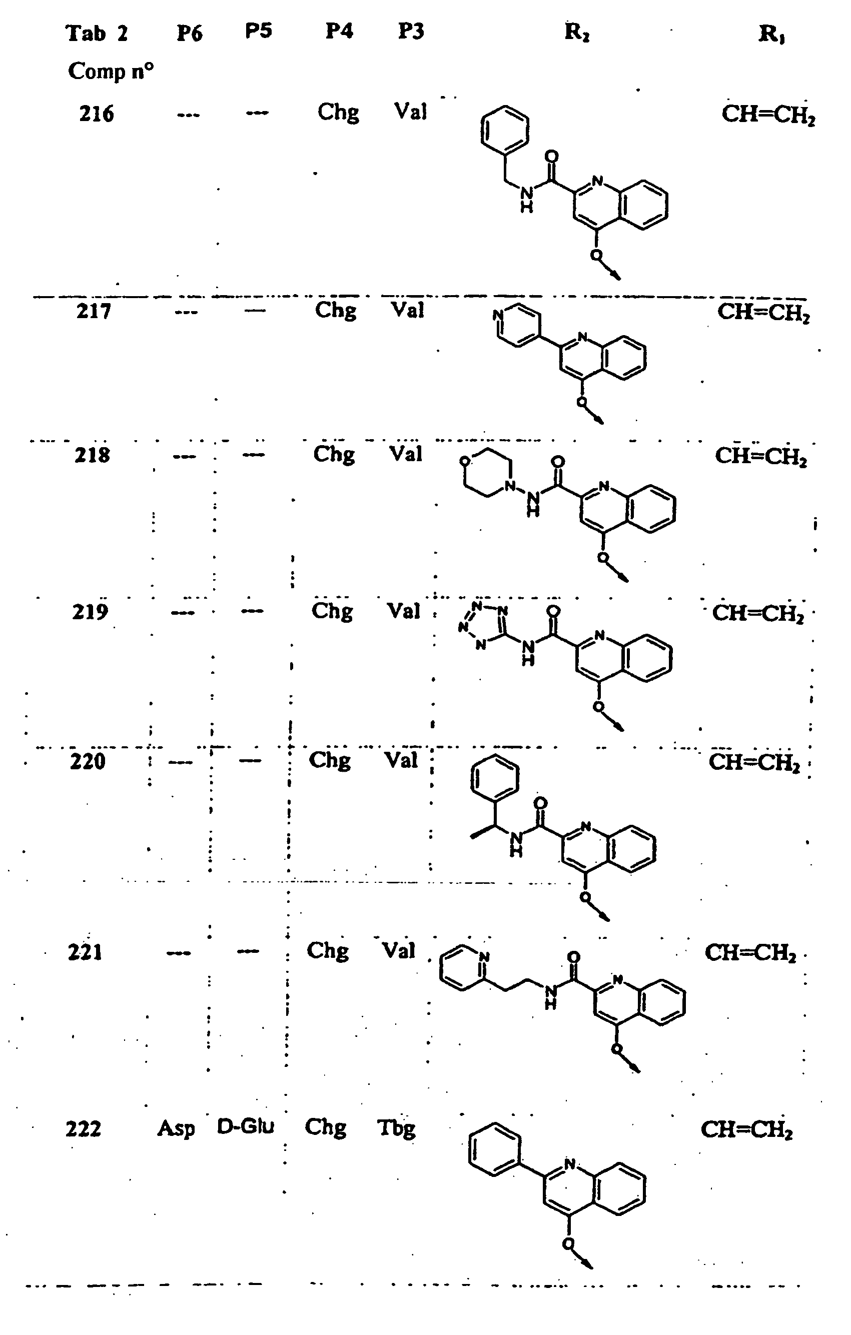

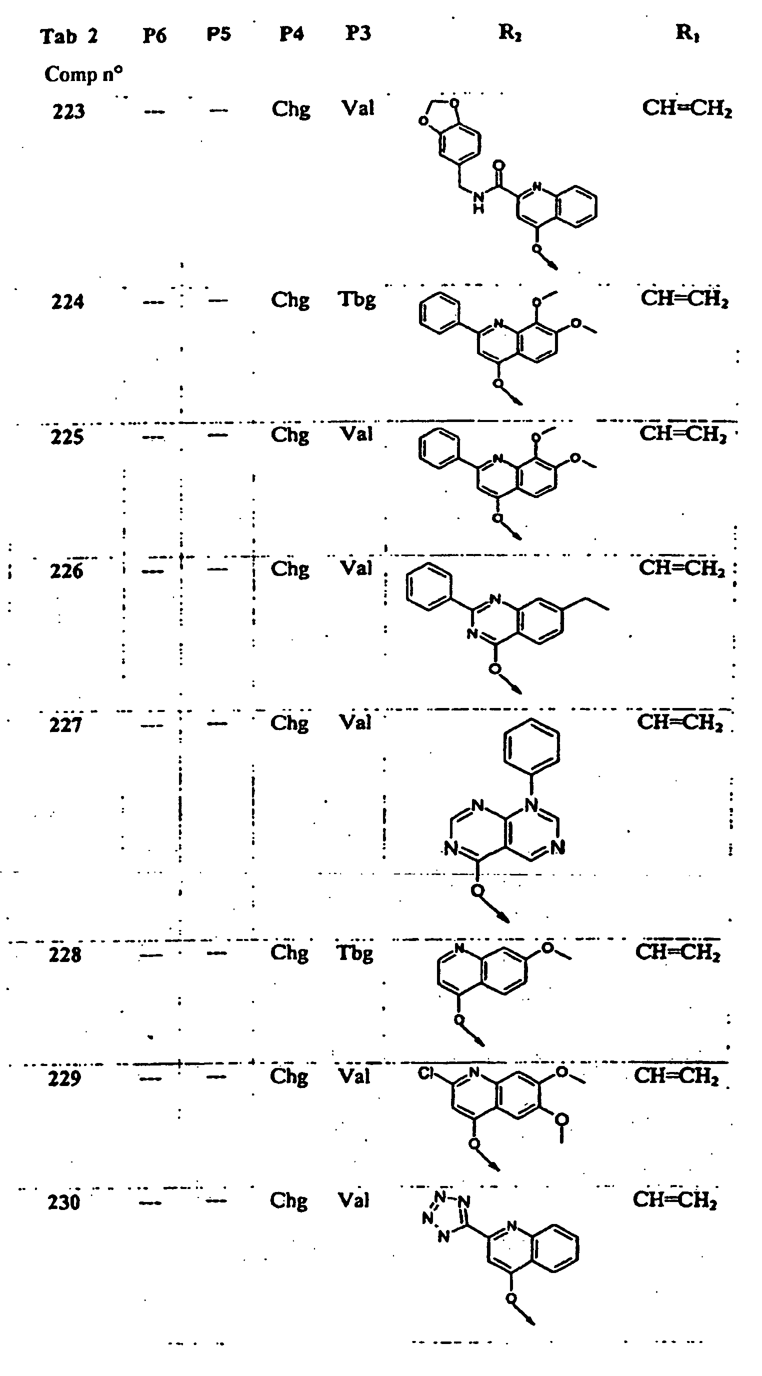

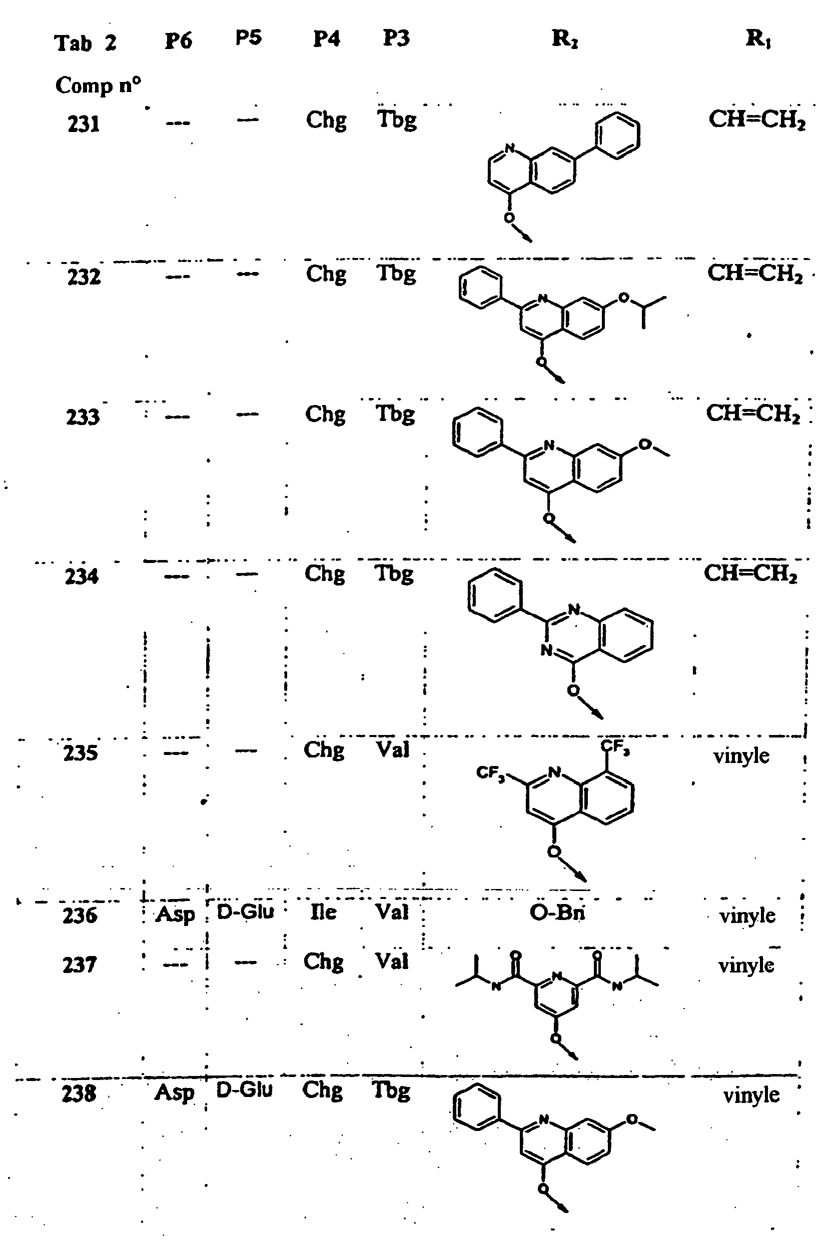

45. Composé selon la revendication 40 représenté par la formule : 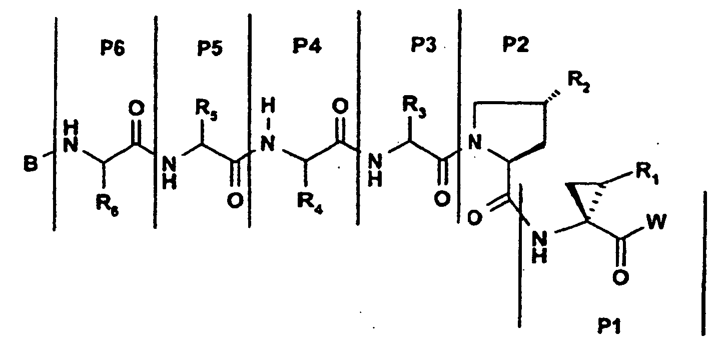
où **B, P6, P5, P4, P3, R**_{**2**}**, R**_{**1**} et **W** sont définis comme ci-dessous :

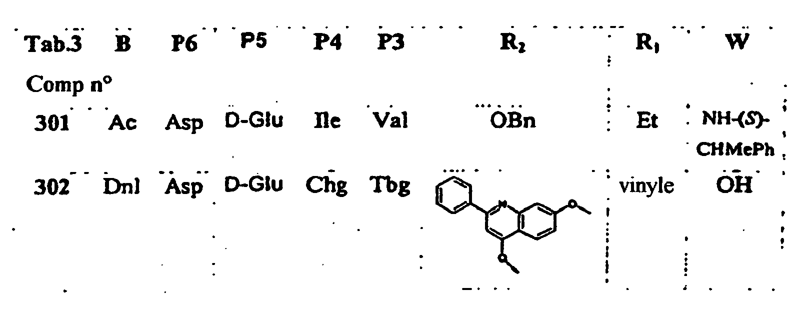

46. Composé selon la revendication 40 représenté par la formule : 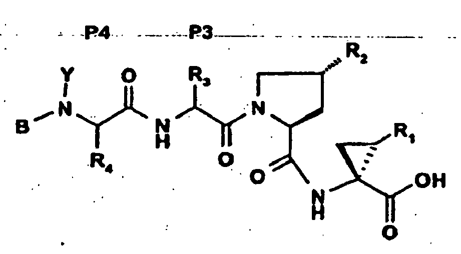
où **B, Y, P4, P3, R**_{**2**} et **R**_{**1**} sont définis comme ci-dessous :

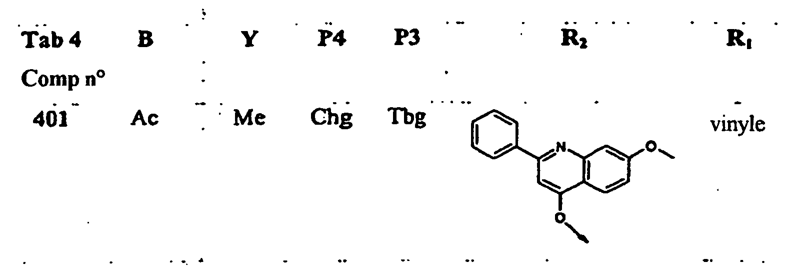

47. Composé selon la revendication 40 représenté par la formule : 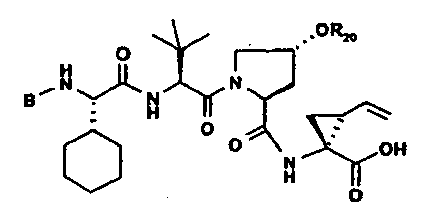
où **B** et **R**_{**20**} sont définis comme ci-dessous :

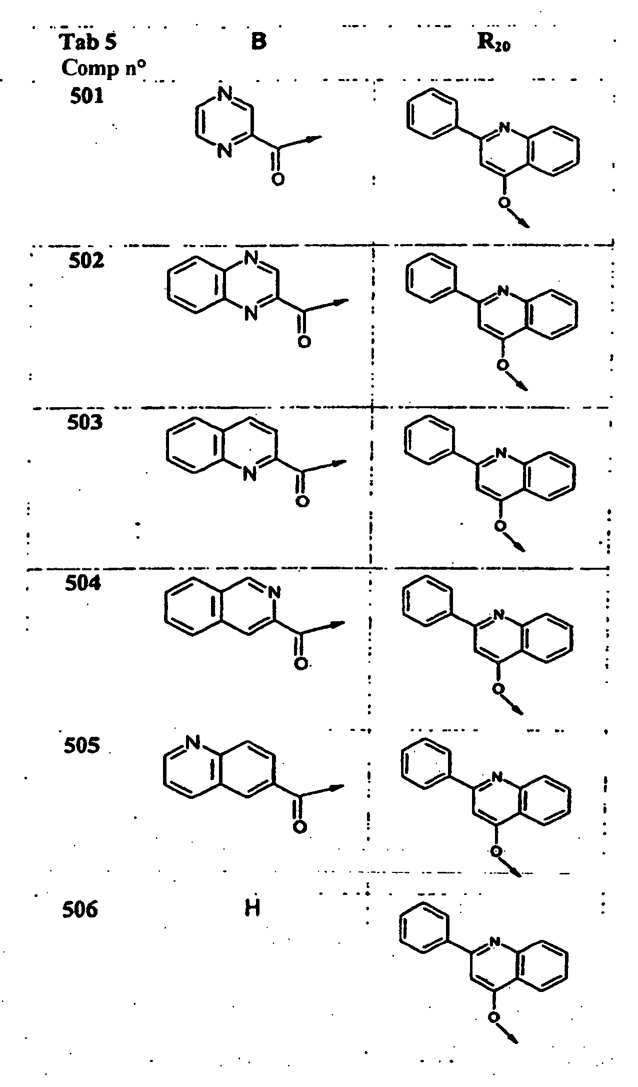

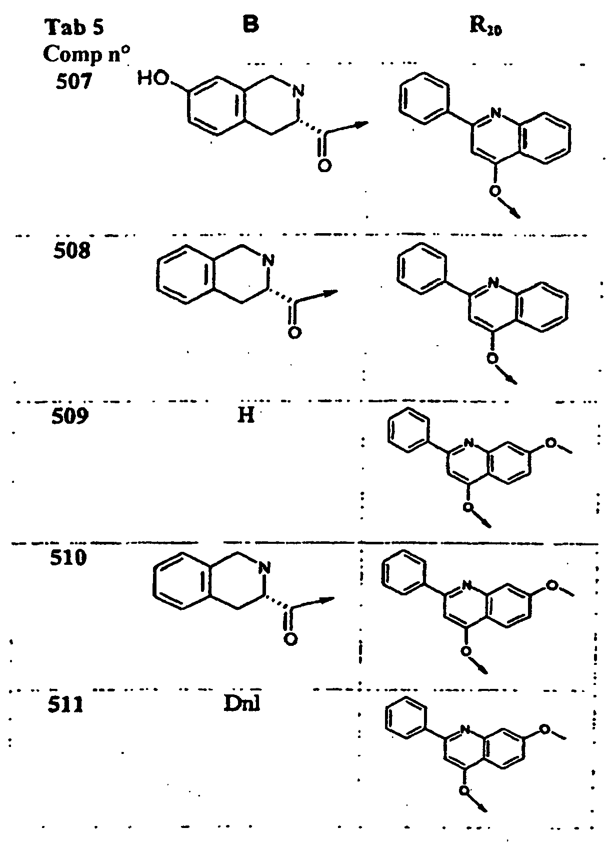

48. Hexapeptide de formule I selon la revendication 43 choisi dans le groupe consistant en le composé n°. 108 ; 116 ; 117 et 120.

49. Hexapeptide de formule I selon la revendication 44 choisi dans le groupe consistant en le composé n°. 212 ; 222 ; 236 ; et 238.

50. Hexapeptide de formule I selon la revendication 45 choisi dans le groupe consistant en le composé n°. 301 et 302.

51. Tétrapeptide de formule I selon la revendication 43 choisi dans le groupe consistant en le composé n°. 122 ; et 123.

52. Tétrapeptide de formule I selon la revendication 44 choisi dans le groupe consistant en le composé n°. 202 ; 203 ; 205 ; 206 ; 207 ; 208 ; 209 ; 210 ; 211 ; 214 ; 215 ; 216 ; 218 ; 219 ; 220 ; 221 ; 223 ; 224 ; 225 ; 226 ; 228 ; 229 ; 230 ; 231 ; 232 ; 233 ; 234 ; 235.

53. Tétrapeptide de formule I selon la revendication 46 choisi dans le groupe consistant en le composé n°. 401.

54. Tétrapeptide de formule I selon la revendication 47 choisi dans le groupe consistant en le composé n°. 501 ; 502 ; 503 ; 504 ; 505 ; 506 ; 507 ; 508 ; 509;510;et 511.

55. Composé selon l'une quelconque des revendications 1 à 54 comme médicament.

56. Composition pharmaceutique comprenant une quantité anti-hépatite C viralement efficace d'un composé de formule I selon la revendication 1, ou d'un sel ou ester thérapeutiquement acceptable de celui-ci, en mélange avec un milieu support ou agent auxiliaire pharmaceutiquement acceptable.

57. Utilisation d'un composé de formule I selon la revendication 1, ou d'un sel ou ester thérapeutiquement acceptable de celui-ci pour la préparation d'un médicament pour le traitement d'une infection virale de type hépatite C.

58. Procédé *in vitro* d'inhibition de la réplication du virus de l'hépatite C par exposition du virus à une quantité inhibant la protéase NS3 du virus de l'hépatite C du composé de formule 1 selon la revendication 1, ou d'un sel ou ester thérapeutiquement acceptable de celui-ci ou d'une composition selon la revendication 56.

59. Utilisation d'une combinaison du composé de formule I selon la revendication 1, ou d'un sel ou ester thérapeutiquement acceptable de celui-ci, et d'un interféron pour la préparation d'un médicament pour le traitement d'une infection virale de-type hépatite C.

60. Composition pharmaceutique selon la revendication 56, comprenant en outre un second agent antiviral.

61. Composition pharmaceutique selon la revendication 60 où ledit second agent antiviral est la ribavirine ou l'amantadine.

62. Composition pharmaceutique selon la revendication 56 comprenant en outre d'autres inhibiteurs de protéase de VHC.

63. Composition pharmaceutique selon la revendication 56 comprenant en outre un inhibiteur d'autres cibles dans le cycle vital de VHC, choisies parmi : hélicase, polymérase, métalloprotéase ou IRES.

64. Procédé pour la préparation d'un analogue de peptide de formule (I) selon la revendication 1 où P1 est un groupement acide aminocyclopropylcarboxylique substitué, comprenant l'étape de :
■ couplage d'un peptide choisi dans le groupe consistant en : APG-P6-P5-P4-P3-P2 ; APG-P5-P4-P3-P2 ; APG-P4-P3-P2 ; APG-P3-P2 ; et APG-P2 ;
■ avec un intermédiaire P1 de formule : 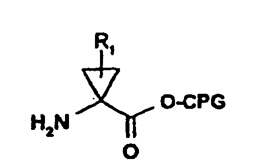
où **R**_{**1**} est C₁₋₆ alkyle ou C₂₋₆ alcényle éventuellement substitué avec halogène, CPG est un groupe protecteur de carboxyle et P6 à P2 sont définis comme dans la revendication 1.

65. Procédé pour la préparation d'un analogue de peptide de formule (I) selon la revendication 1 où P1 est un groupement acide aminocyclopropylcarboxylique substitué, comprenant l'étape de :
■ couplage d'un peptide choisi dans le groupe consistant en : APG-P6-P5-P4-P3-P2 ; APG-P5-P4-P3-P2 ; APG-P4-P3-P2 ; APG-P3-P2 ; et APG-P2 ;
■ avec un intermédiaire P1 de formule : 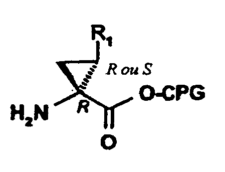
où **R**_{**1**} est éthyle, vinyle ou bromovinyle, CPG est un groupe protecteur de carboxyle et P6 à P2 sont définis comme dans la revendication 1.

66. Procédé pour la préparation d'un analogue de peptide de formule (I) selon la revendication 1 où P1 est un groupement acide aminocyclopropylcarboxylique substitué, comprenant l'étape de :
■ couplage d'un peptide choisi dans le groupe consistant en : APG-P6-P5-P4-P3-P2 ; APG-P5-P4-P3-P2 ; APG-P4-P3-P2 ; APG-P3-P2 ; et APG-P2 ;
■ avec un intermédiaire P1 de formule : 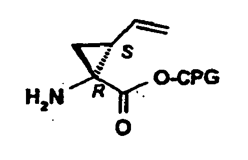
où CPG est un groupe protecteur de carboxyle et P6 à P2 sont définis comme dans la revendication 1.

67. Procédé selon la revendication 64, 65 ou 66 où ledit groupe protecteur de carboxyle (CPG) est choisi dans le groupe consistant en :
les esters d'alkyle, les esters d'aralkyle et les esters clivables par traitement avec une base modérée ou des moyens réducteurs modérés.

68. Utilisation d'un analogue d'aminoacide de formule : 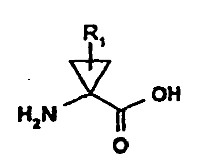
où **R**_{**1**} est C₁₋₆ alkyle ou C₂₋₆ alcényle éventuellement substitué avec halogène, pour la préparation d'un composé de formule I selon la revendication 1.

69. Utilisation d'un analogue d'aminoacide de formule : 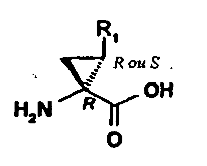
où **R**_{**1**} est éthyle, vinyle ou bromovinyle, pour la préparation d'un composé de formule I selon la revendication 1.

70. Utilisation d'un analogue d'aminoacide de formule: 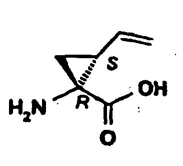
pour la préparation d'un composé de formule I selon la revendication 1.
